# EUROPEAN PATENT SPECIFICATION

(11) **EP 2 081 927 B1**
(45) Date of publication and mention of the grant of the patent: **17.08.2011**
(21) Application number: 07838310.6
(22) Date of filing: 17.09.2007
(51) Int. Cl.: C07D 407/12, A61K 31/453, A61P 9/00, C07D 309/04

(54) **PIPERIDINE DERIVATIVES AS RENIN INHIBITORS**
PIPERIDINDERIVATE ALS RENIN-INHIBITOREN
DERIVES DE PIPERIDINE UTILISES EN TANT QU'INHIBITEURS DE RENINE

(30) Priority: 18.09.2006 US 845331 P
(43) Date of publication of application: 29.07.2009
(73) Proprietor: Vitae Pharmaceuticals, Inc., Fort Washington, PA 19034 (US)
(72) Inventor: BALDWIN, John, J., Gwynedd, PA 19437 (US); CLAREMON, David, A., Maple Glen, PA 19002 (US); TICE, Colin, M., Ambler, PA 19002 (US); CACATIAN, Salvacion, Blue Bell, Pennsylvania 19422 (US); DILLARD, Lawrence, W., Yardley, PA 19067 (US); ISHCHENKO, Alexey, V., Somerville, Massachusetts 02144 (US); YUAN, Jing, Lansdale, PA 19446 (US); XU, Zhenrong, Horsham, PA 19044 (US); MCGEEHAN, Gerard, Garnet Valley, PA 19061 (US); ZHAO, Wei, Eagleville, PA 19403 (US); SIMPSON, Robert, D., Wilmington, DE 19802 (US); SINGH, Suresh, B., Kendall Park, NJ 08824 (US); FLAHERTY, Patrick, T., Pittsburgh, PA 15216 (US)
(74) Representative: Bublak, Wolfgang
(86) International application number: PCT/US2007/020086
(87) International publication number: WO 2008/036216

(56) References cited:
- WO-A-2006/042150
- WO-A-2007/070201

## Description

### BACKGROUND OF THE INVENTION

Aspartic proteases, including renin, β-secretase (BACE), HIV protease, HTLV protease and plasmepsins I and II, are implicated in a number of disease states. In hypertension elevated levels of angiotensin I, the product of renin catalyzed cleavage of angiotensinogen are present. Elevated levels of β amyloid, the product of BACE activity on amyloid precursor protein, are widely believed to be responsible for the amyloid plaques present in the brains of Alzheimer's disease patients. The viruses HIV and HTLV depend on their respective aspartic proteases for viral maturation. *Plasmodium falciparum* uses plasmepsins I and II to degrade hemoglobin.

In the renin-angiotensin-aldosterone system (RAAS), the biologically active peptide angiotensin II (Ang II) is generated by a two-step mechanism. The highly specific aspartic protease renin cleaves angiotensinogen to angiotensin I (Ang I), which is then further processed to Ang II by the less specific angiotensin-converting enzyme (ACE). Ang II is known to work on at least two receptor subtypes called AT₁ and AT₂. Whereas AT₁ seems to transmit most of the known functions of Ang II, the role of AT₂ is still unknown.

Modulation of the RAAS represents a major advance in the treatment of cardiovascular diseases (Zaman, M. A. et al Nature Reviews Drug Discovery 2002, 1, 621-636). ACE inhibitors and AT₁ blockers have been accepted as treatments of hypertension (Waeber B. el al., "The renin-angiotensin system: role in experimental and human hypertension," in Berkenhager W. H., Reid J. L. (eds): Hypertension, Amsterdam, Elsevier Science Publishing Co, 1996, 489-519; Weber M. A., Am. J. Hypertens., 1992, 5, 247S). In addition, ACE inhibitors are used for renal protection (Rosenberg M. E. et al., Kidney International, 1994, 45, 403; Breyer J. A. et al., Kidney International, 1994, 45, S156), in the prevention of congestive heart failure (Vaughan D. E. et al., Cardiovasc. Res., 1994, 28, 159; Fouad-Tarazi F. et al., Am. J. Med, 1988, 84 (Suppl. 3A), 83) and myocardial infarction (Pfeffer M. A. et al., N Engl. J: Med, 1992, 327, 669).

Interest in the development of renin inhibitors stems from the specificity of renin (Kleinert H. D., Cardiovasc. Drugs, 1995, 9, 645). The only substrate known for renin is angiotensinogen, which can only be processed (under physiological conditions) by renin. In contrast, ACE can also cleave bradykinin besides Ang I and can be bypassed by chymase, a serine protease (Husain A., J. Hypertens., 1993, 11, 1155). In patients, inhibition of ACE thus leads to bradykinin accumulation, causing cough (5-20%) and potentially life-threatening angioneurotic edema (0.1-0.2%) (Israili Z. H. et al., Annals of Internal Medicine, 1992, 117, 234). Chymase is not inhibited by ACE inhibitors. Therefore, the formation of Ang II is still possible in patients treated with ACE inhibitors. Blockade of the ATI receptor (e.g., by losartan) on the other hand overexposes other AT-receptor subtypes to Ang II, whose concentration is dramatically increased by the blockade of AT1 receptors. In summary, renin inhibitors are not only expected to be superior to ACE inhibitors and AT₁ blockers with regard to safety, but more importantly also with regard to their efficacy in blocking the RAAS.

Only limited clinical experience (Azizi M. et al., J. Hypertens., 1994, 12, 419; Neutel J. M. et al., Am. Heart, 1991, 122, 1094) has been generated with renin inhibitors because their peptidomimetic character imparts insufficient oral activity (Kleinert H. D., Cardiovasc. Drugs, 1995, 9, 645). The clinical development of several compounds has been stopped because of this problem together with the high cost of goods. It appears as though only one compound has entered clinical trials (Rahuel J. et al., Chem. Biol., 2000, 7, 493; Mealy N. E., Drugs of the Future, 2001, 26, 1139). Thus, metabolically stable, orally bioavailable and sufficiently soluble renin inhibitors that can be prepared on a large scale are not available. Recently, the first non-peptide renin inhibitors were described which show high *in vitro* activity (Oefner C. et al., Chem. Biol., 1999, 6, 127; Patent Application WO 97/09311; Maerki H. P. et al., Il Farmaco, 2001, 56, 21). WO2006/042150 describes diaminoalkane aspartic protease inhibitors. The present invention relates to the unexpected identification of renin inhibitors of a non-peptidic nature and of low molecular weight. Orally active renin inhibitors which are active in indications beyond blood pressure regulation where the tissular renin-chymase system may be activated leading to pathophysiologically altered local functions such as renal, cardiac and vascular remodeling, atherosclerosis, and restenosis, are described.

### SUMMARY OF THE INVENTION

One embodiment of the invention is an aspartic protease inhibitor, which is a compound represented by Structural Formula (I): 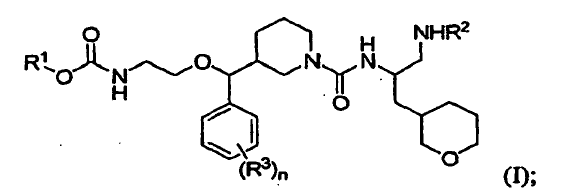 or a pharmaceutically acceptable salt thereof, wherein:
R¹ is alkyl, cycloalkyl or cycloalkylalkyl;
R² is H or alkyl;
R³ is F, Cl, Br, cyano, nitro, alkyl, haloalkyl, alkoxy, haloalkoxy or alkanesulfonyl; and
n is 0, 1, 2, or 3.

Another embodiment of the invention is an aspartic protease inhibitor, which is a compound represented by Structural Formula (II): 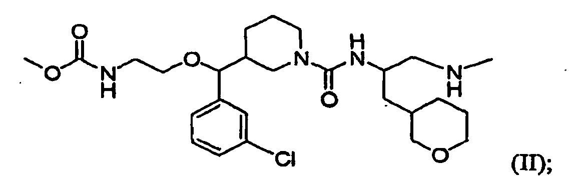 or a pharmaceutically acceptable salt thereof.

Another embodiment of the invention is an aspartic protease inhibitor, which is a compound represented by Structural Formula (IIa): 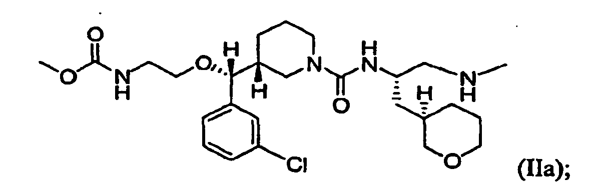 or a pharmaceutically acceptable salt thereof, wherein the inhibitor is at least 90% optically pure.

Another embodiment of the invention is an aspartic protease inhibitor, which is a compound represented by Structural Formula (III): 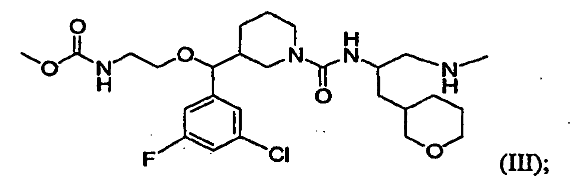 or a phamaceutically acceptable salt thereof.

Another embodiment of the invention is an aspartic protease inhibitor, which is a compound represented by Structural Formula (IIIa): 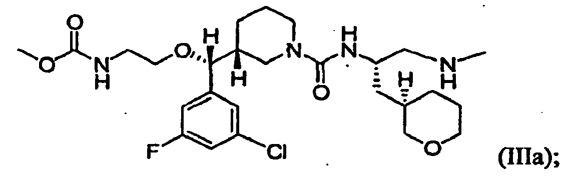 or a pharmaceutically acceptable salt thereof, wherein the inhibitor is at least 90% optically pure.

Another embodiment of the invention is a pharmaceutical composition comprising a pharmaceutically acceptable carrier or diluent and an aspartic protease inhibitor disclosed herein (e.g., a compound represented by Structural Formulas (I)-(IIIa) or a pharmaceutically acceptable salt thereof). The pharmaceutical composition is used in therapy, e.g., for inhibiting an aspartic protease mediated disorder in a subject.

Another embodiment of the invention is a compound of formula (I) for use in a method of antagonizing one or more aspartic proteases in a subject in need of such treatment. The method comprises administering to the subject an effective amount of an aspartic protease inhibitor disclosed herein (e.g., a compound represented by Structural Formulas (I)-(IIIa) or a pharmaceutically acceptable salt thereof).

Another embodiment of the invention is a compound of formula (I) for use in a method of treating an aspartic protease mediated disorder in a subject. The method comprises administering to the subject an effective amount of an aspartic protease inhibitor disclosed herein (e.g., a compound represented by Structural Formulas (I)-(IIIa) or a pharmaceutically acceptable salt thereof).

Another embodiment of the invention is the use of an aspartic protease inhibitor disclosed herein (e.g., a compound represented by Structural Formulas (I)-(IIIa) or a pharmaceutically acceptable salt thereof) for the manufacture of a medicament for antagonizing one or more proteases in a subject in need of such treatment.

Another embodiment of the invention is the use of an aspartic protease inhibitor disclosed herein (e.g., a compound represented by Structural Formulas (I)-(IIIa) or a pharmaceutically acceptable salt thereof) for the manufacture of a medicament for treating an aspartic protease mediated disorder in a subject.

Another embodiment of the invention is the aspartic protease inhibitor disclosed herein (e.g., a compound represented by Structural Formulas (I), (II), (IIa) or a pharmaceutically acceptable salt thereof) for use in therapy, such as treating an aspartic protease mediated disorder in a subject. Values for the variables of Structural Formulas (I) are as described above.

Another embodiment of the invention is the an aspartic protease inhibitor disclosed herein (e.g., a compound represented by Structural Formulas (I), (II), (IIa) or a pharmaceutically acceptable salt thereof) for use in treating a subject having hypertension, congestive heart failure, cardiac hypertrophy, cardiac fibrosis, cardiomyopathy post-infarction, nephropathy, vasculopathy and neuropathy, a disease of the coronary vessels, post-surgical hypertension, restenosis following angioplasty, raised intra-ocular pressure, glaucoma, abnormal vascular growth, hyperaldosteronism, an anxiety state, or a cognitive disorder, wherein values for the variables of Structural Formula (I) are as described above.

### BRIEF DESCRIPTION OF THE DRAWINGS

Figure 1 is an x-ray powder diffraction pattern obtained from a sample of the L-tartrate salt of the compound represented by Structural Formula (IIa).
Figure 2 is a plot showing mean plasma concentrations of compound **6a** in transgenic rats over time following oral adminstration of 10 mg/kg of compound 6a.
Figure 3 is a plot showing changes in mean arterial blood pressures of transgenic rats treated with 10mg/kg of compound **6a**.

### DETAILED DESCRIPTION OF THE INVENTION

The invention is directed to an aspartic protease inhibitor represented by Structural Formula (I), or a pharmaceutically acceptable salt thereof.

In another embodiment, the aspartic protease inhibitor of the present invention is a compound represented by the Structural Formula (Ia): 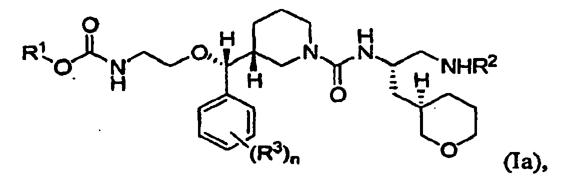 or a pharmaceutically acceptable salt thereof.

Values and specific values for the variables in Structural Formulas (I) and (Ia) are defined as follows:
R¹ is alkyl, cycloalkyl (e.g., cyclopropyl) or cycloalkylalkyl (e.g., cyclopropyl(C₁-C₃)alkyl); more specifically, R¹ is (C₁-C₃)alkyl; even more specifically, R¹ is methyl or ethyl;
R² is H or alkyl; more specifically, R² is H or (C₁-C₃)alkyl; even more specifically, R² is methyl;
R³ is F, Cl, Br, cyano, nitro, alkyl, haloalkyl, alkoxy, haloalkoxy or alkanesulfonyl; more specifically, R³ is F, Cl, Br, cyano, nitro, (C₁-C₃)alkyl, halo(C₁-C₃)alkyl, (C₁-C₃)alkoxy, halo(C₁-C₃)alkoxy or (C₁-C₃)alkanesulfonyl; even more specifically R³ is F, Cl, and methyl; and
n is 0, 1, 2, or 3; more specifically, n is 0, 1, or 2; even more specifically n is 1 or 2.

In one specific embodiment, the aspartic protease inhibitor is represented by Structural Formula (I) or (Ia), wherein R¹ is (C₁-C₃)alkyl; R² is H or (C₁-C₃)alkyl; R³ is F, Cl, Br, cyano, nitro, (C₁C₃)alkyl, halo(C₁-C₃)alkyl, (C₁-C₃)alkoxy, halo(C₁-C₃)alkoxy or (C₁-C₃)alkanesulfonyl; and n is 0, 1, 2, or 3.

In another specific embodiment, the aspartic protease inhibitor is represented by Structural Formula (I) or (Ia), wherein R² is methyl and R¹ is methyl or ethyl; values and specific values for other variables are as defined above for Formulas (I) and (Ia). In another specific embodiment, R² is methyl; R¹ is methyl or ethyl; and R³ is F, Cl or methyl; values and specific values for other variables are the same as described above for Formulas (I) and (Ia).

In another specific embodiment, the aspartic protease inhibitor of the present invention is one of the following compounds or their enantiomers diastereomers. Also included are pharmaceutically acceptable salts and solvates (e.g., hydrates) of all of the following and their enantiomers and diastereomers:

| Compound Number | Structure | Name |
|---|---|---|
| **1** | 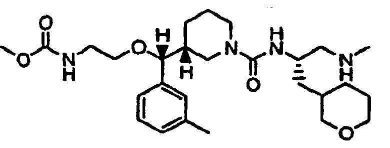 | methyl 2-((*R*)-((*R*)-1-((*S*)-1-(methylamino)-3-(tetrahydro-2*H*-pyran-3-yl)propan-2-ylcarbamoyl)piperidin-3-yl)(*m*-tolyl)-methoxy)ethylcarbamate |
| **2a** | 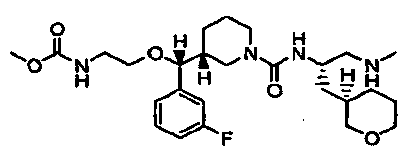 | methyl 2-((*R*)-(3-fluorophenyl)((*R*)-1-((*S*)-1-(methylamino)-3-((*R)-*tetrahydro-2*H*-pyran-3-yl)propan-2-ylcarbamoyl)piperidin-3-yl)methoxy)ethylcarbamate |
| **2b** | 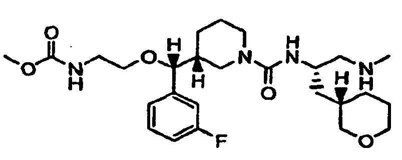 | methyl 2-((*R*)-(3-fluorophenyl)((*R*)-1-((*S*)-1-(methylamino)-3-((*S*)-tetrahydro-2*H*-pyran-3-yl)propan-2-ylcarbamoyl)piperidin-3-yl)methoxy)ethylcarbamate, |
| **3a** | 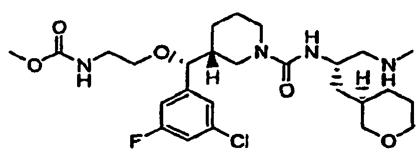 | methyl 2-((*R*)-(3-chloro-5-fluorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate |
| **3b** | 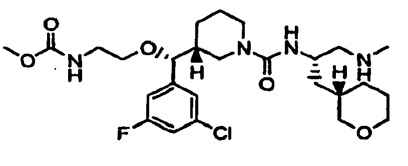 | methyl 2-((*R*)-(3-chloro-5-fluorophenyl)((*R*)-1-((*S*)-1-(methylamino)-3-((*S*)-tetrahydro-2*H*-pyran-3-yl)propan-2-ylcarbamoyl)piperidin-3-yl)methoxy)ethylcarbamate |
| **3c, 3d** | 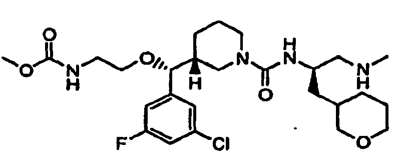 | methyl 2-((*R*)-(3-chloro-5-fluorophenyl)((3*R*)-1-((*R*)-1-(methylamino)-3-(tetrahydro-2*H*-pyran-3-yl)propan-2-ylcarbamoyl)piperidin-3-yl)methoxy)ethylcarbamate |
| **4a** | 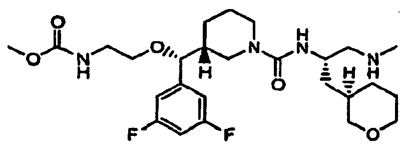 | methyl 2-((*R*)-(3,5difluorophenyl)((*R*)-1-((*S*)-1(methylamino)-3-((*R*)tetrahydro-2*H*-pyran-3yl)propan-2ylcarbamoyl)piperidin-3-yl)methoxy)ethylcarbamate |
| **4b** | 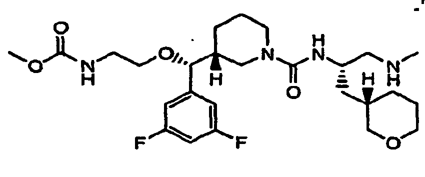 | methyl 2-((*R*)-(3,5difluorophenyl)((*R*)-1-((*S*)-1-(methylamino)-3-((*S*)-tetrahydro-2*H*-pyran-3-yl)propan-2-ylcarbamoyl)piperidin-3-yl)methoxy)ethylcarbamate |
| **5a** | 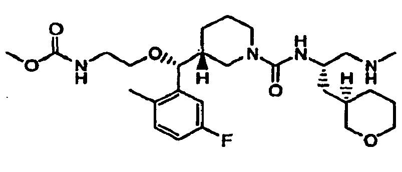 | methyl 2-((*R*)-(5-fluoro-2-methylphenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate |
| **5b** | 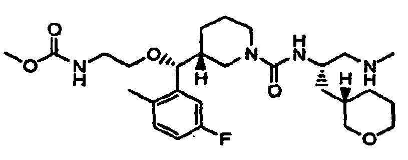 | methyl 2-((*R*)-(5-fluoro-2-methylphenyl)((*R*)-1-((*S*)-1-(methylamino)-3-((*S*)-tetrahydro-2*H*-pyran-3-yl)propan-2-ylcarbamoyl)piperidin-3-yl)methoxy)ethylcarbamate |
| **6a** | 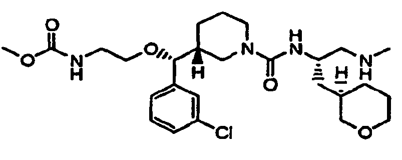 | methyl 2-((*R*)-(3-chlorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate |
| **6b** | 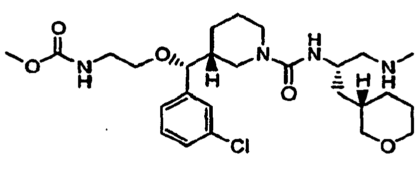 | methyl 2-((*R*)-(3-chlorophenyl)((*R*)-1-((*S*)-1-(methylamino)-3-((*S*)-tetrahydro-2*H*-pyran-3-yl)propan-2-ylcarbamoyl)piperidin-3-yl)methoxy)ethylcarbamate |
| **7** | 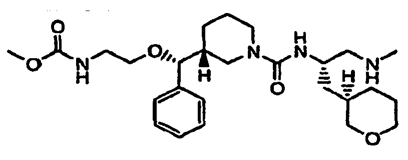 | methyl 2-((*R*)-((*R*)-1-((*S*)-1- (methylamino)-3-((*R*)- tetrahydro-2*H*-pyran-3-yl)propan-2-ylcarbamoyl)piperidin-3-yl)(phenyl)methoxy)ethylcarbam ate |
| **8** | 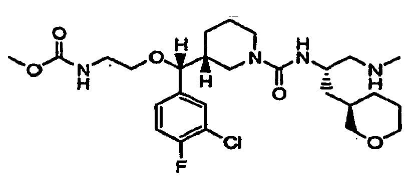 | methyl 2-((*R*)-(3-chloro-4-fluorophenyl)((*R*)-1*-((S)-1*-(methylamino)-3-((*R*)-tetrahydro-2*H*-pyran-3-yl)propan-2-ylcarbamoyl)piperidin-3-yl)methoxy)ethylcarbamate |
| **9** | 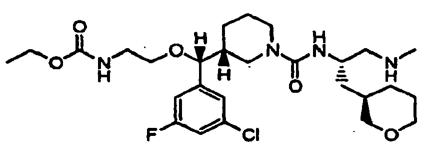 | ethyl 2-((*R*)-(3-chloro-5-fluorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate |
| **10** | 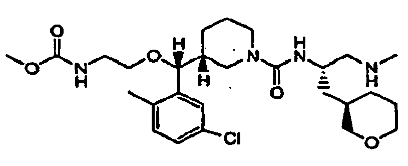 | methyl 2-((*R*)-(5-chloro-2-methylphenyl)((*R*)-1*-*((*S*)-1-(methylamino)-3-((*R*)-tetrahydro-2*H*-pyran-3-yl)propan-2-ylcarbamoyl)piperidin-3-yl)methoxy)ethylcarbamate |

Another embodiment of the invention is directed to an intermediate for synthesizing the aspartic protease inhibitors disclosed herein, represented by Structural Formulas (IV), (IVa), (IVb), (IVc) or (IVd) and salts thereof (preferably pharmaceutically acceptable salts): 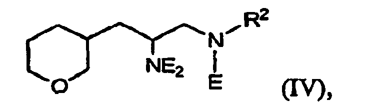 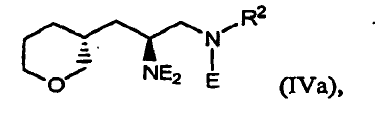 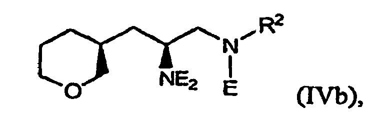 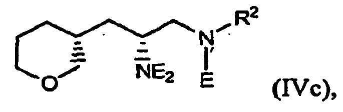 or

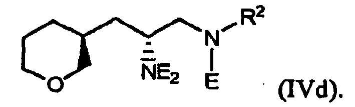

In Structural Formulas (IV), (IVa), (IVb), (IVc), and (IVd), E, for each occurrence, is independently H or an amine protecting group. Amine protecting groups include carbamate, amide, and sulfonamide protecting groups known in the art (T. W. Greene and P. G. M. Wuts "Protective Groups in Organic Synthesis" John Wiley & Sons, Inc., New York 1999, ). Specific amine protecting groups include *tert-*butoxycarbonyl (Boc), benzyloxycarbonyl (Cbz) and 1-[2-(trimethylsilyl)ethoxycarbonyl] (Teoc). Values and specific values for R² are as described for Structural Formula (I).

In a specific embodiment, the intermediate is each of the following compounds or their enantiomers or diastereomers. Pharmaceutically acceptable salts of all of the following are also included:

| Cpd No. | Cpd Name |
|---|---|
| IVa-1 | *tert*-butyl (*S*)-1-(methylamino)-3-((*R*)-tetrahydro-2*H*-pyran-3-yl)propan-2-ylcarbamate |
| IVa-2 | (*S*)-*tert*-butyl-1-(N-methyl-2-(trimethylsilyl)ethoxycarbonylamino)-3-((*R*)-tetrahydro-2*H-*pyran-3-yl)propylcarbamate |
| IVa-3 | 2-(trimethylsilyl)ethyl (*S*)-2-amino-3-((*R*)-tetrahydro-2*H*-pyran-3-yl)propyl(methyl)carbamate |
| IV-1 | *tert*-butyl (*S*)-1-(methylamino)-3-(tetrahydro-2*H*-pyran-3-yl)propan-2-ylcarbamate |
| IV-2 | (*S*)-*tert*-butyl-1-(N-methyl-2-(trimethylsilyl)ethoxycarbonylamino)-3-(tetrahydro-2*H*-pyran-3-yl)propylcarbamate |
| IV-3 | 2-(trimethylsilyl)ethyl (*S*)-2-amino-3-((*R*)-tetrahydro-2*H*-pyran-3-yl)propyl(methyl)carbamate |
| IVb-1 | *tert*-butyl (*S*)-1-(methylamino)-3-((*S*)-tetrahydro-2*H*-pyran-3-yl)propan-2-ylcarbamate |
| IVb-2 | *(S)-tert*-butyl-1-(N-methyl-2-(trimethylsilyl)ethoxycarbonylamino)-3-((*S*)-tetrahydro-2*H*-pyran-3-yl)propylcarbamate |
| IVb-3 | 2-(trimethylsilyl)ethyl (*S*)-2-aniino-3-((*S*)-tetrahydro-2*H*-pyran-3-yl)propyl(methyl)carbamate |

When any variable (e.g., R³) occurs more than once in a compound, its definition on each occurrence is independent of any other occurrence. For example, R³, for each occurrence, is independently selected from the group consisting of F, Cl, Br, cyano, nitro, alkyl, haloalkyl, alkoxy, haloalkoxy and alkanesulfonyl.

When the "aspartic protease inhibitor" of the present invention is named or depicted by structure, it also includes pharmaceutically acceptable salts thereof.

"Alkyl", alone or part of another moiety (such as cycloalkylalkyl, alkoxy, haloalkoxy, haloalkyl or alkoxy), means a saturated aliphatic branched or straight-chain mono- or divalent hydrocarbon radical. Alkyls commonly have from one to six carbon atoms, typically from one to three carbon atoms. Thus, "(C₁-C₃)alkyl" means a radical having from 1-3 carbon atoms in a linear or branched arrangement. "(C₁-C₃)alkyl" includes methyl, ethyl, propyl and isopropyl.

"Cycloalkyl", alone or as part of another moiety (such as cycloalkylalkyl) means a saturated aliphatic cyclic mono-valent hydrocarbon radical. Typically, cycloalkyls have from three to ten carbon atoms and are mono, bi or tricyclic. Tricyclic cycloalkyls can be fused or bridged. Typically, cycloalkyls are C₃-C₈ monocyclic and are more commonly cyclopropyl.

"Cycloalkylalkyl" means an alkyl radical substituted with a cycloalkyl group.

"Haloalkyl" includes mono, poly, and perhaloalkyl groups where the halogens are independently selected from fluorine, chlorine, and bromine.

"Alkoxy" means an alkyl radical attached through an oxygen linking atom. "(C₁-C₃)-alkoxy" includes the methoxy, ethoxy, and propoxy.

"Haloalkoxy" is a haloalkyl group which is attached to another moiety via an oxygen linker.

"Alkanesulfonyl" is an alkyl radical attached through a 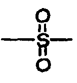 linking group. "(C₁-C₃)alkanesulfonyl" includes methanesulfonyl, ethanesulfonyl and propanesulfonyl.

Certain of the disclosed aspartic protease inhibitors may exist in various tautomeric forms. The invention encompasses all such forms, including those forms not depicted structurally.

Certain of the disclosed aspartic protease inhibitors may exist in various stereoisomeric forms. Stereoisomers are compounds which differ only in their spatial arrangement. Enantiomers are pairs of stereoisomers whose mirror images are not superimposable, most commonly because they contain an asymmetrically substituted carbon atom that acts as a chiral center. "Enantiomer" means one of a pair of molecules that are mirror images of each other and are not superimposable. Diastereomers are stereoisomers that are not related as mirror images, most commonly because they contain two or more asymmetrically substituted carbon atoms. "R" and "S" represent the configuration of substituents around one or more chiral carbon atoms. When a chiral center is not defined as R or S and the configuration at the chiral center is not defined by other means, either configuration can be present or a mixture of both configurations is present.

"Racemate" or "racemic mixture" means a compound of equimolar quantities of two enantiomers, wherein such mixtures exhibit no optical activity; i.e., they do not rotate the plane of polarized light.

"R" and "S" indicate configurations relative to the core molecule.

" 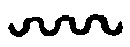 " represents " 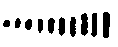 ", " 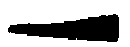 " or" 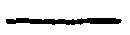 ", wherein the depicted enantiomer (e.g., " 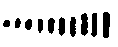 " or " 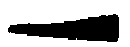 ") is at least 60%, 70%, 80%, 90%, 99% or 99.9% optically pure.

The disclosed aspartic protease inhibitors may be prepared as individual isomers by either isomer-specific synthesis or resolved from an isomeric mixture. Conventional resolution techniques include forming the salt of a free base of each isomer of an isomeric pair using an optically active acid (followed by fractional crystallization and regeneration of the free base), forming the salt of the acid form of each isomer of an isomeric pair using an optically active amine (followed by fractional crystallization and regeneration of the free acid), forming an ester or amide of each of the isomers of an isomeric pair using an optically pure acid, amine or alcohol (followed by chromatographic separation and removal of the chiral auxiliary), or resolving an isomeric mixture of either a starting material or a final product using various well known chromatographic methods.

When the stereochemistry of a disclosed aspartic protease inhibitor is named or depicted by structure, the named or depicted stereoisomer is at least 60%, 70%, 80%, 90%, 99% or 99.9% by weight pure relative to the other stereoisomers. When a single enantiomer is named or depicted by structure, the depicted or named enantiomer is at least 60%, 70%, 80%, 90%, 99% or 99.9% optically pure. Percent optical purity by weight is the ratio of the weight of the enatiomer over the weight of the enantiomer plus the weight of its optical isomer.

When a disclosed aspartic protease inhibitor is named or depicted by structure without indicating the stereochemistry, and the inhibitor has at least one chiral center, it is to be understood that the name or structure encompasses one enantiomer of inhibitor free from the corresponding optical isomer, a racemic mixture of the inhibitor and mixtures enriched in one enantiomer relative to its corresponding optical isomer.

When a disclosed aspartic protease inhibitor is named or depicted by structure without indicating the stereochemistry and has at least two chiral centers, it is to be understood that the name or structure encompasses a diastereomer free of other diastereomers, a pair of diastereomers free from other diastereomeric pairs, mixtures of diastereomers, mixtures of diastereomeric pairs, mixtures of diastereomers in which one diastereomer is enriched relative to the other diastereomer(s) and mixtures of diastereomeric pairs in which one diastereomeric pair is enriched relative to the other diastereomeric pair(s).

Pharmaceutically acceptable salts of the compounds of the aspartic protease inhibitors are included in the present invention. For example, an acid salt of an aspartic protease inhibitor containing an amine or other basic group can be obtained by reacting the compound with a suitable organic or inorganic acid, resulting in pharmaceutically acceptable anionic salt forms. Examples of anionic salts include the acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrobromide, hydrochloride, hydroxynaphthoate, iodide, isethionate, lactate, lactobionate, palate, maleate, mandelate, mesylate, methylsulfate, mucate, napsylate, nitrate, pamoate, pantothenate, phosphate/diphospate, polygalacturonate, salicylate, stearate, subacetate, succinate, sulfate, tannate, tartrate, teoclate, tosylate, and triethiodide salts.

Salts of the compounds of the aspartic protease inhibitors containing an acidic functional group can be prepared by reacting with a suitable base. Such a pharmaceutically acceptable salt may be made with a base which affords a pharmaceutically acceptable cation, which includes alkali metal salts (especially sodium and potassium), alkaline earth metal salts (especially calcium and magnesium), aluminum salts and ammonium salts, as well as salts made from physiologically acceptable organic bases such as trimethylamine, triethylamine, morpholine, pyridine, piperidine, picoline, dicyclohexylamine, N,N'-dibenzylethylenediamine, 2-hydroxyethylamine, bis-(2-hydroxyethyl)amine, tri-(2-hydroxyethyl)amine, procaine, dibenzylpiperidine, dehydroabietylamine, N,N'-bisdehydroabietylamine, glucamine, N-methylglucamine, collidine, quinine, quinoline, and basic amino acids such as lysine and arginine.

In accordance with the present invention, non-pharmaceutically acceptable salts of the compounds of the aspartic protease inhibitors and their synthetic intermediates are also included. These salts (for example, TFA salt) may be used, for example, for purification and isolation of the the compounds of the aspartic protease inhibitors and their synthetic intermediates.

When a disclosed aspartic protease inhibitor is named or depicted by structure, it is to be understood that solvates (e.g., hydrates) of the aspartic protease inhibitor or its pharmaceutically acceptable salts are also included. "Solvates" refer to crystalline forms wherein solvent molecules are incorporated into the crystal lattice during crystallization. Solvate may include water or nonaqueous solvents such as ethanol, isopropanol, DMSO, acetic acid, ethanolamine, and EtOAc. Solvates, wherein water is the solvent molecule incorporated into the crystal lattice, are typically referred to as "hydrates". Hydrates include stoichiometric hydrates as well as compositions containing variable amounts of water.

When a disclosed aspartic protease inhibitor is named or depicted by structure, it is to be understood that the compound, including solvates thereof, may exist in crystalline forms, non-crystalline forms or a mixture thereof. The aspartic protease inhibitor or solvates may also exhibit polymorphism (i.e. the capacity to occur in different crystalline forms). These different crystalline forms are typically known as "polymorphs." It is to be understood that when named or depicted by structure, the disclosed aspartic protease inhibitors and solvates (e.g., hydrates) also include all polymorphs thereof. Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. One of ordinary skill in the art will appreciate that different polymorphs may be produced, for example, by changing or adjusting the conditions used in solidfying the compound. For example, changes in temperature, pressure, or solvent may result in different polymorphs. In addition, one polymorph may spontaneously convert to another polymorph under certain conditions.

It may be necessary and/or desirable during synthesis to protect sensitive or reactive groups on any of the molecules concerned. Representative conventional protecting groups are described in T.W. Greene and P. G. M. Wuts "Protective Groups in Organic Synthesis" John Wiley & Sons, Inc., New York 1999. Protecting groups may be added and removed using methods well known in the art.

The disclosed aspartic protease inhibitors are useful for ameliorating or treating disorders or diseases in which decreasing the levels of aspartic protease products is effective in treating the disease state or in treating infections in which the infectious agent depends upon the activity of an aspartic protease. For example, the disclosed aspartic protease inhibitors are useful for ameliorating or treating disorders or diseases in which decreasing the levels of renin products is effective in treating a disease state. In hypertension, elevated levels of angiotensin I, the product of renin-catalyzed cleavage of angiotensinogen, are present. Thus, the disclosed aspartic protease inhibitors can be used in the treatment of hypertension, congestive heart failure, cardiac hypertrophy, cardiac fibrosis, cardiomyopathy post-infarction, complications resulting from diabetes, such as nephropathy, vasculopathy and neuropathy, diseases of the coronary vessels, proteinuria, albumenuria, post-surgical hypertension, metabolic syndrome, obesity, restenosis following angioplasty, raised intra-ocular pressure, glaucoma, abnormal vascular growth, hyperaldosteronism, anxiety states, and cognitive disorders (Fisher N.D.; Hollenberg N. K. Expert Opin. Investig. Drugs. 2001, 10, 417-26).

A pharmaceutical composition of the invention may, alternatively or in addition to a disclosed aspartic protease inhibitor, comprise a prodrug or pharmaceutically active metabolite of such a compound or salt and one or more pharmaceutically acceptable carriers or diluent therefor.

The invention includes a compound of formula (I) for use in a therapeutic method for treating or ameliorating an aspartic protease mediated disorder in a subject in need thereof comprising administering to a subject in need thereof an effective amount of an aspartic protease inhibitor disclosed herein.

Administration methods.include administering an effective amount of a compound or composition of the invention at different times during the course of therapy or concurrently in a combination form. The methods of the invention include all known therapeutic treatment regimens.

"Effective amount" means that amount of drug substance (i.e. aspartic protease inhibitors of the present invention) that elicits the desired biological response in a subject. Such response includes alleviation of the symptoms of the disease or disorder being treated. The effective amount of a disclosed aspartic protease inhibitor in such a therapeutic method is from about .01 mg/kg/day to about 10 mg/kg/day, preferably from about 0.5 mg/kg/day to 5 mg/kg/day.

The invention includes the use of a disclosed aspartic protease inhibitor for the preparation of a composition for treating or ameliorating an aspartic protease mediated chronic disorder or disease or infection in a subject in need thereof, wherein the composition comprises a mixture of one or more of the disclosed aspartic protease inhibitors and an optional pharmaceutically acceptable carrier.

"Pharmaceutically acceptable carrier" means compounds and compositions that are of sufficient purity and quality for use in the formulation of a composition of the invention that, when appropriately administered to an animal or human, do not produce an adverse reaction, and that are used as a vehicle for a drug substance (i.e. aspartic protease inhibitors of the present invention).

""Pharmaceutically acceptable diluent" means compounds and compositions that are of sufficient purity and quality for use in the formulation of a composition of the invention that, when appropriately administered to an animal or human, do not produce an adverse reaction, and that are used as a diluting agent for a drug substance (i.e. aspartic protease inhibitors of the present invention).

"Aspartic protease mediated disorder or disease" includes disorders or diseases associated with the elevated expression or overexpression of aspartic proteases and conditions that accompany such diseases.

An embodiment of the invention includes administering an aspartic protease inhibitor disclosed herein in a combination therapy (see USP 5,821,232, USP 6,716,875, USP 5,663,188, Fossa, A. A.; DePasquale, M. J.; Ringer, L. J.; Winslow, R. L. "Synergistic effect on reduction in blood pressure with coadministration of a renin inhibitor or an angiotensin-converting enzyme inhibitor with an angiotensin II receptor antagonist" Drug Development Research 1994, 33(4), 422-8, the aforementioned article and patents are hereby incorporated by reference) with one or more additional agents for the treatment of hypertension including α-blockers, β-blockers, calcium channel blockers, diuretics, natriuretics, saluretics, centrally acting antiphypertensives, angiotensin converting enzyme (ACE) inhibitors, dual ACE and neutral endopeptidase (NEP) inhibitors, angiotensin-receptor blockers (ARBs), aldosterone synthase inhibitor, aldosterone-receptor antagonists, or endothelin receptor antagonist.

α-Blockers include doxazosin, prazosin, tamsulosin, and terazosin.

β-Blockers for combination therapy are selected from atenolol, bisoprol, metoprolol, acetutolol, esmolol, celiprolol, taliprolol, acebutolol, oxprenolol, pindolol, propanolol, bupranolol, penbutolol, mepindolol, carteolol, nadolol, carvedilol, and their pharmaceutically acceptable salts.

Calcium channel blockers include dihydropyridines (DHPs) and non-DHPs. The preferred DHPs are selected from the group consisting of amlodipine, felodipine, ryosidine, isradipine, lacidipine, nicardipine, nifedipine, nigulpidine, niludipine, nimodiphine, nisoldipine, nitrendipine, and nivaldipine and their pharmaceutically acceptable salts. Non-DHPs are selected from flunarizine, prenylamine, diltiazem, fendiline, gallopamil, mibefradil, anipamil, tiapamil, and verampimil and their pharmaceutically acceptable salts.

A diuretic is, for example, a thiazide derivative selected from amiloride, chlorothiazide, hydrochlorothiazide, methylchlorothiazide, and chlorothalidon.

Centrally acting antiphypertensives include clonidine, guanabenz, guanfacine and methyldopa.

ACE inhibitors include alacepril, benazepril, benazaprilat, captopril, ceronapril, cilazapril, delapril, enalapril, enalaprilat, fosinopril, lisinopril, moexipiril, moveltopril, perindopril, quinapril, quinaprilat, ramipril, ramiprilat, spirapril, temocapril, trandolapril, and zofenopril. Preferred ACE inhibitors are benazepril, enalpril, lisinopril, and ramipril.

Dual ACE/NEP inhibitors are, for example, omapatrilat, fasidotril, and fasidotrilat.

Preferred ARBs include candesartan, eprosartan, irbesartan, losartan, olmesartan, tasosartan, telmisartan, and valsartan.

Preferred aldosterone synthase inhibitors are anastrozole, fadrozole, and exemestane.

Preferred aldosterone-receptor antagonists are spironolactone and eplerenone.

A preferred endothelin antagonist is, for example, bosentan, enrasentan, atrasentan, darusentan, sitaxentan, and tezosentan and their pharmaceutically acceptable salts.

An embodiment of the invention includes administering an aspartic protease inhibitor disclosed herein or composition thereof in a combination therapy with one or more additional agents for the treatment of AIDS reverse transcriptase inhibitors, non-nucleoside reverse transcriptase inhibitors, other HIV protease inhibitors, HIV integrase inhibitors, entry inhibitors (including attachment, co-receptor and fusion inhibitors), antisense drugs, and immune stimulators..

Preferred reverse transcriptase inhibitors are zidovudine, didanosine, zalcitabine, stavudine, lamivudine, abacavir, tenofovir, and emtricitabine.

Preferred non-nucleoside reverse transcriptase inhibitors are nevirapine, delaviridine, and efavirenz.

Preferred HIV protease inhibitors are saquinavir, ritonavir, indinavir, nelfinavir, amprenavir, lopinavir, atazanavir, and fosamprenavir.

Preferred HIV integrase inhibitors are L-870,810 and S-1360.

Entry inhibitors include compounds that bind to the CD4 receptor, the CCR5 receptor or the CXCR4 receptor. Specific examples of entry inhibitors include enfuvirtide (a peptidomimetic of the HR2 domain in gp41) and sifurvitide.

A preferred attachment and fusion inhibitor is enfuvirtide.

An embodiment of the invention includes administering an aspartic protease inhibitor disclosed herein or composition thereof in a combination therapy with one or more additional agents for the treatment of Alzheimer's disease including tacrine, donepezil, rivastigmine, galantamine, and memantine.

An embodiment of the invention includes administering an aspartic protease inhibitor disclosed herein or composition thereof in a combination therapy with one or more additional agents for the treatment of malaria including artemisinin, chloroquine, halofantrine, hydroxychloroquine, mefloquine, primaquine, pyrimethamine, quinine, sulfadoxine.

Combination therapy includes co-administration of an aspartic protease inhibitor disclosed herein and said other agent, sequential administration of the disclosed aspartic protease inhibitor and the other agent, administration of a composition containing the aspartic protease inhibitor and the other agent, or simultaneous administration of separate compositions containing the aspartic protease inhibitor and the other agent.

The invention further includes the process for making the composition comprising mixing one or more of the disclosed aspartic protease inhibitors and an optional pharmaceutically acceptable carrier; and includes those compositions resulting from such a process, which process includes conventional pharmaceutical techniques. For example, an aspartic protease inhibitor disclosed herein may be nanomilled prior to formulation. An aspartic protease inhibitor disclosed herein may also be prepared by grinding, micronizing or other particle size reduction methods known in the art. Such methods include, but are not limited to, those described in U.S. Pat. Nos. 4,826,689, 5,145,684, 5,298,262, 5,302,401, 5,336,507, 5,340,564, 5,346,702, 5,352,459, 5,354,560, 5,384,124, 5,429,824, 5,503,723, 5,510,118, 5,518,187, 5,518,738, 5,534,270, 5,536,508, 5,552,160, 5,560,931, 5,560,932, 5,565,188, 5,569,448, 5,571,536, 5,573,783, 5,580,579, 5,585,108, 5,587,143, 5,591,456, 5,622,938, 5,662,883, 5,665,331, 5,718,919, 5,747,001, PCT applications WO 93/25190, WO 96/24336, and WO 98/35666. The pharmaceutical compositions of the invention may be prepared using techniques and methods known to those skilled in the art. Some of the methods commonly used in the art are described in Remington's Pharmaceutical Sciences (Mack Publishing Company),

The compositions of the invention include ocular, oral, nasal, transdermal, topical with or without occlusion, intravenous (both bolus and infusion), and injection (intraperitoneally, subcutaneously, intramuscularly, intratumorally, or parenterally). The composition may be in a dosage unit such as a tablet, pill, capsule, powder, granule, liposome, ion exchange resin, sterile ocular solution, or ocular delivery device (such as a contact lens and the like facilitating immediate release, timed release, or sustained release), parenteral solution or suspension, metered aerosol or liquid spray, drop, ampoule, auto-injector device, or suppository; for administration ocularly, orally, intranasally, sublingually, parenterally, or rectally, or by inhalation or insufflation.

Compositions of the invention suitable for oral administration include solid forms such as pills, tablets, caplets, capsules (each including immediate release, timed release, and sustained release formulations), granules and powders; and, liquid forms such as solutions, syrups, elixirs, emulsions, and suspensions. Forms useful for ocular administration include sterile solutions or ocular delivery devices. Forms useful for parenteral administration include sterile solutions, emulsions, and suspensions.

The dosage form containing the composition of the invention contains an effective amount of the drug substance (i.e. aspartic protease inhibitors of the present invention) necessary to provide a therapeutic and/or prophylactic effect. The composition may contain from about 5,000 mg to about 0.5 mg (preferably, from about 1,000 mg to about 0.5 mg) of a disclosed aspartic protease inhibitor or salt form thereof and may be constituted into any form suitable for the selected mode of administration. The compositions of the invention may be administered in a form suitable for once-weekly or once-monthly administration. For example, an insoluble salt of the drug substance (i.e. aspartic protease inhibitors of the present invention) may be adapted to provide a depot preparation for intramuscular injection (e.g., a decanoate salt) or to provide a solution for ophthalmic administration. Daily administration or post-periodic dosing may also be employed, wherein the composition may be administered about 1 to about 5 times per day.

For oral administration, the composition is preferably in the form of a tablet or capsule containing, e.g., 1000 to 0.5 milligrams of the drug substance (i.e. aspartic protease inhibitors of the present invention), more specifically 500 mg to 5 mg. Dosages will vary depending on factors associated with the particular patient being treated (e.g., age, weight, diet, and time of administration), the severity of the condition being treated, the compound being employed, the mode of administration, and the strength of the preparation.

The oral composition is preferably formulated as a homogeneous composition, wherein the drug substance (i.e. aspartic protease inhibitors of the present invention) is dispersed evenly throughout the mixture, which may be readily subdivided into dosage units containing equal amounts of a disclosed aspartic protease inhibitor. Preferably, the compositions are prepared by mixing a disclosed aspartic protease inhibitor with one or more optionally present pharmaceutical carriers (such as a starch, sugar, diluent, granulating agent, lubricant, glidant, binding agent, and disintegrating agent), one or more optionally present inert pharmaceutical excipients (such as water, glycols, oils, alcohols, flavoring agents, preservatives, coloring agents, and syrup), one or more optionally present conventional tableting ingredients (such as corn starch, lactose, sucrose, sorbitol, talc, stearic acid, magnesium stearate, dicalcium phosphate, and any of a variety of gums), and an optional diluent (such as water).

Binding agents include starch, gelatin, natural sugars (e.g., glucose and beta-lactose), corn sweeteners and natural and synthetic gums (e.g., acacia and tragacanth). Disintegrating agents include starch, methyl cellulose, agar, and bentonite.

Tablets and capsules represent an advantageous oral dosage unit form. Tablets may be sugarcoated or filmcoated using standard techniques. Tablets may also be coated or otherwise compounded to provide a prolonged, control-release therapeutic effect. The dosage form may comprise an inner dosage and an outer dosage component, wherein the outer component is in the form of an envelope over the inner component. The two components may further be separated by a layer which resists disintegration in the stomach (such as an enteric layer) and permits the inner component to pass intact into the duodenum or a layer which delays or sustains release. A variety of enteric and non-enteric layer or coating materials (such as polymeric acids, shellacs, acetyl alcohol, and cellulose acetate or combinations thereof) may be used.

The disclosed aspartic protease inhibitors may also be administered via a slow release composition, wherein the composition includes a disclosed aspartic protease inhibitor and a biodegradable slow release carrier (e.g., a polymeric carrier) or a pharmaceutically acceptable non-biodegradable slow release carrier (e.g., an ion exchange carrier).

Biodegradable and non-biodegradable slow release carriers are well known in the art. Biodegradable carriers are used to form particles or matrices which retain drug substance(s) (i.e. aspartic protease inhibitors of the present invention) and which slowly degrade/dissolve in a suitable environment (e.g., aqueous, acidic, basic and the like) to release drug substances. Such particles degrade/dissolve in body fluids to release the drug substance(s) (i.e. aspartic protease inhibitors of the present invention) therein. The particles are preferably nanoparticles (e.g., in the range of about 1 to 500 run in diameter, preferably about 50-200 nm in diameter, and most preferably about 100 nm in diameter). In a process for preparing a slow release composition, a slow release carrier and a disclosed aspartic protease inhibitor are first dissolved or dispersed in an organic solvent. The resulting mixture is added into an aqueous solution containing an optional surface-active agent(s) to produce an emulsion. The organic solvent is then evaporated from the emulsion to provide a colloidal suspension of particles containing the slow release carrier and the disclosed aspartic protease inhibitor.

The disclosed aspartic protease inhibitors may-be incorporated for administration orally or by injection in a liquid form, such as aqueous solutions, suitably flavored syrups, aqueous or oil suspensions, flavored emulsions with edible oils such as cottonseed oil, sesame oil, coconut oil or peanut oil and the like, or in elixirs or similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone, and gelatin. The liquid forms in suitably flavored suspending or dispersing agents may also include synthetic and natural gums. For parenteral administration, sterile suspensions and solutions are desired. Isotonic preparations, which generally contain suitable preservatives, are employed when intravenous administration is desired.

The disclosed aspartic protease inhibitors may be administered parenterally via injection. A parenteral formulation may consist of the drug substance (i.e. aspartic protease inhibitors of the present invention) dissolved in or mixed with an appropriate inert liquid carrier. Acceptable liquid carriers usually comprise aqueous solvents and other optional ingredients for aiding solubility or preservation. Such aqueous solvents include sterile water, Ringer's solution, or an isotonic aqueous saline solution. Other optional ingredients include vegetable oils (such as peanut oil, cottonseed oil, and sesame oil), and organic solvents (such as solketal, glycerol, and formyl). A sterile, non-volatile oil may be employed as a solvent or suspending agent. The parenteral formulation is prepared by dissolving or suspending the drug substance (i.e. aspartic protease inhibitors of the present invention) in the liquid carrier whereby the final dosage unit contains from 0.005 to 10% by weight of the drug substance (i.e. aspartic protease inhibitors of the present invention). Other additives include preservatives, isotonizers, solubilizers, stabilizers, and pain-soothing agents. Injectable suspensions may also be prepared, in which case appropriate liquid carriers, suspending agents and the like may be employed.

The disclosed aspartic protease inhibitors may be administered intranasally using a suitable intranasal vehicle.

The disclosed aspartic protease inhibitors may also be administered topically using a suitable topical transdermal vehicle or a transdermal patch.

For ocular administration, the composition is preferably in the form of an ophthalmic composition. The ophthalmic compositions are preferably formulated as eye-drop formulations and filled in appropriate containers to facilitate administration to the eye, for example a dropper fitted with a suitable pipette. Preferably, the compositions are sterile and aqueous based, using purified water. In addition to the disclosed aspartic protease inhibitor, an ophthalmic composition may contain one or more of: a) a surfactant such as a polyoxyethylene fatty acid ester; b) a thickening agents such as cellulose, cellulose derivatives, carboxyvinyl polymers, polyvinyl polymers, and polyvinylpyrrolidones, typically at a concentration n the range of about 0.05 to about 5.0% (wt/vol); c) (as an alternative to or in addition to storing the composition in a container containing nitrogen and optionally including a free oxygen absorber such as Fe), an anti-oxidant such as butylated hydroxyanisol, ascorbic acid, sodium thiosulfate, or butylated hydroxytoluene at a concentration of about 0.00005 to about 0.1% (wt/vol); d) ethanol at a concentration of about 0.01 to 0.5% (wt/vol); and e) other excipients such as an isotonic agent, buffer, preservative, and/or pH-controlling agent. The pH of the ophthalmic composition is desirably within the range of 4 to 8.

The invention is further defined by reference to the examples, which are intended to be illustrative and not limiting.

Representative compounds of the invention can be synthesized in accordance with the general synthetic schemes described above and are illustrated in the examples that follow. The methods for preparing the various starting materials used in the schemes and examples are well within the knowledge of persons skilled in the art.

The following abbreviations have the indicated meanings:

| Abbreviation | Meaning |
|---|---|
| Aq | aqueous |
| Boc | *tert*-butoxy carbonyl or *t*-butoxy carbonyl |
| (Boc)₂O | di-*tert*-butyl dicarbonate |
| Brine | saturated aqueous NaCl |
| Cbz | Benzyloxycarbonyl |
| CbzCl | Benzyl chloroformate |
| CDI | carbonyl diimidazole |
| CH₂Cl₂ | methylene chloride |
| CH₃CN or MeCN | acetonitrile |
| Cpd | compound |
| d | day |
| DAST | diethylaminosulfur trifluoride |
| DBU | 1,8-diazabicyclo[5.4.0]undec-7-ene |
| DCC | N,N'-dicyclohexylcarbodiimide |
| DCM | dichloromethane |
| DCU | N,N'-dicyclohexylurea |
| DIAD | diisopropyl azodicarboxylate |
| DiBAIH | Diisobutylaluminum hydride |
| DIEA | N,N-diisopropylethylamine |
| DMAP | 4-(dimethylamino)pyridine |
| DMF | N,N-dimethylformamide |
| DMPU | 1,3-dimethyl-3,4,5,6-tetrahydro-2(1H)-pyrimidinone |
| 2,4-DNP | 2,4-dinitrophenylhydrazine |
| EDCI•HCl | 1-[3-(dimethylamino)propyl]-3-ethylcarbodiimide hydrochloride |
| Equiv | equivalents |
| Et | ethyl |
| Et₂O | ethyl ether |
| EtOAc | ethyl acetate |
| Fmoc | 1-[[(9H-fluoren-9-ylmethoxy)carbonyl]oxy]- |
| Fmoc-OSu | 1-[[(9H-fluoren-9-ylmethoxy)carbonyl]oxy]-2,5-pyrrolidinedione |
| h, hr | hour |
| HOBt | 1-hydroxybenzotriazole |
| HATU | 2-(7-Aza-1H-benzotriazole-1-yl)-1,1,3,3-tetramethyluronium hexafluorophosphate |
| HBTU | 2-(1H-Benzotriazole-1-yl)-1,1,3,3-tetramethyluronium hexafluorophosphate |
| KHMDS | potassium hexamethyldisilazane |
| LiHMDS | lithium hexamethyldisilazane |
| LAH or LiAIH₄ | lithium aluminum hydride |
| LC-MS | liquid chromatography-mass spectroscopy |
| LHMDS | lithium hexamethyldisilazane |
| Me | methyl |
| MeCN | acetonitrile |
| MeOH | methanol |
| MsCl | methanesulfonyl chloride |
| min | minute |
| MS | mass spectrum |
| NaH | sodium hydride |
| NaHCO₃ | sodium bicarbonate |
| NaN₃ | sodium azide |
| NaOH | sodium hydroxide |
| Na₂SO₄ | sodium sulfate |
| NMM | N-methylmorpholine |
| NMP | N-methylpyrrolidinone |
| Pd₂(dba)₃ | tris(dibenzylideneacetone)dipalladium(0) |
| PE | petroleum ether |
| Ph | phenyl |
| PTSA | p-toluene sulfonic acid |
| Quant | quantitative yield |
| rt | room temperature |
| Satd | saturated |
| SOCl₂ | thionyl chloride |
| SPE | solid phase extraction |
| TBS | t-butyldimethylsilyl |
| TBSCl | t-butyldimethylsilyl chloride |
| TEA | triethylamine or Et₃N |
| TEAF | tetraethylammonium fluoride |
| TEMPO | 2,2,6,6-tetramethyl-1-piperidinyloxy free radical |
| Teoc | 1-[2-(trimethytsilyl)ethoxycarbonyl] |
| Teoc-OSu | 1-[2-(trimethylsilyl)ethoxycarbonyloxy]pyrrolidin-2,5-dione |
| TFA | trifluoroacetic acid |
| THF | tetrahydrofuran |
| tlc | thin layer chromatography |
| TMS | trimethylsilyl |
| TMSCI | chlorotrimethylsilane or trimethylsilyl chloride |
| *t*_{R} | retention time |
| TsOH | p-toluenesulfonic acid |
| TsCl | p-toluenesulfonyl chloride |

### EXAMPLE 1

The compounds of present invention can be synthesized by coupling a pyran intermediate represented by the following structure: 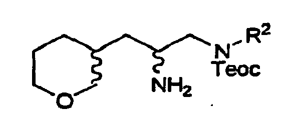 with a piperidine intermediate represented by the following structure: 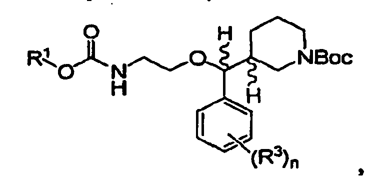 described in the following scheme:

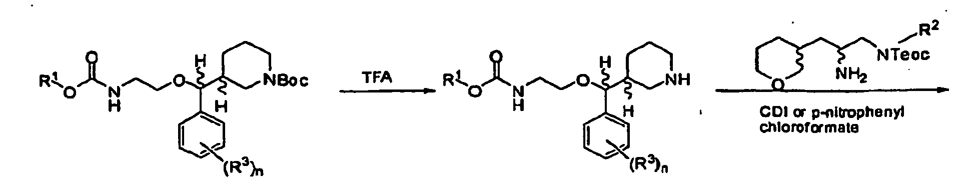

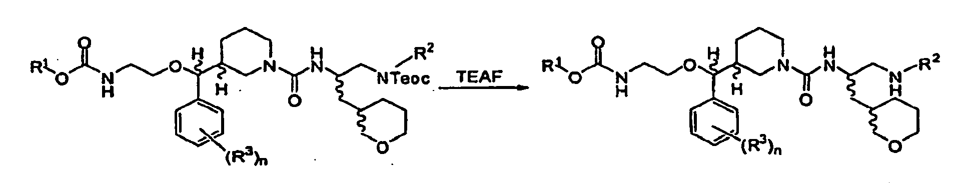

### Preparation of the Pyran Intermediate from Glutamic Ester

The pyran intermediate can be prepared from glutamic ester using the following synthetic scheme:

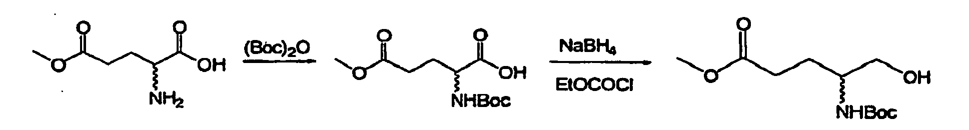

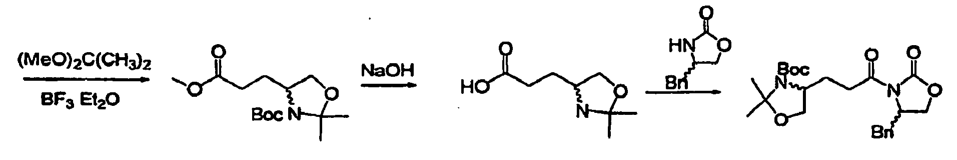

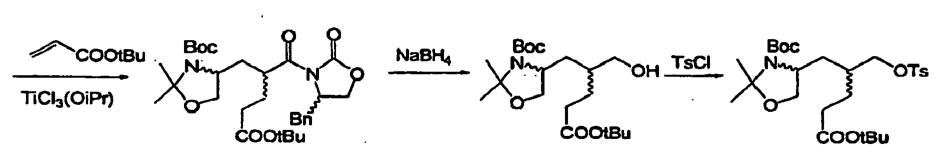

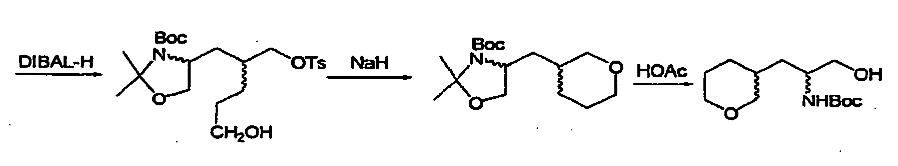

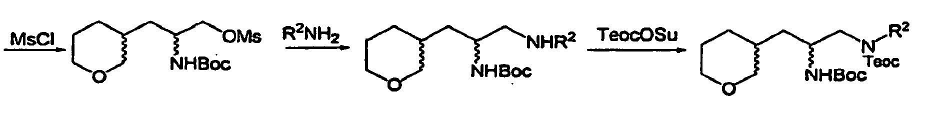

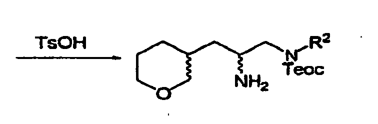

### Preparation of the Plan Intermediate from Pyroglutamic Ester

The pyran intermediate can also be prepared from pyroglutamic ester using the following synthetic scheme:

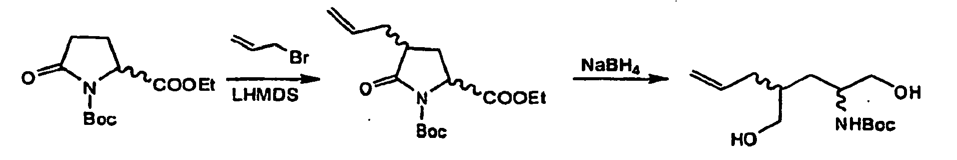

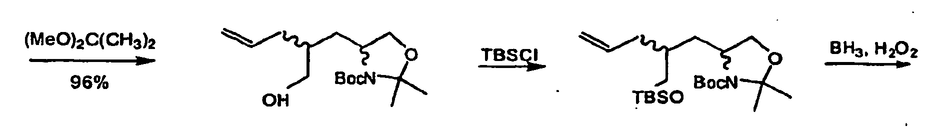

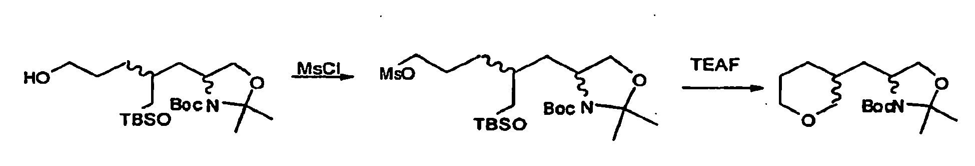

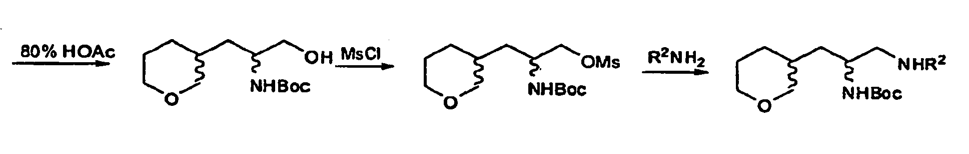

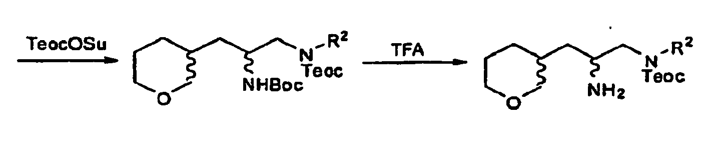

### Preparation of the Piperidine Intermediate

The piperidine intermediate can be prepared by using the following synthetic scheme.

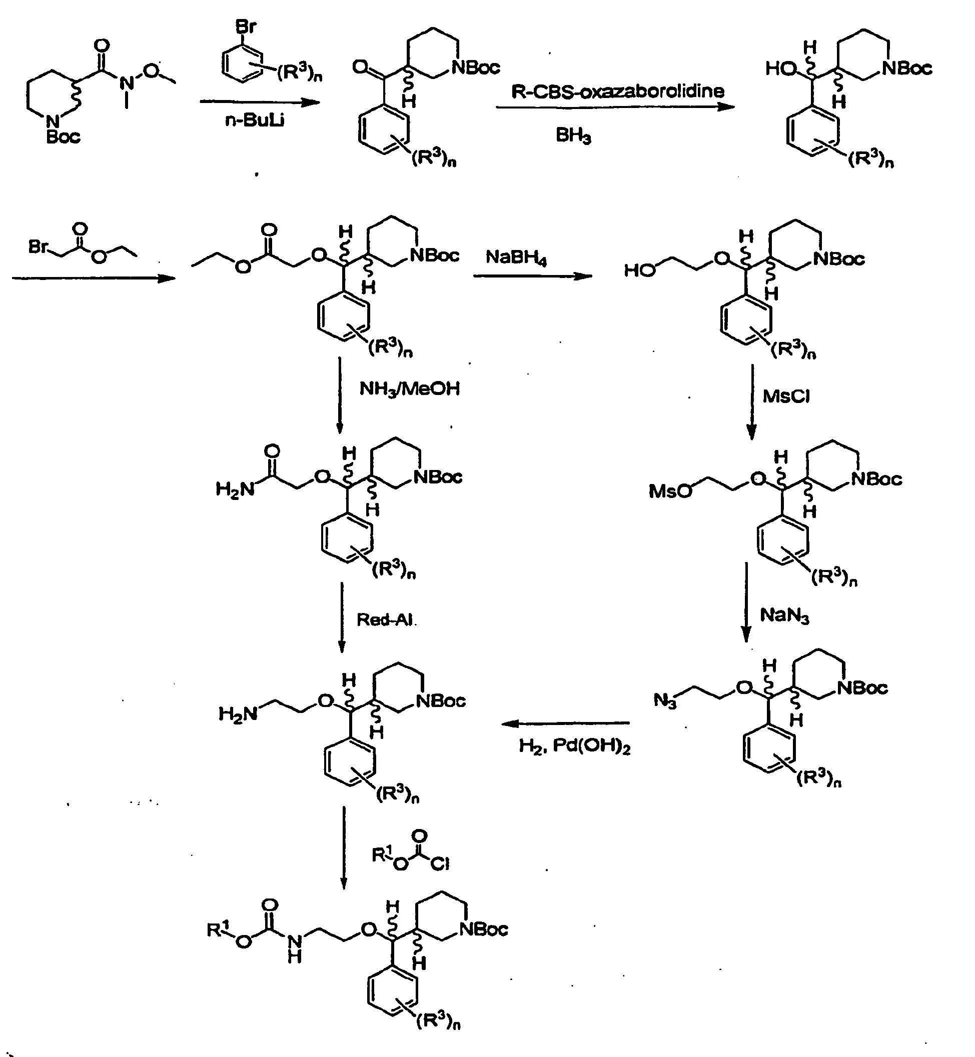

Alternatively, the piperidine intermediate can be prepared using the following synthetic scheme:

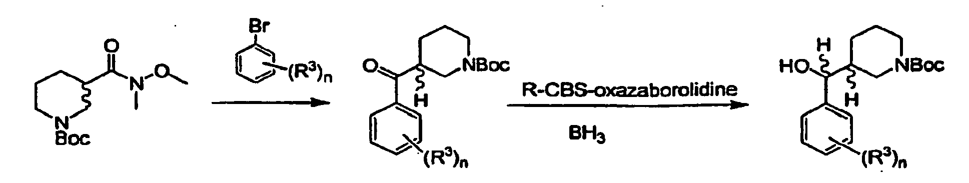

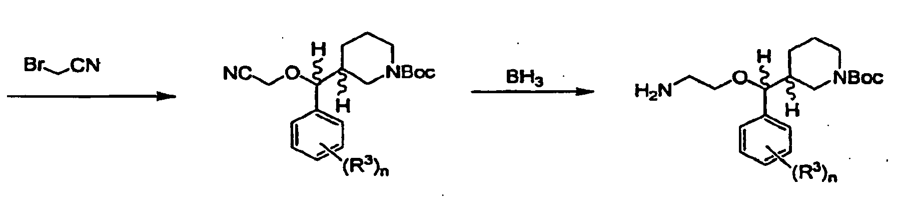

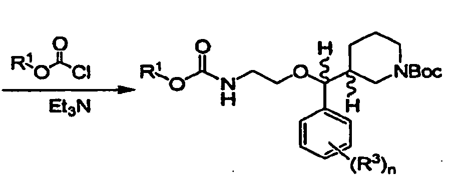

Specific conditions for synthesizing the disclosed aspartic protease inhibitors according to the above schemes are provided in Examples 2-18.

### EXAMPLE 2

### (R)-tert-butyl 3-((R)-(3-chlorophenyl)(2-(methoxycarbonylamino)ethoxy)methyl)piperidine-1-carboxylate

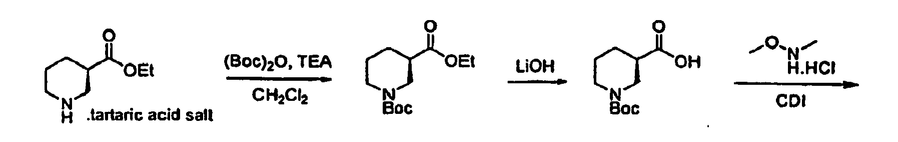

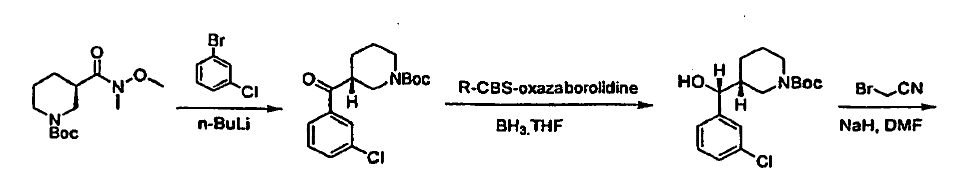

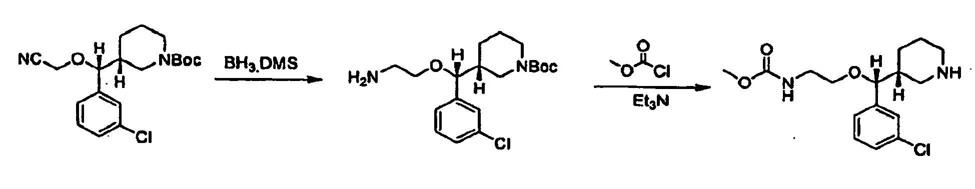

### Step 1. (R)-1-tert-butyl 3-ethyl piperidine-1,3-dicarboxylate

To a 20 L of round bottom flask was placed (*R*)-ethyl piperidine-3-carboxylate tartaric acid salt (2.6 kg, 8.47 mol, 1eq) and CH₂Cl₂ (14 L). To the above solution, at 0 °C was added TEA (2.137 kg, 21.17 mol, 2.5 eq), followed by drop wise addition of (Boc)₂O (2.132 kg, 9.74 mol, 1.15 eq). The mixture was allowed to stir overnight at room temperature. The mixture was washed with saturated citric acid solution (3x2.5 L), saturated NaHCO₃ solution (3x2.5 L) and brine (2x2 L). The organic phase was dried over Na₂SO₄, filtered and the filtrate was evaporated to give colorless oil (2.2 kg, yield 100%).

### Step 2. (R)-1-(tert-butoxycarbonyl)piperidine-3-carboxylic acid

To a solution of (*R*)-1-*tert*-butyl 3-ethyl piperidine-1,3-dicarboxylate (2.2 kg, 8.469 mol, 1 eq) in 5 L of MeOH was added a solution of LiOH (629.6 g, 15 mol, 1.77 eq) in 7.5 L of water at 0-5 °C. After addition, the mixture was stirred overnight at room temperature. TLC showed the starting material was consumed. The pH of the system was adjusted to 7 by addition of saturated citric acid solution. Most of the methanol was removed. The pH was adjusted to 4-5 with citric acid. The mixture was extracted 3 times with 5 L of CH₂Cl₂, the organic layers were combined and dried over Na₂SO₄ and evaporated to afford a white solid (1.775 kg, 92%).

### Step 3. (R)-tert-butyl 3-(methoxy(methyl)carbamoyl)piperidine-1-carboxylate

To a stirred solution of (*R*)-1-(*tert*-butoxycarbonyl)piperidine-3-carboxylic acid (233 g, 1.2 mol) in THF (1.2 L) was added carbonyldiimidazole (230 g, 1.42 mol). The mixture was stirred for 1 h under ice-water bath. A suspension of triethylamine (207 mL, 1.41 mol) and *N*, *O*-dimethylhydroxylamine hydrochloride (138 g, 1.42 mol) in THF (900 mL) was added. The reaction mixture was allowed to warm to room temperature and stirred overnight. TLC showed the reaction was complete. The solvent was evaporated, and the residue was dissolved in CH₂Cl₂ (1.2 L) and washed successively with 0.5 N hydrochloride solution, saturated solution of sodium carbonate and brine, dried over anhydrous sodium sulfate and evaporated to give crude compound (*R*)-*tert-*butyl 3-(methoxy(methyl)carbamoyl)piperidine-1-carboxylate (250 g, 91 %), which was used in the next step directly without purification. ¹H NMR (400MHz, CDCl₃): 4.05-4.19 (m, 2H), 3.72 (s, 3H), 3.17 (s, 3H), 2.75-2.85 (m, 2H), 2.65 (t, 1H), 1.90 (d, 1H), 1.60-1.78 (m, 2H), 1.44 (s, 9H).

### Step 4. (R)-tert-butyl3-(3-chlorobenzoyl)piperidine-1-carboxylate

To a solution of 1-bromo-3-chlorobenzene (54.3 g, 0.286 mol) in anhydrous THF (500 mL) at -78 °C under nitrogen was added drop wise a solution of 2.5 M n-BuLi in hexane (114 mL, 0.286 mol). After stirring for 1 hr at-78 °C, a solution of (*R*)-*tert*-butyl 3-(methoxy(methyl)carbamoyl)piperidine-1-carboxylate (65.8 g, 0.242 mol) in anhydrous THF (300 mL) was added drop wise. After addition, the reaction mixture was allowed to warm to room temperature and stirred for 2 h. TLC indicated the reaction was complete. The mixture was quenched with saturated NH₄Cl solution (300 mL) and extracted with ethyl acetate (3×200 mL). The combined organic layers were washed with brine, dried over Na₂SO₄ and concentrated *in vacuo* to give the crude product (*R*)-*tert*-butyl 3-(3-chlorobenzoyl)piperidine-1-carboxylate (92 g, 100%), which was used immediately for next step without purification.

### Step 5. (R)-tert-butyl 3-((R)-(3-chlorophenyl)(hydroxy)methyl)piperidine-1-carboxylate

To a solution of (*R*)*-tert*-butyl 3-(3-chlorobenzoyl)piperidine-1-carboxylate (92 g, 0.286 mol) in anhydrous THF (300 mL) at -15 °C under nitrogen was added drop wise a solution of 1 M *R*-CBS-oxazaborolidine in toluene (45 mL, 45 mmol, 0.15 eq). After stirring for 1 hr at -15 °C, a solution of 10 M BH₃ in THF (33 mL, 0.33 mol, 1.1 eq) was added drop wise. After addition, the reaction mixture was stirred for 2 h at -15 °C. TLC indicated the starting material was consumed. Methanol (200 mL) was added drop wise carefully at -15 °C. The solvent was removed under reduced pressure, the residue was purified by column chromatography on silica gel eluting with AcOEt/hexane (1:30→1:15) to provide a light yellow oil (82 g, HPLC≥70%, ratio≥3:1). The mixture was dissolved in ethyl acetate until the alcohol was just dissolved (about 5mL/1g), the solvent was removed on the rotary evaporator until a few of crystals appeared. The solution was cooled to room temperature slowly and stood for 1-2 h. To the above solution was added hexane (about 300 mL) and then filtered , the crystals were washed with cool hexane and recrystallized another two times to afford (*R*)-*tert*-butyl 3-((*R*)-(3-chlorophenyl)(hydroxy)methyl)piperidine-1-carboxylate as the pure isomer (32.5 g, ee.≥99%, yield 35% for two steps).

### Step 6. (R)-tert-butyl 3-((R)-(3-chlorophenyl)(cyanomethoxy)methyl)piperidine-1-carboxylate

To a solution of (*R*)-*tert*-butyl 3-((*R*)-(3-chlorophenyl)(hydroxy)methyl)piperidine-1-carboxylate (32.5 g, 0.1 mol), NaH (12 g, 0.3 mol) was added at 0 °C. The mixture was stirred for 1 h at room temperature. The mixture was cooled to -40 °C, then bromoacetonitrile (35.7 g, 0.3 mol) was added drop wise. The mixture was stirred an additional 0.5 h at -20 °C. HPLC indicated the reaction was ~30% complete. The addition of NaH and bromoacetonitrile was repeated two more times. HPLC indicated the reaction was ~60% completed. The reaction was quenched with sat. NH₄Cl. The mixture was extracted with CH₂Cl₂. The organic layer was dried over Na₂SO₄, concentrated to give the crude product as brown oil (36.8 g), which was used for the next step without purification.

### Step 7. (R)-tert-butyl 3-((R)-(2-aminoethoxy)(3-chlorophenyl)methyl)piperidine-1-carboxylate

(*R*)-*tert*-butyl 3-((*R*)-(3-chlorophenyl)(cyanomethoxy)methyl)piperidine-1-carboxylate (36.8 g, 0.10 mol) was dissolved in anhydrous THF (350 mL), and the solution was heated under reflux under a nitrogen atmosphere. A solution of BH₃.Me₂S (30 mL, 0.30 mol) in THF was added drop wise, and stirring was continued under reflux overnight. The resulting solution was cooled to room temperature. The reaction was quenched by careful, drop wise addition of MeOH until bubbling ceased. After evaporation of the solution, the crude product was obtained (70 g), which was used for the next step without purification.

### Step 8. (R)-tert-butyl 3-((R)-(3-chlorophenyl)(2-(methoxycarbonylamino)ethoxy)methyl)piperidine-1-carboxylate

To a solution of (*R*)-*tert*-butyl 3-((*R*)-(2-aminoethoxy)(3-chlorophenyl)methyl)piperidine-1-carboxylate (70 g, crude, 0.1 mol) and DMAP (1.83 g, 15 mmol, 0.15 eq) in dry CH₂Cl₂ (150 mL), Et₃N (12.1 g, 15.8 mL, 120 mmol) was added. The resulting mixture was cooled to 0~5 °C using a ice-water bath, a solution of methyl chloroformate (11.28 g, 120 mmol, 1.2 eq) in dry CH₂Cl₂ (100 mL) was added drop wise. After addition, the reaction mixture was stirred for 3 h at 0~5 °C. TLC showed the starting material had disappeared. Water (80 mL) was added. The aqueous layer was extracted with CH₂Cl₂ (3×100 mL), the combined organic layers were washed with 10% citric acid (2×150 mL) and brine (100 mL), then dried over Na₂SO₄, filtered and concentrated to the crude product, which was purified by preparative HPLC to afford (*R*)-*tert*-butyl 3-((*R*)-(3-chlorophenyl)(2-(methoxycarbonylamino)ethoxy)methyl)piperidine-1-carboxylate (10.7 g, the total yield for three steps is 25%). ¹H NMR (400MHz, CDCl₃):1.12-1.40(m, 4H), 1.43(s, 9H), 1.64(m, 2H), 2.82(m, 2H), 3.25(m, 2H), 3.61(s, 3H), 3.74(m, 1H), 4.05(m, 1H), 4.16(m, 1H), 7.22 (m, 1H), 7.32 (m, 3H).

### Step 9. methyl 2-((R)-(3-chlorophenyl)((R)-piperidin-3-yl)methoxy)ethylcarbamate

(*R*)-*tert*-butyl 3-((*R*)-(3-chlorophenyl)(2-(methoxycarbonylamino)ethoxy)-methyl)piperidine-1-carboxylate (10.7 g, 25 mmol) was dissolved in a solution of 20% (V/V) TFA/CH₂Cl₂ (150 mL). The reaction mixture was stirred at room temperature for 1 h. TLC showed the reaction was completed. A solution of saturated sodium bicarbonate was added drop wise to adjust pH 8-9. The resulting mixture was extracted with CH₂Cl₂ (3×200 mL), washed with brine, dried over Na₂SO₄, concentrated *in vacuo* to afford methyl 2-((*R*)-(3-chlorophenyl)((*R*)-piperidin-3-yl)methoxy)ethylcarbamate (11.2 g, 100%), which was used for next steep directly without purification.

Alternatively, (*R*)-*tert*-butyl 3-((*R*)-(3-chlorophenyl)(2-(methoxycarbonylamino)ethoxy)methyl)piperidine-1-carboxylate may be prepared by the following procedures:

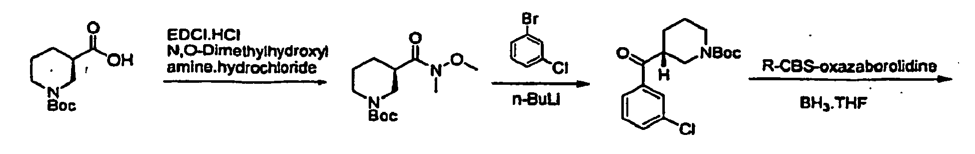

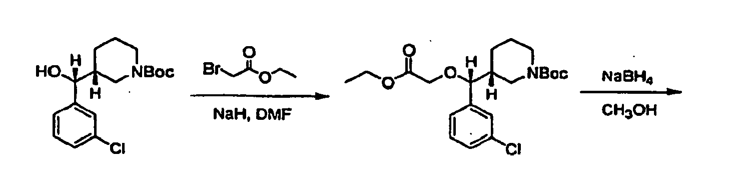

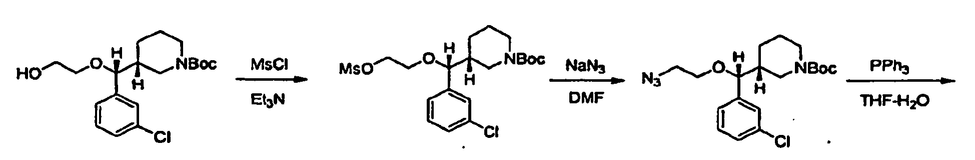

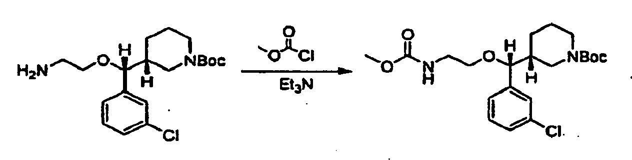

### Step 1. (R)-tert-butyl 3-(methoxy(methyl)carbamoyl)piperidine-1-carboxylate

(*R*)-1-(*tert*-Butoxycarbonyl)piperidine-3-carboxylic acid (25 g, 0.11 mol, 1.0 equiv), N,O-dimethylhydroxylamine hydrochloride, (10.5 g, 0.14 mol, 1.25 equiv) and EDCI.HCl (26.3 g, 0.14 mol, 1.25 equiv) and diisopropylethylamine (48 mL, 0.28 mol, 2.5 equiv) were dissolved in CH₂Cl₂ (400 mL) and stirred overnight at rt. The reaction mixture was diluted with EtOAc, washed with 5% aq HCl (2 X 150mL), satd aq NaHCO₃ (150 mL), brine (100 mL), and dried over Na₂SO₄. Concentration afforded (*R*)-*tert*-butyl 3-(methoxy(methyl)carbamoyl)piperidine-1-carboxylate (24.42 g, 82%) as a clear oil. The crude product was used for next step without further purification. MS ESI +ve m/z 295 (M+Na). ¹H NMR (CDCl₃) δ 4.19-4.00 (m, 2H), 3.77 (m, 3H), 3.12 (s, 3H), 2.79 (m, 2H), 2.64 (m, 1H), 1.89 (m, 1H), 1.71-1.52 (m, 2H), 1.51-1.33 (m, 10H).

### Step 2. (R)-tert-butyl 3-(3-chlorobenzoyl)piperidine-1-carboxylate

To a solution of 1-bromo-3-chlorobenzene (100 g, 0.52 mol) in anhydrous THF (550 mL) at -78 °C under nitrogen was added dropwise a solution of 2.5 M *n-*BuLi in hexane (210 mL, 0.52 mol). After stirring for 1 hr at -78 °C, a solution of (*R*)-*tert*-butyl 3-(methoxy(methyl)carbamoyl)piperidine-1-carboxylate (120 g, 0.44 mol) in anhydrous THF (500 mL) was added dropwise. After addition, the reaction mixture was allowed to warm to rt and stirred for 2 hr. The mixture was quenched with saturated NH₄Cl solution (500 mL) and extracted with EtOAc (3×400 mL). The combined organic layers were washed with brine, dried over Na₂SO₄ and concentrated *in vacuo* to give the crude (*R*)-*tert*-butyl 3-(3-chlorobenzoyl)piperidine-1-carboxylate (178 g), which was used immediately for next step without purification.

### Step 3. (R)-tert-butyl 3-((R)-(3-chlorophenyl)(hydroxy)methyl)piperidine-1-carboxylate

To a solution of (*R*)*-tert*-butyl 3-(3-chlorobenzoyl)piperidine-1-carboxylate (178 g, 0.55 mol) in anhydrous THF (600 mL) at -15 °C under nitrogen was added dropwise a solution of 1 M *R*-CBS-oxazaborolidine in toluene (82 mL, 82 mmol, 0.15 eq). After stirring for 1 hr at -15 °C, a solution of 10 M BH₃ in THF (60 mL, 0.60 mol, 1.1 eq) was added dropwise. After addition, the reaction mixture was stirred for 2 hr at -15 °C. Methanol (400 mL) was added dropwise carefully at ~15 °C. The solvent was removed under reduced pressure, the residue was purified by column chromatography on silica gel eluting with EtOAc/hexane (1:30→1:15) to provide the light yellow oil (95 g, HPLC≥70%, ratio ≥ 3:1). The mixture was dissolved in EtOAc till the alcohol was just dissolved (about 5mL/1g), the solvent was removed on the rotary evaporator until a few crystals appeared. The solution was cooled to rt slowly and stood for 1-2 hr. To the above solution was added hexane (about 300 mL) and then filtered, the crystals were washed with cool hexane and re-crystallized from EtOAc-hexane twice to afford the pure isomer (*R*)*-tert-*butyl 3-((*R*)-(3-chlorophenyl)(hydroxy)methyl)piperidine-1-carboxylate (20 g, ee ≥ 99%).

### Step 4. (R)-tert-butyl 3-((R)-(3-chlorophenyl)(2-ethoxy-2-oxoethoxy)methyl)piperidine-1-carboxylate

To a suspension ofNaH (7.44 g, 161 mmol) in anhydrous DMF (50 mL) at 0-5 °C was added dropwise a solution of (*R*)-*tert*-butyl 3-((*R*)-(3-chlorophenyl)(hydroxy)methyl)piperidjne-1-carboxylate (17.45 g, 54 mmol) in anhydrous DMF (100 mL), the reaction mixture was stirred for 1 hr at rt. A solution of ethyl bromoacetate (17.82 g, 11.87 mL, 107 mmol) in anhydrous DMF (100 mL) was added dropwise to the above mixture at 0-5 °C. After addition, the reaction mixture was stirred for 2-3 hr at rt. The reaction mixture was poured into saturated aqueous NH₄Cl and EtOAc (1000 mL) was added. The organic layer was washed with water (3×200 mL) and brine, dried over Na₂SO₄, filtered and concentrated in *vacuo.* The residue was purified on silica gel chromatography to afford (*R*)-*tert-*butyl 3-((*R*)-(3-chlorophenyl)(2-ethoxy-2-oxoethoxy)methyl)piperidine-1-carboxylate (14 g, 64% yield).

### Step 5. (R)-tert-butyl 3-((R)-(3-chlorophenyl)(2-hydroxyethoxy)methyl)piperidine-1-carboxylate

To a solution of (*R*)-*tert*-butyl 3-((*R*)-(3-chlorophenyl)(2-ethoxy-2-oxoethoxy)methyl)piperidine-1-carboxylate (14 g, 34 mmol) in MeOH (200 mL) was added NaBH4 (10.35 g, 272 mmol) in portions while the temperature was lower than 40 °C. After addition, the mixture was stirred at rt for 2-3 hr. The solvent was removed in *vacuo* to provide a residue which was partitioned between water and EtOAc. The organic layer was washed with H₂O and brine, dried over Na₂SO₄ and evaporated to give the crude (*R*)-*tert-*butyl 3-((*R*)-(3-chlorophenyl)(2-hydroxyethoxy)methyl)piperidine-1-carboxylate (12.50g), which was used in the next step without purification.

### Step 6. (R)-tert-butyl 3-((R)-(3-chlorophenyl)(2-(methylsulfonyloxy)ethoxy)methyl)piperidine-1-carboxylate

To a solution of (*R*)-*tert-*butyl 3-((*R*)(3-chlorophenyl)(2-hydroxyethoxy)methyl)piperidine-1-carboxylate (12.50 g, 34 mmol) in dry CH₂Cl₂ (150 mL) was added Et₃N (13.74 g, 18.3 mL, 136 mmol, 4 eq) at -5-0 °C. Then a solution of MsCl (7.75 g, 5.16 mL, 68 mmol, 2 eq) in dry CH₂Cl₂ (50 mL) was added dropwise at the same temperature. After addition, it was allowed to warm to rt gradually. Upon reaction completion water (100 mL) was added. The aqueous layer was extracted with CH₂Cl₂ (3x80 mL)), the combined organic layers was washed with 10% citric acid, sat. NaHCO₃ and brine, then dried over Na₂SO₄, filtered and concentrated to give (*R*)-*tert-*butyl 3-((*R*)-(3-chlorophenyl)(2-(methylsulfonyloxy)ethoxy)methyl)piperidine-1-carboxylate (15g), which was used in the next step without purification.

### Step 7. (R)-tert-butyl 3-((R)-(2-azidoethoxy)(3-chlorophenyl)methyl)piperidine-1-carboxylate

(*R*)*-tert*-Butyl 3-((*R*)-(3-chlorophenyl)(2-(methylsulfonyloxy)ethoxy)methyl)piperidine-1-carboxylate (15 g, 34 mmol) was dissolved into anhydrous DMF (150 mL), solid NaN₃ (6.7 g, 102 mmol, 3 eq) was added and the reaction mixture was heated to 80 °C for overnight. The reaction mixture was cooled to rt and EtOAc (500 mL) was added. The organic phase was washed with water (3×100 mL) and brine (2×80 mL), dried over Na₂SO₄ and concentrated in *vacuo* to provide crude (*R*)-*tert*-butyl 3-((*R*)-(2-azidoethoxy)(3-chlorophenyl)methyl)piperidine-1-carboxylate (13.3 g), which was used for next step without purification.

### Step 8. (R)-tert-butyl 3-((R)-(2-aminoethoxy)(3-chlorophenyl)methyl)piperidine-1-carboxylate

(*R*)-*tert*-Butyl 3-((*R*)-(2-azidoethoxy)(3-chlorophenyl)methyl)piperidine-1-carboxylate (13.3 g, 33.8 mmol) was dissolved in THF/H₂O (20:1, 180 mL / 9 mL), triphenylphosphane (36.0 g, 135 mmol) was added in portions. The reaction mixture was stirred overnight at rt. The solvent was removed under reduced pressure to the residue, which was purified on silica gel chromatography to afford (*R*)*-tert*-butyl 3-((*R*)-(2-aminoethoxy)(3-chlorophenyl)metbyl)piperidine-1-carboxylate (10.4 g, purity: HPLC=75%).

### Step 9. (R)-tert-butyl 3-((R)-(3-chlorophenyl)(2-(methoxycarbonylamino)ethoxy)methyl)piperidine-1-carboxylate

To a solution of (*R*)-*tert*-butyl 3-((*R*)-(2-aminoethoxy)(3-chlorophenyl)methyl)piperidine-1-carboxylate (7.7 g, 21 mmol, HPLC=75%) and DMAP (1.27 g, 10 mmol, 0.5 eq) in dry CH₂Cl₂ (120 mL), Et₃N (6.38 g, 8.45 mL, 63 mmol) was added. The resulting mixture was cooled to 0-5 °C under ice-water bath, a solution of methyl chloroformate (8.1 mL, 104.5 mmol, 5 eq) in dry CH₂Cl₂ (50 mL) was added dropwise. After addition, the reaction mixture was stirred for 1-2 hr at 0-5 °C. The reaction was quenched with water (80 mL). The aqueous layer was extracted with CH₂Cl₂ (3×50 mL), the combined organic layers were washed with 10% citric acid (2x80 mL) and brine, then dried over Na₂SO₄, filtered and concentrated to the crude product, which was purified by preparative HPLC to afford (*R*)-*tert*-butyl 3-((*R*)-(3-chlorophenyl)(2-(methoxycarbonylamino)ethoxy)methyl)piperidine-1-carboxylate (4.4 g, HPLC ≥ 98%, the total yield for five steps is 41 %).

The following compounds were prepared following procedures analogous to those described above:
1) (*R*)-*tert-*butyl 3-((*R*)-(3,5-difluorophenyl)(2-(methoxycarbonylamino)ethoxy)methyl)piperidine-1-carboxylate using (3,5-difluorophenyl)lithium in Step 2.

Alternatively, (*R*)-*tert-*butyl 3-((*R*)-(2-aminoethoxy)(3-chlorophenyl)methyl)-piperidine-1-carboxylate may also be prepared by the following procedures:

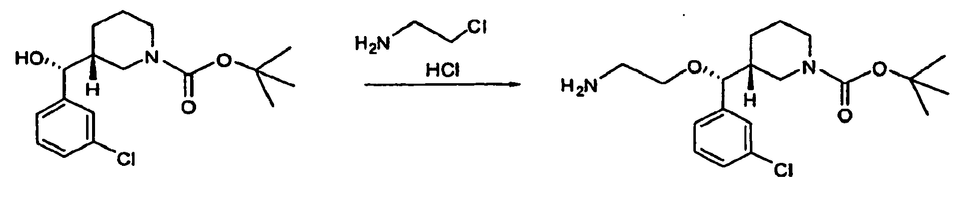

To a solution of (1.00 g, 3.07 mmol) (R)-tert-butyl 3-((R)-(3-chlorophenyl)(hydroxy)methyl)piperidine-1-carboxylate (98:2 diastereomeric ratio) in 10 ml (10 vol) of PhCF₃ was added, sequentially, 8.1 ml (50 eq) of a 50% by weight solution of NaOH in water, tetrabutylammonium hydrogensulfate (0.261 g, 0.25 eq), and chloroethylamine HCl (1.068g, 3 eq), and stirred at 50°C for a period of 20 h. HPLC analysis showed 88% conversion with minor impurities as well as approx. 9% starting alcohol. The reaction was allowed to cool to RT and the layers separate. The addition of 10 vol. of water was needed to ensure the clean separation of the layers. The organic layer was retained and rinsed with 10 vol brine. The organic layer was retained and concentrated under vacuum. The resulting residual oil was dissolved in 10 vol *tert-*butyl methyl ether (TBME) at which point 10 vol of a 20% weight solution of citric acid in water was added. (Note: tartaric acid works as well while acids such as HCl, oxalic acid, TsOH result in deprotection of the NBoc). HPLC analysis showed that clean extraction of the desired amine into the aq. layer had been achieved and the undesired starting alcohol was in the organic layer; the TBME layer was discarded. The aq. layer was rinsed once more with 5 vol of TBME in order to ensure the removal of the undesired starting alcohol. The organic TBME layer was discarded. The aq. layer was brought to a pH of approx. 13 by the addition of 2 vol of 50% weight NaOH in water at which point 10 vol DCM (dichloromethane) was added. Clean extraction of the desired product into the DCM was achieved. The organic extract was rinsed with 10 vol brine (no purification seen by HPLC), dried over NaSO4, and concentrated to afford 750mg (66% yield, 97% purity) of the desired product (confirmed by HPLC/MS and NMR).

Alternatively, (*R*)-*tert-*butyl 3-((*R*)-(3-chlorophenyl)(2-(methoxycarbonylamino)ethoxy)methyl)piperidine-1-carboxylate may also be prepared by the following process:

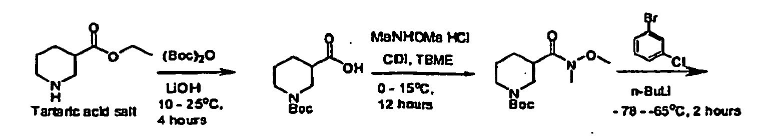

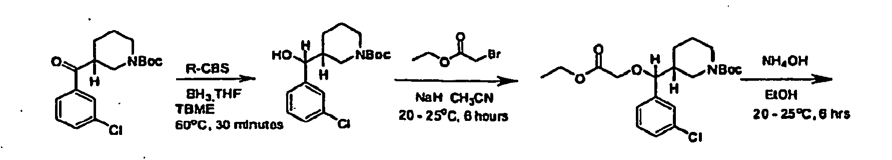

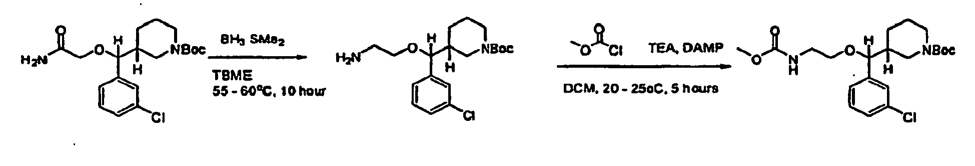

### EXAMPLE 3

### (R)-tert-butyl 3-((R)-(2-(methoxycarbonylamino)ethoxy)(m-tolyl)methyl)piperidine-1-carboxylate

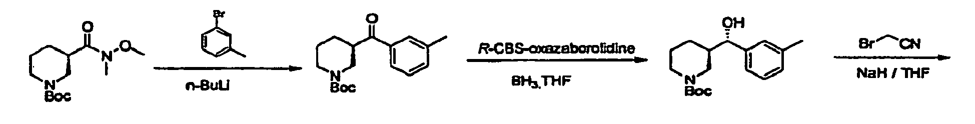

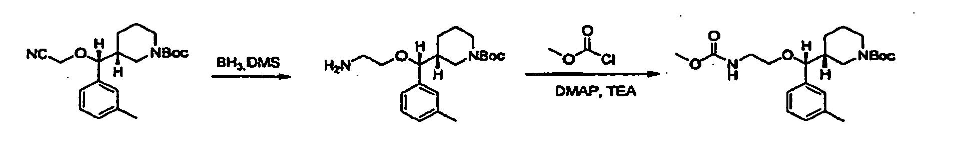

### Step 1. (R)-tert-butyl 3-(3-methylbenzoyl)piperidine-1-carboxylate

To a solution of 1-bromo-3-methylbenzene (88.4 g, 0.52 mol) in anhydrous THF (550 mL) at -78 °C under nitrogen was added dropwise a solution of 2.5 M n-BuLi in hexane (210 mL, 0.52 mol). After stirring for 1 hr at -78 °C, a solution of (*R*)-*tert*-butyl 3-((*R*)-(2-(methoxycarbonylamino)ethoxy)(m-tolyl)methyl)piperidine-1-carboxylate (120 g, 0.44 mol) in anhydrous THF (500 mL) was added dropwise. After addition, the reaction mixture was allowed to warm to rt and stirred for 2 hr. The mixture was quenched with saturated NH₄Cl solution (500 mL) and extracted with EtOAc (3×400 mL). The combined organic layers were washed with brine, dried over Na₂SO₄ and concentrated in *vacuo* to give crude (*R*)-*tert*-butyl 3-(3 - methylbenzoyl)piperidine-1-carboxylate (168 g), which was used immediately for next step without purification.

### Step 2. (R)-tert-butyl 3-((S)-hydroxy(m-tolyl)methyl)piperidine-1-carboxylate

To a solution of (*R*)-*tert*-butyl 3-(3-methylbenzoyl)piperidine-1-carboxylate (168 g, 0.55 mol) in anhydrous THF (600 mL) at -15 °C under nitrogen was added dropwise a solution of 1 M R-CBS-oxazaborolidine in toluene (82 mL, 82 mmol, 0.15 eq). After stirring for 1 hr at -15 °C, a solution of 10 M BH₃ in THF (60 mL, 0.60 mol, 1.1 eq) was added dropwise. After addition, the reaction mixture was stirred for 2 hr at -15 °C. TLC indicated the starting material was disappeared. Methanol (400 mL) was added dropwise carefully at -15 °C. The solvent was removed under reduced pressure, the residue was purified by column chromatography on silica gel eluting with EtOAc/hexane (1:30→1:15) to provide the light yellow oil (95 g, HPLC ≥ 70%, ratio ≥ 3:1). The mixture was dissolved in EtOAc until the alcohol was just dissolved (about 5 mL/1 g), the solvent was removed on the rotary evaporator until a few crystals appeared. The solution was cooled to rt slowly and stood for 1-2 hr. To the above solution was added hexane (about 300 mL) and then filtered , the crystals were washed with cool hexane and re-crystallized two more times to afford the pure isomer (*R*)-*tert*-butyl 3-((*S*)-hydroxy(m-tolyl)methyl)piperidine-1-carboxylate (20 g, ee≥99%).

### Step 3. (R)-tert-butyl 3-((R)-(cyanomethoxy)(m-tolyl)methyl)piperidine-1-carboxylate

To a solution of (*R*)-*tert*-butyl 3-((*S*)-hydroxy(*m*-tolyl)methyl)piperidine-1-carboxylate (30.5 g, 0.1 mol) in MeCN (300 mL), NaH (12 g, 0.3 mol) was added at 0 °C. The mixture was stirred for 1 hr at rt. The mixture was cooled to -40 °C, then bromoacetonitrile (35.7 g, 0.3 mol) was added in portions. The mixture was stirred for 0.5 hr at -20 °C continually. The reaction was quenched with sat. NH₄Cl. The mixture was extracted with CH₂Cl₂. The organic layer was dried over Na₂SO₄, concentrated. Crude (*R*)-*tert-*butyl 3-((*R*)-(cyanomethoxy)(m-tolyl)methyl)piperidine-1-carboxylate was used for the next step without purification.

### Step 4. (R)-tert-butyl 3-((R)-(2-aminoethoxy)(m-tolyl)methyl)piperidine-1-carboxylate

(*R*)-*tert-*Butyl 3-((*R*)-(cyanomethoxy)(m-tolyl)methyl)piperidine-1-carboxylate (20 g, 0.04 mol) was dissolved in anhydrous THF (300 mL), and the solution was heated to reflux under nitrogen. A solution of BH₃.Me₂S (12 mL, 0.12 mol) in THF was added dropwise, and stirring was continued under reflux overnight. The resulting solution was cooled to rt and MeOH was added dropwise to quench the excess borane. After evaporation of the solution, the crude (*R*)-*tert-*butyl 3-((*R*)-(2-aminoethoxy)(m-tolyl)methyl)piperidine-1-carboxylate was obtained and used without further purification.

### Step 5. (R)-tert-butyl 3-((R)-(2-(methoxycarbonylamino)ethoxy)(m-tolyl)methyl)piperidine-1-carboxylate

To a solution of (*R*)-*tert*-butyl 3-((*R*)-(2-aminoethoxy)(m-tolyl)methyl)piperidine-1-carboxylate and DMAP in anhydrous CH₂Cl₂, Et₃N was added. The resulting mixture was cooled to 0-5 °C under ice-water bath, a solution of methyl chloroformate in anhydrous CH₂Cl₂ was added dropwise. After addition, the reaction mixture was stirred for 1-2 hr at 0-5 °C. Water was added to quench the reaction. The aqueous layer was extracted with CH₂Cl₂, the combined organic layers were washed with 10% citric acid and brine, then dried over Na₂SO₄, filtered and concentrated to the crude product, which was purified by preparative TLC to afford (*R*)-*tert-*butyl 3-((*R*)-(2-(methoxycarbonylamino)ethoxy)(*m-*tolyl)methyl)piperidine-1-carboxylate.

### EXAMPLE 4

### (R)-tert-butyl 3-((R)-(3-chloro-4-fluorophenyl)(2-(methoxycarbonylamino)ethoxy)methyl)piperidine-1-carboxylate

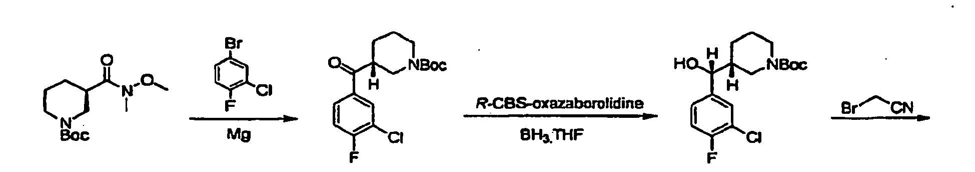

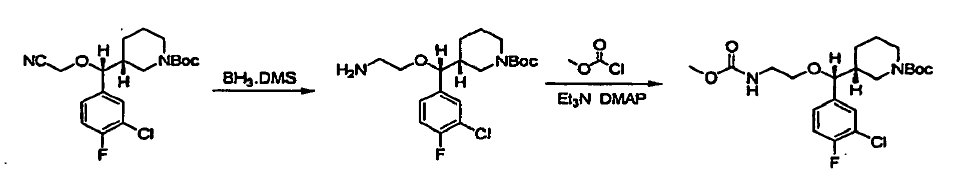

### Step 1. (R)-tert-butyl 3-(3-chloro-4-fluorobenzoyl)piperidine-1-carboxylate

A solution of 4-bromo-2-chloro-1-fluoro-benzene (31.3 g, 0.15 mol) in anhydrous THF (150 mL) was added dropwise to Mg (4.8 g, 0.2 mol) in THF (50 mL) at rt under nitrogen. The mixture was stirred at 50-60 °C for 1 hr at which time most of the magnesium was consumed. The resulting Grignard reagent was used for the next step. The Grignard reagent was added dropwise to a solution of *(R)-tert-*butyl 3-(methoxy(methyl)carbamoyl)piperidine-1-carboxylate (27.2 g, 0.1 mol) in anhydrous THF (300 mL) at -78 °C under nitrogen. After addition, the mixture was allowed to stir at rt for 1.5 hr. The mixture was quenched with saturated NH₄Cl solution (300 mL) and extracted with EtOAc (3×200 mL). The combined organic layers were washed with brine, dried over Na₂SO₄ and concentrated in *vacuo* to give crude (*R*)-*tert-*butyl 3-(3-chloro-4-fluorobenzoyl)piperidine-1-carboxylate (31.5 g, 92%), which was used immediately for next step without purification.

### Step 2. (R)-tert-butyl 3-((R)-(3-chloro-4-fluorophenyl)(hydroxy)methyl)piperidine-1-carboxylate

To a solution of 1 M *R*-CBS-oxazaborolidine in toluene (13.8 mL, 13.8 mmol, 0.15 eq) and 10 M BH₃ in THF (9.2 mL, 92.4 mmol, 1.0 eq) at -15 °C under nitrogen was added dropwise a solution of (*R*)-*tert-*butyl 3-(3-chloro-4-fluorobenzoyl)piperidine-1-carboxylate (31.5 g, 92.4 mmol) in anhydrous THF (300 mL). After addition, the reaction mixture was stirred for 1 hr at rt. Methanol (200 mL) was added dropwise carefully at 0 °C. The solvent was removed under reduced pressure to provide the crude product. The crude product was dissolved in EtOAc till the alcohol was just dissolved (about 5 mL/1 g), the solvent was removed on the rotary evaporator until a few crystals appeared. To the above solution was added petroleum ether (about 300 mL) under stirring, which was allowed to stir at rt for 2 hr and then filtered, the crystals were washed with petroleum ether and re-crystallized 6 times to afford the *(R)-tert-*butyl 3-((*R*)-(3-chloro-4-fluorophenyl)(hydroxy)methyl)piperidine-1-carboxylate (10 g, 32%, 93%e.e.). ¹HNMR (CD₃OD, 400 MHZ) δ 7.44 (d, 1 H), 7.25 (d, 1 H), 7.20 (t, 1 H), 4.34 (d, 1 H), 4.20 (s, 1 H), 3.93 (d, 1 H), 2.68 (m, 2 H), 1.62 (m, 2 H), ), 1.41 (s, 9H), 1.32 (m, 2H), 1.21 (m, 1 H).

### Step 3. (R)-tert-butyl 3-((R)-(3-chloro-4-fluorophenyl)(cyanomethoxy)methyl)piperidine-1-carboxylate

To a solution of (*R*)-*tert*-butyl 3-((*R*)-(3-chloro-4-fluorophenyl)(hydroxy)methyl)piperidine-1-carboxylate (5.1 g, 15 mmol) in CH₃CN (150 mL), NaH (1.8 g, 45 mmol) was added at 0 °C. The mixture was stirring for 1 hour. Then the mixture was cooled to -40 °C, the bromoacetonitrile (5.4 g, 45 mmol) was added dropwise. The mixture was allowed to warm to 0 °C gradually. The addition of NaH and bromoacetonitrile was repeated three times. The mixture was quenched with H₂O and exacted with CH₂Cl₂. The organic layer was dried over Na₂SO₄ and concentrate to get the crude (*R*)-*tert*-butyl 3-((*R*)-(3-chloro-4-fluorophenyl)(cyanomethoxy)methyl)piperidine-1-carboxylate (6.5g, 100%).

### Step 4. (R)-tert-butyl 3-((R)-(2-aminoethoxy)(3-chloro-4-fluorophenyl)methyl)piperidine-1-carboxylate

(*R*)-*tert*-Butyl 3-((*R*)-(3 -chloro-4-fluorophenyl)(cyanomethoxy)methyl)piperidine-1-carboxylate (2.28 g, 6 mmol) was dissolved in anhydrous THF (50 mL), and the solution was heated to reflux under nitrogen. A solution of 10 M of BH₃.Me₂S (1.8 mL, 18 mmol) in THF was added dropwise and stirring was continued under reflux overnight. The resulting solution was cooled to 0 °C, CH₃OH was added dropwise to quench the reaction. Evaporation of the solvent led to crude (*R*)-*tert-*butyl 3-((*R*)-(2-aminoethoxy)(3-chloro-4-fluorophenyl)methyl)piperidine-1-carboxylate (2 g, yield 87%), which was used in the next step without further purification.

### Step 5. (R)-tert-butyl 3-((R)-(3-chloro-4-fluorophenyl)(2-(methoxycarbonylamino)ethoxy)methyl)piperidine-1-carboxylate

To a solution of (*R*)-*tert-*butyl 3-((*R*)-(2-aminoethoxy)(3-chloro-4-fluorophenyl)methyl)piperidine-1-carboxylate (1 g, 2.6 mmol) and DMAP (79 mg, 0.62 mmol) in dry CH₂Cl₂ (20 mL), Et₃N (657 mg, 6.5 mmol) was added. The resulting mixture was cooled to 0-5 °C under ice-water bath, a solution of methyl chloroformate (1.22 g, 13 mmol, 5 eq) was added dropwise. After addition, the reaction mixture was stirred for 1-2 hr at rt. Water (20 mL) was added to quench the reaction. The aqueous layer was extracted with CH₂Cl₂ (3×20 mL), the combined organic layers were dried over Na₂SO₄, filtered and concentrated to give the crude product, which was purified by preparative HPLC to afford (*R*)-*tert-*butyl *3-*((*R*)*-*(3-chloro-4-fluorophenyl)(2-(methoxycarbonylamino)ethoxy)methyl)piperidine-1-carboxylate (50 mg, yield 4.3%). ¹H NMR (CDCl₃, 400 MHz) δ 7.27 (m, 1 H), 7.12 (m, 2 H), 4.30 (s, 1 H), 3.91 (d, 2 H), 3.66 (s, 3 H), 3.10-3.40 (m, 5 H), 2.90 (m, 1 H), 1.75 (s, 1 H), 1.55 (d, 1 H), 1.46 (s, 9 H), 1.33 (m, 2 H), 1.04 (m, 1 H).

The following compounds were prepared following procedures analogous to those described above:
1) (*R*)-*tert-*butyl 3-((*R*)-(5-fluoro-2-methylphenyl)(2-(methoxycarbonylamino)ethoxy)methyl)piperidine-1-carboxylate using (5-fluoro-2-methylphenyl)magnesium bromide in Step 1.
2) (*R*)-*tert-*butyl 3-((*R*)-(3-chloro-5-fluorophenyl)(2-(methoxycarbonylamino)ethoxy)methyl)piperidine-1-carboxylate using (3-chloro-5-fluorophenyl)magnesium bromide in Step 1.

### EXAMPLE 5

### (R)-tert-butyl 3-((R)-(3-fluorophenyl)(2-(methoxycarbonylamino)ethoxy)methyl)piperidtne-1-carboxylate

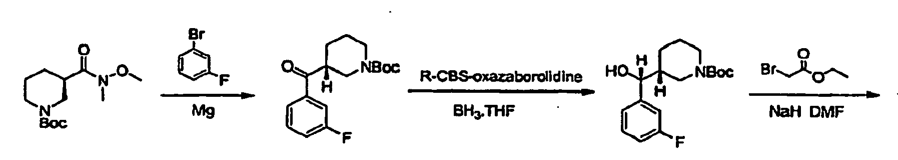

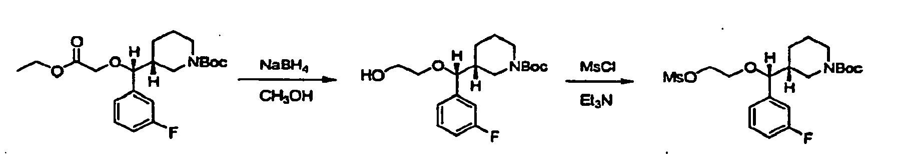

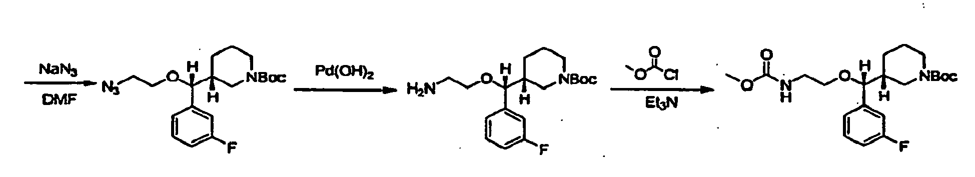

### Step 1. (R)-tert-butyl 3-(3-fluorobenzoyl)piperidine-1-carboxylate

A solution of 1-bromo-3-fluoro-benzene (57.7 g, 0.33 mol) in anhydrous THF (480 mL) was added dropwise to Mg (10.6 g, 0.44 mol) at rt under nitrogen. The mixture was stirred at 50-60 °C for 1 hr. The resulting Grignard reagent was used for the next step. The Grignard reagent was added dropwise to a solution of *(R)-tert-butyl* 3-(methoxy(methyl)carbamoyl)piperidine-1-carboxylate (60g, 0.22 mol) in anhydrous THF (600 mL) at -78 °C under nitrogen. After addition, the mixture was allowed to stir at rt for 1.5 hr. The mixture was quenched with saturated NH₄Cl solution (300 mL) and extracted with EtOAc (3×200 mL). The combined organic layers were washed with brine, dried over Na₂SO₄ and concentrated in *vacuo* to give crude (*R*)-*tert*-butyl 3-(3-fluorobenzoyl)piperidine-1-carboxylate (67.5 g, 100%), which was used immediately in the next step without purification.

### Step 2. (R)-tert-butyl 3-((R)-(3-fluorophenyl)(hydroxy)methyl)piperidine-1-carboxylate

To a solution of 1 M *R*-CBS-oxazaborolidine in toluene (33 mL, 33 mmol, 0.15 eq) and 10 M BH₃ in THF (22 mL, 0.22 mol, 1.0 eq) at -15°C under nitrogen was added dropwise a solution of (*R*)-*tert*-butyl 3-(3-fluorobenzoyl)piperidine-1-carboxylate (67.5 g, 0.22 mol) in anhydrous THF (300 mL). After addition, the reaction mixture was stirred for 1 hr at rt. Methanol (200 mL) was added dropwise carefully at 0 °C. The solvent was removed under reduced pressure to provide the crude product. The crude product was dissolved in EtOAc until the alcohol was just dissolved (about 5mL/1 g), the solvent was removed on the rotary evaporator until a few crystals appeared. To the above solution was added petroleum ether (about 300 mL) under stirring, which was allowed to stir at rt for 2 hr and then filtered, the crystals were washed with petroleum ether and re-crystallized to afford the pure *R*)-*tert-*butyl 3-((*R*)-(3-fluorophenyl)(hydroxy)methyl)piperidine-1-carboxylate (26 g, 39%).

### Step 3. (R)-tert-butyl 3-((R)-(2-ethoxy-2-oxoethoxy)(3-fluorophenyl)methyl)piperidine-1-carboxylate

To a suspension ofNaH (4.8 g, 120 mmol) in THF (400 mL) at 0-5 °C was added dropwise a solution of (*R*)-*tert*-butyl 3-((*R*)-(2-ethoxy-2-oxoethoxy)(3-fluorophenyl)methyl)piperidine-1-carboxylate (30.9 g, 100 mmol) in anhydrous THF (100 mL), the reaction mixture was stirred for 1 hr at rt. A solution of ethyl bromoacetate (20.04 g, 13.40 mL, 120 mmol) in anhydrous THF (100 mL) was added dropwise to the above mixture, and the reaction was heated to reflux for 3-5 hr. The reaction mixture was poured into saturated aqueous NH₄Cl, then extracted with EtOAc (3×100 mL). The organic layer was washed with water (3×100 mL) and brine, dried over Na₂SO₄, filtered and concentrated in *vacuo* to afford crude (*R*)-*tert-*butyl 3-((*R*)-(2-ethoxy-2-oxoethoxy)(3-fluorophenyl)methyl)piperidine-1-carboxylate (29.88g 76 %), which was used for next step without purification.

### Step 4. (R)-tert-butyl 3-((R)-(3-fluorophenyl)(2-hydroxyethoxy)methyl)piperidine-1-carboxylate

To a solution of (*R*)-*tert-*butyl 3-((*R*)-(2-ethoxy-2-oxoethoxy)(3-fluorophenyl)methyl)piperidine-1-carboxylate (29.88 g, 75.9 mmol) in MeOH (300 mL) was added NaBH₄ (23 g, 605.2 mmol) in portions while the temperature was lower than 40 °C. After addition, the mixture was stirred at rt for 2-3 hr. The solvent was removed in *vacuo* to give a residue which was partitioned between water and EtOAc. The organic layer was washed with H₂O and brine, dried over Na₂SO₄, filtered and concentrated in *vacuo.* The residue was purified on silica gel chromatography to afford (*R*)-*tert-*butyl 3-((*R*)-(3-fluorophenyl)(2-hydroxyethoxy)methyl)piperidine-1-carboxylate (11g, 41%).

### Step 5. (R)-tert-butyl 3-((R)-(3-fluorophenyl)(2-(methylsulfonyloxy)ethoxy)methyl)piperidine-1-carboxylate

To a solution of (*R*)-*tert-*butyl 3-((*R*)-(3-fluorophenyl)(2-hydroxyethoxy)methyl)piperidine-1-carboxylate (11 g, 31.16 mmol) in dry CH₂Cl₂ (140 mL) was added Et₃N (12.60 g, 16.68 mL, 124.65 mmol, 4 eq) at -5-0 °C. Then a solution of MsCl (7.1 g, 4.72 mL, 62.32 mmol, 2 eq) in dry CH₂Cl₂ (40 mL) was added dropwise at the same temperature. After addition, it was allowed to warm to rt gradually. Water (100 mL) was added. The aqueous layer was extracted with CH₂Cl₂ (3×80 mL), the combined organic layers was washed with 10% citric acid, sat. NaHCO₃ and brine, then dried over Na₂SO₄, filtered and concentrated to give (*R*)-*tert-*butyl 3-((*R*)-(3-fluorophenyl)(2-(methylsulfonyloxy)ethoxy)methyl)piperidine-1-carboxylate (13.8 g), which was used in the next step without purification.

### Step 6. (R)-tert-butyl 3-((R)-(2-azidoethoxy)(3-fluorophenyl)methyl)piperidine-1-carboxylate

(*R*)-*tert-*Butyl 3-((*R*)-(3-fluorophenyl)(2-(methylsulfonyloxy)ethoxy)methyl)piperidine-1-carboxylate (13.8 g, 32 mmol) was dissolved into anhydrous DMF (150 mL), solid NaN₃ (6.1 g, 96 mmol, 3 eq) was added and the reaction mixture was heated to 80 ° for overnight. The reaction mixture was cooled to rt and then was added with EtOAc (500 mL), the organic phase was washed with water (3×100 mL) and brine (2×80 mL), dried over Na₂SO₄ and concentrated in *vacuo* to give crude (*R*)-*tert-*butyl 3-((*R*)-(2-azidoethoxy)(3-fluorophenyl)methyl)piperidine-1-carboxylate (12 g), which was used in the next step without further purification.

### Step 7. (R)-tert-butyl 3-((R)-(2-aminoethoxy)(3-fluorophenyl)methyl)piperidine-1-carboxylate

A suspension of (*R*)-*tert-*butyl 3-((*R*)-(2-azidoethoxy)(3-fluorophenyl)methyl)piperidine-1-carboxylate (12 g, 31.75 mmol) and Pd(OH)₂/C (1.2 g) in MeOH (240 ml) was stirred under H₂ for 1 hr. The mixture was filtered and evaporated under reduced pressure to give desired (*R*)-*tert-*butyl 3-((*R*)-(2-aminoethoxy)(3-fluorophenyl)methyl)piperidine-1-carboxylate (10 g).

### Step 8. (R)-tert-butyl 3-((R)-(3-fluorophenyl)(2-(methoxycarbonylamino)ethoxy)methyl)piperidine-1-carboxylate

To a solution of (*R*)-*tert-*butyl 3-((*R*)-(2-aminoethoxy)(3-fluorophenyl)methyl)piperidine-1-carboxylate (10 g, 28.41 mmol) and DMAP (1.8 g, 14.21 mmol, 0.5 eq) in dry CH₂Cl₂ (150 mL), Et₃N (8.62 g, 11.42 mL, 85.23 mmol) was added. The resulting mixture was cooled to 0-5 °C under ice-water bath, a solution of methyl chloroformate (10.95 mL, 142.05 mmol, 5 eq) in dry CH₂Cl₂ (60 mL) was added dropwise. After addition, the reaction mixture was stirred for 1 - 2 hr at 0-5 °C. Water (80 mL) was added to quench the reaction. The aqueous layer was extracted with CH2Cl₂ (3×50 mL), the combined organic layers were washed with 10% citric acid (2×80 mL) and brine, then dried over Na₂SO₄, filtered and concentrated to the crude product, which was purified by silica gel to afford (*R*)*-tert-*butyl 3-((*R*)-(3-fluorophenyl)(2-(methoxycarbonylamino)ethoxy)methyl)piperidine-1-carboxylate (11.3 g, 97%).

### EXAMPLE 6

### (R)-tert-butyl 3-((R)-(3-chloro-5-fluorophenyl)(2-(ethoxycarbonylamino)ethoxy)methyl)piperidine-1-carboxylate

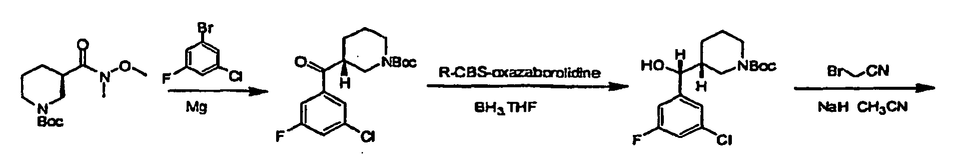

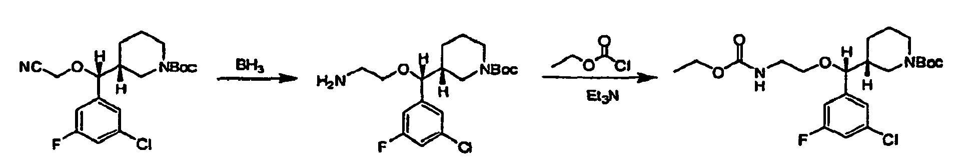

### Step 1. (R)-tert-butyl 3-(3-chloro-5-fluorobenzoyl)piperidine-1-carboxylate

- A solution of 1-bromo-3-chloro-5-fluoro-benzene (31.5 g, 0.15 mol) in anhydrous THF (120 mL) was added dropwise to the Mg (5.4 g, 0.22 mol) at rt under nitrogen. The mixture was stirred at 50-60 °C for 1 hr until most of the magnesium was consumed. The resulting Grignard reagent was used for the next step. The Grignard reagent was added dropwise to a solution of (*R*)-*tert-*butyl 3-(methoxy(methyl)carbamoyl)piperidine-1-carboxylate (20.4 g, 0.075 mol) in anhydrous THF (200 mL) at -78 °C under nitrogen. After addition, the mixture was allowed to stir at rt for 1.5 hr. The mixture was quenched with saturated NH₄Cl solution (300 mL) and extracted with EtOAc (3×200 mL). The combined organic layers were washed with brine, dried over Na₂SO₄ and concentrated in *vacuo* to give crude (*R*)-*tert-*butyl 3-(3-chloro-5-fluorobenzoyl)piperidine-1-carboxylate (25 g, 98%), which was used in the next step without further purification.

### Step 2. (R)-tert-butyl 3-((R)-(3-chloro-5-fluorophenyl)(hydroxy)methyl)piperidine-1-carboxylate

To a solution of 1 M *R*-CBS-oxazaborolidine in toluene (11 mL, 11 mmol, 0.15 eq) and 10 M BH₃ in THF (7.3 L, 73 mmol, 1.0 eq) at -15 °C under nitrogen was added dropwise a solution of (*R*)-*tert-*butyl 3-(3-chloro-5-fluorobenzoyl)piperidine-1-carboxylate (25 g, 73 mmol) in anhydrous THF (50 mL). After addition, the reaction mixture was stirred for 1 hr at rt. Methanol (100 mL) was added dropwise carefully at 0 °C. The solvent was removed under reduced pressure to provide the crude product. The crude product was dissolved in EtOAc until the alcohol was just dissolved (about 5 mL/1 g), the solvent was removed on the rotary evaporator until a few crystals appeared. To the above solution was added petroleum ether (about 300 mL) under stirring, which was allowed to stir at rt for 2 hr and then filtered, the crystals were washed with petroleum ether and re-crystallized a few more times to afford pure (*R*)-*tert-*butyl 3-((*R*)-(3-chloro-5-fluorophenyl)(hydroxy)methyl)piperidine-1-carboxylate (9.2 g, 37%). ¹H NMR (DMSO, 400 MHz): δ 7.44 (d, 1 H), 7.38 (s, 1 H), 7.30 (d, 1 H), 4.48 (t, 1 H), 4.20 (brs, 1 H), 3.98 (d, 1 H), 2.73 (s, 2 H), 1.70 (s, 2 H), 1.48 (s, 10H), 1.36-1.39 (m, 2 H).

### Step 3. (R)-tert-butyl 3-((R)-(3-chloro-5-fluorophenyl)(cyanomethoxy)methyl)piperidine-1-carboxylate

To a solution of (*R*)-*tert-*butyl 3-((*R*)-(3-chloro-5-fluorophenyl)(hydroxy)methyl)piperidine-1-carboxylate (3.5 g, 10.2 mmol) in CH₃CN (140 mL), NaH (1.2 g, 30.6 mmol) was added at 0 °C. The mixture was stirred for 1 hr. Then the mixture was cooled to -20 °C, bromoacetonitrile (3.6 g, 30.6 mmol) was added dropwise. The mixture was allowed warm to 0 °C gradually. Another batch of NaH and bromoacetonitrile was added in the same manner. The mixture was quenched with H₂O and extracted with CH₂Cl₂. The organic layer was dried over Na₂SO₄ and concentrate to give the crude (*R*)-*tert-*butyl 3-((*R*)-(3-chloro-5-fluorophenyl)(cyanomethoxy)methyl)piperidine-1-carboxylate (4.4, 100%).

### Step 4. (R)-tert-butyl 3-((R)-(2-aminoethoxy)(3-chloro-5-fluorophenyl)methyl)piperidine-1-carboxylate

(*R*)-*tert-*Butyl 3-((*R*)-(3-chloro-5-fluorophenyl)(cyanomethoxy)methyl)piperidine-1-carboxylate (4.4 g, 10.2 mmol, crude) was dissolved in anhydrous THF (60 mL), and the solution was heated to reflux under nitrogen. A solution of 10 M of BH₃.Me₂S (3 mL, 30.6 mmol) in THF was added dropwise and stirring was continued under reflux overnight. The resulting solution was cooled to 0 °C, CH₃OH was added dropwise to quench the reaction. Evaporation of the solvent to give the crude product, which was purified by silica column to give (*R*)-*tert-*butyl 3-((*R*)-(2-aminoethoxy)(3-chloro-5-fluorophenyl)methyl)piperidine-1-carboxylate (1.1 g, yield 28%), which was used in the next step without further purification.

### Step 5. (R)-tert-butyl 3-((R)-(3-chloro-5-fluorophenyl)(2-(ethoxycarbonylamino)ethoxy)methyl)piperidine-1-carboxylate

To a solution of (*R*)-*tert*-butyl 3-((*R*)-(2-aminoethoxy)(3-chloro-5-fluorophenyl)methyl)piperidine-1-carboxylate (1.1 g, 2.85 mmol) in dry CH₂Cl₂ (20 mL), Et₃N (2 mL) was added. The resulting mixture was cooled to 0-5 °C under ice-water bath, a solution of ethyl chloroformate (615 mg, 5.7 mmol) in dry CH₂Cl₂ (2 mL) was added dropwise. After addition, the reaction mixture was stirred for 1-2 hr at rt. Water (20 mL) was added to quench the reaction. The aqueous layer was extracted with CH₂Cl₂ (3×20 mL), the combined organic layers were dried over Na₂SO₄, filtered and concentrated to give the crude (*R*)-*tert-*butyl 3-((*R*)-(3-chloro-5-fluorophenyl)(2-(ethoxycarbonylamino)ethoxy)methyl)piperidine-1-carboxylate (1.3 mg, 100%). ¹H NMR (CD₃OD, 400 MHz) δ 7.01 (d, 2 H), 6.87 (d, 1 H), 4.32 (m, 2 H), 4.09 (m, 2 H), 3.92 (m, 2 H), 3.33 (m, 5 H), 1.75 (s, 1 H), 1.55 (m, 1 H), 1.43 (s, 9 H), 1.34 (m, 2 H), 1.23 (t, 3 H), 1.09 (t, 1 H).

### EXAMPLE 7

### (R)-tert-butyl 3-((R)-(5-chloro-2-methylphenyl)(2-(methoxycarbonylamino)ethoxy)methyl)piperidine-1-carboxylate

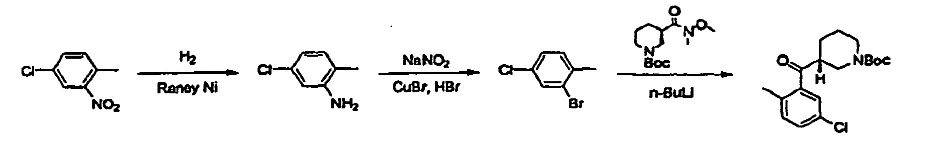

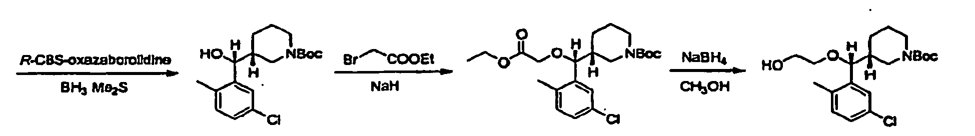

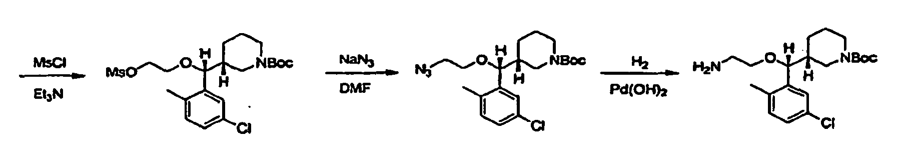

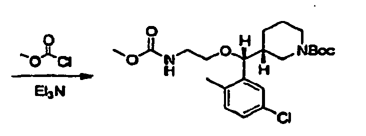

### Step 1. 5-chloro-2-methylbenzenamine

A 2 L flask was charged the solution of 4-chloro-1-methyl-2-nitrobenzene (60 g, 0.35 mol) in MeOH (1 L), Raney Ni was added, the air in flask was replaced three times with H₂, the mixture was stirred for 3 hr at rt. The solution was filtered and concentrated. The residue was dissolved in CH₂Cl₂ (500 mL), and the solution was washed with brine, dried over Na₂SO₄. Solvent removal gave 5-chloro-2-methylbenzenamine (50 g, 0.35 mol). ¹H NMR (CDCl₃, 400MHz) δ 7.02-6.93 (d, 2H), 6.70-6.60 (d, 2H), 3.67 (s, 2H), 2.14 (s, 3H).

### Step 2. 2-bromo-4-chloro-1-methylbenzene

5-Chloro-2-methylbenzenamine (50 g, 0.355 mol) was dissolved in aq HBr solution (1.5 M, 100 mL) and cooled to 0 °C, a solution of NaNO₂ (27.6 g, 0.4 mol) in water (200 mL) was added dropwise. After addition, the mixture was stirred for 1 hr. In another flask CuBr (30 g, 0.21 mol) was added to HBr solution (1.5 M, 30 mL) and heated to 60 °C, then the mixture was added to the above solution. The mixture was heated to reflux for 1 hr then cooled to rt. The reaction was quenched with water (500 mL), the aqueous layer was extracted 3 times with CH₂Cl₂, dried over Na₂SO₄, solvent removal and purification by column chromatography afforded 2-bromo-4-chloro-1-methylbenzene (53 g, 0.26 mol). ¹H NMR (CDCl₃, 400MHz) δ 7.53 (s, 1H), 7.20-7.10 (m, 2H), 2.36 (s, 3H).

### Step 3. (R)-tert-butyl 3-(5-chloro-2-methylbenzoyl)piperidine-1-carboxylate

To a solution of 2-bromo-4-chloro-1-methylbenzene (53 g, 0.26mol) in anhydrous THF (600 mL) at -78 °C under nitrogen was added dropwise a solution of 2.5 M n-BuLi in hexane (103 mL, 0.26 mol). After stirring for 1 hr at -78 °C, a solution of the (*R*)-*tert*-butyl 3-(methoxy(methyl)carbamoyl)piperidine-1-carboxylate (67 g, 0.246 mol) in anhydrous THF (300 mL) was added dropwise. After addition, the reaction mixture was allowed to warm to rt and stirred for 2 hr. The mixture was quenched with saturated NH₄Cl solution (500 mL) and extracted with EtOAc (3×400 mL). The combined organic layers were washed with brine, dried over Na₂SO₄ and concentrated in *vacuo* to give crude (*R*)-*tert-*butyl 3-(5-chloro-2-methylbenzoyl)piperidine-1-carboxylate (86 g), which was used immediately in the next step without purification.

### Step 4. (R)-tert-butyl 3-((R)-(5-chloro-2-methylphenyl)(hydroxy)methyl)piperidine-1-carboxylate

A mixture of 10 M BH₃.Me₂S in THF (25.4 mL, 0.254 mol ) and 1 M *R-*CBS-oxazaborolidine in toluene (38 mL, 0.038 mol ) were dissolved in 100 mL anhydrous THF and cooled to-15 °C. (*R*)-*tert*-butyl 3-(5-chloro-2-methylbenzoyl)piperidine-1-carboxylate in 200 mL anhydrous THF was added dropwise to the above solution and stirred at -15 °C for 2 hr. The reaction was quenched with methanol (300 mL). The solvent was removed under reduced pressure, and the residue was purified by column chromatography to give (*R*)*-tert-*butyl 3-((*R*)-(5-chloro-2-methylphenyl)(hydroxy)methyl)piperidine-1-carboxylate (32 g), which contained 30% isomer.

### Step 5. (R)-tert-butyl 3-((R)-(5-chloro-2-methylphenyl)(2-ethoxy-2-oxoethoxy)methyl)piperidine-1-carboxylate

To a suspension of NaH (5.64 g, 0.141 mol) in the mixed solvent of DMF (70 mL) and THF (70 mL) at -25 °C was added dropwise a solution of (*R*)-*tert-*butyl 3-((*R*)-(5-chloro-2-methylphenyl)(hydroxy)methyl)piperidine-1-carboxylate (16 g, 47 mmol) in anhydrous THF (100 mL), the reaction mixture was stirred for 1 hr at rt. A solution of ethyl bromoacetate (15.6 g, 94 mmol) in anhydrous THF (70 mL) was added dropwise to the above mixture at -10- -5 °C. After addition, the reaction mixture was stirred for 2-3 hr at rt. The reaction was quenched with saturated NH₄Cl solution (100 mL) and EtOAc (500 mL) was added. The organic layer was washed with water (5×50 mL) and brine, dried over Na₂SO₄, filtered and concentrated in *vacuo.* The residue was purified by column chromatography to afford (*R*)-*tert*-butyl 3-((*R*)-(5-chloro-2-methylphenyl)(2-ethoxy-2-oxoethoxy)methyl)piperidine-1-carboxylate (8 g, 18.8 mmol).

### Step 6. (R)-tert-butyl 3-((R)-(5-chloro-2-methylphenyl)(2-hydmxyethoxy)methyl)piperidine-1-carboxylate

To a solution of (*R*)-*tert-*butyl 3-((*R*)-(5-chloro-2-methylphenyl)(2-ethoxy-2-oxoethoxy)methyl)piperidine-1-carboxylate (8g, 18.8mmol)) in MeOH (300 mL) was added NaBH4 (5.6 g, 0.15 mol) in portions while the temperature was lower than 40 °C. After addition, the mixture was stirred overnight. The solvent was removed in *vacuo* to the residue, which was partitioned between water and EtOAc. The organic layer was washed with H₂O and brine, dried over Na₂SO₄ and evaporated to give crude (*R*)-*tert-*butyl 3-((*R*)-(5-chloro-2-methylphenyl)(2-hydroxyethoxy)methyl)piperidine-1-carboxylate (7 g), which was used in the next step without purification.

### Step 7. (R)-tert-butyl 3-((R)-(5-chloro-2-methylphenyl)(2-(methylsulfonyloxy)ethoxy)methyl)piperidine-1-carboxylate

To a solution of (*R*)-*tert-*butyl 3-((*R*)-(5-chloro-2-methylphenyl)(2-hydroxyethoxy)methyl)piperidine-1-carboxylate (7 g, 18.3 mmol) in dry CH₂Cl₂ (100 mL) was added Et₃N (54 g, 10 mL, 0.73 mmol) at -5-0 °C. Then a solution of MsCl (4.2 g, 36.5 mmol) in dry CH₂Cl₂ (50 mL) was added dropwise at the same temperature. After addition, it was allowed to warm to rt gradually. The reaction mixture was washed with 10% citric acid solution (30 mL), NaHCO₃ and brine, then dried over Na₂SO4_{,} filtered and concentrated to give (*R*)-*tert-*butyl 3-((*R*)-(5-chloro-2-methylphenyl)(2-(methylsulfonyloxy)ethoxy)methyl)piperidine-1-carboxylate (8.4 g), which was used in the next step without purification.

### Step 8. (R)-tert-butyl 3-((R)-(2-azidoethoxy)(5-chloro-2-methylphenyl)methyl)piperidine-1-carboxylate

(*R*)-*tert-*Butyl 3-((*R*)-(5-chloro-2-methylphenyl)(2-(methylsulfonyloxy)ethoxy)methyl)piperidine-1-carboxylate (8.4 g, 18.3 mmol) was dissolved in anhydrous DMF (150 mL), solid NaN₃ (3.56 g, 54.8 mmoL) was added and the reaction mixture was heated to 60 °C for overnight. The reaction mixture was cooled to rt and diluted with EtOAc (500 mL), the organic phase was washed with water (5×50 mL) and brine (100 mL), dried over Na₂SO₄ and concentrated in *vacuo* to give (*R*)-*tert-*butyl 3-((*R*)-(2-azidoethoxy)(5-chloro-2-methylphenyl)methyl)piperidine-1-carboxylate (7 g).

### Step 9. (R)-tert-butyl 3-((R)-(2-aminoethoxy)(5-chloro-2-methylphenyl)methyl)piperidine-1-carboxylate

(*R*)-*tert-*Butyl 3-((*R*)-(2-azidoethoxy)(5-chloro-2-methylphenyl)methyl)piperidine-1-carboxylate (7 g, 17.1 mmoL) was dissolved in EtOAc (300 mL), 0.8 g of Pd(OH)₂ was added and the air in bottle was replaced 3 times with H₂, the reaction was stirred at rt for 3 hr. The solution was filtered and concentrated to give (*R*)-*tert-*butyt 3-((*R*)-(2-aminoethoxy)(5-chloro-2-methylphenyl)methyl)piperidine-1-carboxylate (6.2 g), which was used in the next step without further purification.

### Step 10. (R)-tert-butyl 3-((R)-(5-chloro-2-methylphenyl)(2-(methoxycarbonylamino)ethoxy)methyl)piperidine-1-carboxylate

To a solution of (*R*)-*tert*-butyl 3-((*R*)-(2-aminoethoxy)(5-chloro-2-methylphenyl)methyl)piperidine-1-carboxylate (6.2 g, 16.2 mmol) and DMAP (0.2 g, 1.62 mmol) in dry CH₂Cl₂ (70 mL), Et₃N (8 g, 81 mmol) was added. The resulting mixture was cooled to 0-5 °C in ice-water bath, a solution of methyl chloroformate (3.1 g, 32.4 mmol) in dry CH₂Cl₂ (30mL) was added dropwise. After addition, the reaction mixture was stirred for 1-2 hr at 0-5 °C. The reaction was quenched with water. The aqueous layer was extracted with CH₂Cl₂ (3×30 mL), the combined organic layers were washed with brine, then dried over Na₂SO₄, filtered and concentrated to give the crude product, which was firstly purified by column chromatography and then by preparative HPLC to give (*R*)-*tert-*butyl 3-((*R*)-(5-chloro-2-methylphenyl)(2-(methoxycarbonylamino)ethoxy)methyl)piperidine-1-carboxylate (1.5g). ¹H NMR (CD₃OD, 400MHz) δ 7.30 (s, 1H), 7.20-7.10 (d, 2H), 4.81 (s, 1H), 4.46-4.30 (d, 1H), 4.29-4.15 (d, 1H), 3.95-3.83 (d, 1H), 3.62 (s, 3H), 3.30 (s, 4H), 2.90-2.65 (dd, 2H), 2.30 (s, 3H), 1.70 (s, 1H), 1.59 (s, 1H), 1.41 (s, 9H), 1.35-1.20 (m, 3H).

### EXAMPLE 8

### (R)-tert-butyl 3-((R)-(2-(methoxycarbonylamino)ethoxy)(phenyl)methyl)piperidine-1-carboxylate

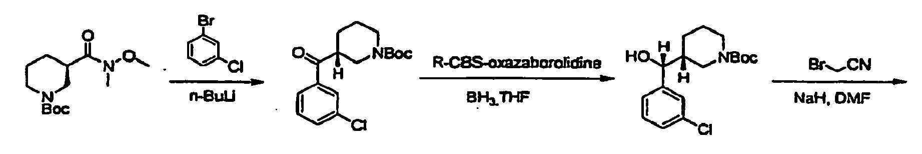

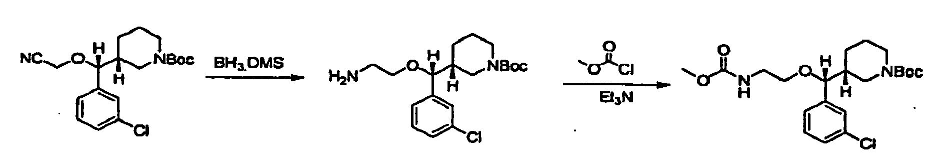

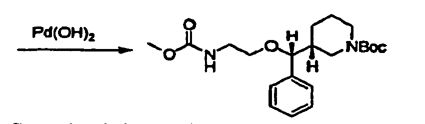

### Step 1. (R)-tert-butyl 3-(3-chlorobenzoyl)piperidine-1-carboxylate

To a solution of 1-bromo-3-chlorobenzene (100 g, 0.52 mol) in anhydrous THF (550 mL) at -78 °C under nitrogen was added dropwise a solution of 2.5 M *n-*BuLi in hexane (210 mL, 0.52 mol). After stirring for 1 hr at -78 °C, a solution of (*R*)-*tert-*butyl 3-(methoxy(methyl)carbamoyl)piperidine-1-carboxylate (120 g, 0.44 mol) in anhydrous THF (500 mL) was added dropwise. After addition, the reaction mixture was allowed to warm to rt and stirred for 2 hr. The mixture was quenched with saturated NH₄Cl solution (500 mL) and extracted with EtOAc (3x400 mL). The combined organic layers were washed with brine, dried over Na₂SO₄ and concentrated in *vacuo* to give crude (*R*)-*tert-*butyl 3-(3-chlorobenzoyl)piperidine-1-carboxylate (178 g), which was used immediately for next step without purification.

### Step 2. (R)-tert-butyl 3-((R)-(3-chlorophenyl)(hydroxy)methyl)piperidine-1-carboxylate

To a solution of (*R*)-*tert-*butyl 3-(3-chlorobenzoyl)piperidine-1-carboxylate (178 g, 0.55 mol) in anhydrous THF (600 mL) at -15 °C under nitrogen was added dropwise a solution of 1 M *R*-CBS-oxazaborolidine in toluene (82 mL, 82 mmol, 0.15 eq). After stirring for 1 hr at -15 °C, a solution of 10 M BH₃ in THF (60 mL, 0.60 mol, 1.1 eq) was added dropwise. After addition, the reaction mixture was stirred for 2 hr at -15 °C. Methanol (400 mL) was added dropwise carefully art -15 °C. The solvent was removed under reduced pressure, the residue was purified by column chromatography on silica gel eluting with EtOAc/hexane (1:30→1:15) to provide the light yellow oil (95 g, HPLC≥70%, ratio≥3:1). The mixture was dissolved in EtOAc until the alcohol was just dissolved (about 5mL/1g), the solvent was removed on the rotary evaporator until a few crystals appeared. The solution was cooled to rt slowly and stood for 1-2 hr. To the above solution was added hexane (about 300 mL) and then filtered , the crystals were washed with cool hexane and re-crystallized an additional two times to afford the pure (*R*)-*tert*-butyl 3-((*R*)-(3-chlorophenyl)(hydroxy)methyl)piperidine-1-carboxylate (20 g, ee≥99%).

### Step 3. (R)-tert-butyl 3-((R)-(3-chlorophenyl)(cyanomethoxy)methyl)piperidine-1-carboxylate

To a solution of (*R*)-*tert*-butyl 3-((*R*)-(3-chlorophenyl)(hydroxy)methyl)piperidine-1-carboxylate (32.5 g, 0.1 mol) in MeCN (325 mL), NaH (12 g, 0.3 mol) was added at 0 °C. The mixture was stirred for 1 hr at rt. The mixture was cooled to -40 °C, then bromoacetonitrile (35.7 g, 0.3 mol) was added in portions. The mixture was stirred for 0.5 hr at -20 °C. After the reaction was complete it was quenched with sat. NH₄Cl. The mixture was extracted with CH₂Cl₂. The organic layer was dried over Na₂SO₄, concentrated. Crude (*R*)-*tert-*butyl 3-((*R*)-(3-chlorophenyl)(cyanomethoxy)methyl)piperidine-1-carboxylate was used for the next step without further purification.

### Step 4. (R)-tert-butyl 3-((R)-(2-aminoethoxy)(3-chlorophenyl)methyl)piperidine-1-carboxylate

(*R*)-*tert-*Butyl 3-((*R*)-(3-chlorophenyl)(cyanomethoxy)methyl)piperidine-1-carboxylate (23 g, 0.04 mol) was dissolved in anhydrous THF (300 mL), and the solution was heated to reflux under nitrogen. A solution of BH₃.Me₂S (12 mL, 0.12 mol) in THF was added dropwise, and stirring was continued at reflux overnight. The resulting solution was cooled to rt and MeOH was added dropwise to quench the reaction. After evaporation of the solution, the crude (*R*)-*tert-*butyl 3-((*R*)-(2-aminoethoxy)(3-chlorophenyl)methyl)piperidine-1-carboxylate was obtained which was used for the next step without purification.

### Step 5. (R)-tert-butyl 3-((R)-(3-chlorophenyl)(2-(methoxycarbonylamino)ethoxy)methyl)piperidine-1-carboxylate

To a solution of (*R*)-*tert-*butyl 3-((*R*)-(2-aminoethoxy)(3-chlorophenyl)methyl)piperidine-1-carboxylate (7.7 g, 21 mmol) and DMAP (1.27 g, 10 mmol), 0.5 eq) in dry CH₂Cl₂ (120 mL), Et₃N (6.38 g, 8.45 mL, 63 mmol) was added. The resulting mixture was cooled to 0-5 °C under ice-water bath, a solution of methyl chloroformate (9.88 g, 8.1 mL, 104.5 mmol, 5 eq) in dry CH₂Cl₂ (50 mL) was added dropwise. After addition, the reaction mixture was stirred for 1-2 hr at 0-5 °C. The reaction was quenched with water (80 mL). The aqueous layer was extracted with CH₂Cl₂ (3×50 mL), the combined organic layers were washed with 10% citric acid (2x80 mL) and brine, then dried over Na₂SO₄, filtered and concentrated to the crude product, which was purified by preparative HPLC to afford (*R*)-*tert*-butyl 3-((*R*)-(3-chlorophenyl)(2-(methoxycarbonylamino)ethoxy)methyl)piperidine-1-carboxylate (4.4 g, the total yield for five steps is 41 %).

### Step 6. (R)-tert-butyl 3-((R)-(3-chlorophenyl)(2-(methoxycarbonylamino)ethoxy)methyl)piperidine-1-carboxylate

To a solution of (*R*)-*tert-*butyl 3-((*R*)-(3-chlorophenyl)(2-(methoxycarbonylamino)ethoxy)methyl)piperidine-1-carboxylate (3 g, 7.04 mmol) in MeOH (60 mL) was added wet Pd(OH)₂/C (300 mg). The reaction mixture was stirred under 50 psi at 50 °C for 3 hr. The suspension was filtered and the filtrate was concentrated in *vacuo.* The crude product was purified by preparative HPLC to afford (*R*)-*tert-*butyl 3-((*R*)-(3-chlorophenyl)(2-(methoxycarbonylamino)ethoxy)methyl)piperidine-1-carboxylate (1.4 g, 51%). ¹H NMR (CD₃OD) δ 7.40-7.22 (m, 5H), 4.20 (m, 1H), 4.01 (m, 1H), 3.81 (m, 1H), 3.6 (s, 3H), 3.27 (m, 3H), 2.84 (m, 2H), 1.8-1.5 (m, 2H), 1.45 (s, 9H). MS ESI +ve m/z 393 (M+1).

### EXAMPLE 9

### 2,2-dimethyl-4-(((R)-tetrahydro-2H-pyran-3-yl)methyl)oxazolidine

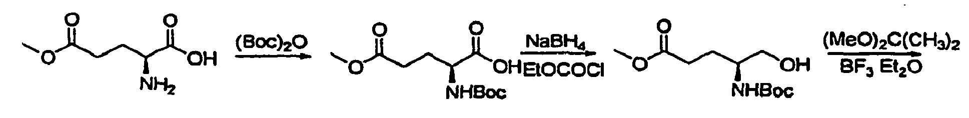

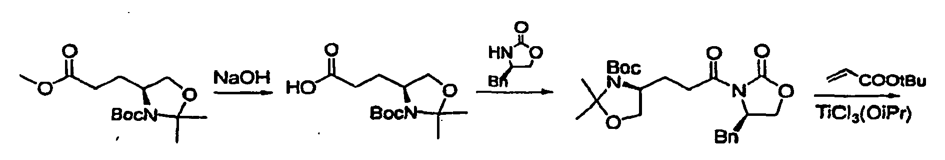

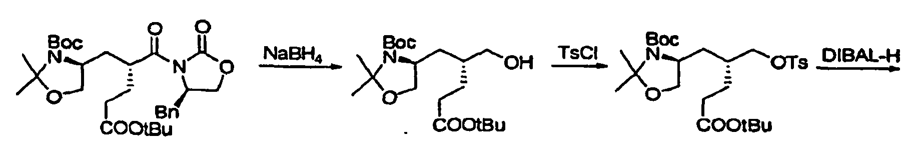

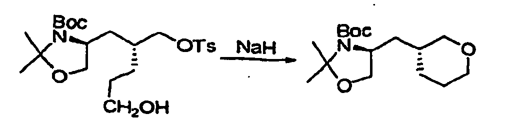

### Step 1. (S)-2-(tert-butoxycarbonylamino)-5-methoxy-5-oxopentanoic acid

To a round bottom flask, Et₃N (303g, 3mol) was added dropwise to a stirred solution of Boc₂O (261.6 g, 1.2 mol) and 2-amino-pentanedioic acid 5-methyl ester (161 g, 1 mol) in water (800 ml) and dioxane (800 ml). After 18 hr the solution was extracted with petroleum ether (2 × 1000ml) and the aqueous phase was cooled on ice and carefully acidified to pH 3 by slow addition of 10% citric acid solution. The urethane was then extracted into EtOAc (3 × 1000ml) and the combined extracts were washed with brine, then dried (Na₂SO₄), filtered and concentrated under reduced pressure to give (*S*)-2-(*tert*-butoxycarbonylamino)-5-methoxy-5-oxopentanoic acid (238g, 91.2%), which was used without further purification.

### Step 2. (S)-methyl 4-(tert-butoxycarbonylamino)-5-hydroxypentanoate

To a stirred solution of (*S*)-2-(*tert-*butoxycarbonylamino)-5-methoxy-5-oxopentanoic acid (35.2 g,0.135 mol) in THF (500 mL) at -10 °C was added N-methylmorpholine (15 mL, 0.135 mol) followed by ethyl chloroformate (14.72 g, 0.135 mol). After 10 min, NaBH₄ (15.37 g, 0.405 mol) was added in one portion. MeOH (1200 mL) was then added dropwise to the mixture over a period of 20 min at 0 °C. The solution was stirred for an additional 20 min and then neutralized with 1M KHSO₄. The organic solvent was removed and the aqueous layer was extracted with EtOAc (3 × 500 ml). The combined organic phases were washed consecutively with 1 M KHSO₄ (300 mL), H₂O (300 mL), 5% aqueous NaHCO₃ (300 mL), and dried (Na₂SO₄). The solvent was evaporated to give a residue, which was purified by column chromatography to give the desired (*S*)-methyl 4-(*tert-*butoxycarbonylamino)-5-hydroxypentanoate (24 g, 72%)

### Step 3. (S)-tert-butyl 4-(3-methoxy-3-oxopropyl)-2,2-dimethyloxazolidine-3-carboxylate

(*S*)-Methyl 4-(*tert*-butoxycarbonylamino)-5-hydroxypentanoate (24 g, 97.2 mmol) and isopropenyl methyl ether (88.8 g, 854.6 mmol) was dissolved in acetone (2000 mL) and BF₃·Et₂O (0.82 mL, 5.84 mmol) was added at rt. The mixture was stirred for I hr at rt. The reaction was quenched by addition of Et₃N (11.6 mL). The reaction solution was washed with aqueous saturated NaHCO₃ (200 mL) and evaporated, and (*S*)-*tert*-butyl 4-(3-methoxy-3-oxopropyl)-2,2-dimethyloxazolidine-3-carboxylate (25.1 g, 90 %) was obtained as an oil, which was used in the next step without further purification.

### Step 4. (S)-3-(3-(tert-butoxycarbonyl)-2,2-dimethyloxazolidin-4-yl)propanoic acid

An aqueous solution of sodium hydroxide (195 mL, 4.0 M in H₂O, 0.261mol, 3.0 eq) was added to a solution of (*S*)-*tert*-butyl 4-(3-methoxy-3-oxopropyl)-2,2-dimethyloxazolidine-3-carboxylate (25.1 g, 0.087 mol), and the resulting cloudy reaction mixture was stirred at 23 °C for 3.5 hr. The mixture was concentrated under reduced pressure to ~50 mL volume and then was partitioned between 0.5 M HCl (360 ml) and EtOAc (2 × 360ml). The combined organic layers were dried over Na₂SO₄ and were filtered. The filtrate was concentrated under reduced pressure to give (*S*)-3-(3-(*tert*-butoxycarbonyl)-2,2-dimethyloxazolidin-4-yl)propanoic acid (21.6 g, 91%), which was used without further purification.

### Step 5. (S)-tert-butyl 2,2-dimethyl-4-(3-((R)-4-methyl-2-oxooxazolidin-3-yl)-3-oxopropyl)oxazolidine-3-carboxylate

A 2000 mL flask was charged with (*S*)-3-(3-(*tert*-butoxycarbonyl)-2,2-dimethyloxazolidin-4-yl)propanoic acid (21.6 g, 79 mmol) and 750 mL of dry THF. The solution was cooled to 0 °C, then triethylamine (23.94 g, 237 mmol, 3.0 equiv) and pivaloyl chloride (9.76 mL, 79 mmol, 1.0 equiv) were sequentially added. The solution was stirred for 4 hr at 0 °C. After this time (*R*)-4-benzyl-2-oxalozolidinone (13.26g, 75.2 mmol, 0.95 equiv) and dried LiCl (3.68 g, 86.4 mmol, 1.1 equiv) were added and the reaction was allowed to stir for 13 hr with concomitant warming to ambient temperature. After this time 560 mL of 0.5 M HCl was added, the mixture was transferred to a separatory funnel and the layers were separated. The aqueous layer was extracted with EtOAc (3×370 mL), and the combined organic layers washed with 10% K₂CO₃ (2×370 mL), and brine (2×370 mL ), then dried over Na₂SO₄, and evaporated. The crude material was purified by flash chromatography, eluting with 0-29% EtOAc in hexanes. This afforded 26.3g (81%) of (*S*)-*tert*-butyl 2,2-dimethyl-4-(3-((*R*)4-methyl-2-oxooxazolidin-3-yl)-3-oxopropyl)oxazolidine-3-carboxylate as a clear syrup.

### Step 6. (S)-tert-butyl 4-((R)-5-tert-butoxy-2-((R)-4-methyl-2-oxooxazolidine-3-carbonyl)-5-oxopentyl)-2,2-dimethyloxazolidine-3-carboxylate

At 0 °C, 1.0M TiCl₄ in CH₂Cl₂ solution (8.55 mL, 0.7 eq) was added to CH₂Cl₂ (100 mL) followed by the addition of 1.0M TiCl(Oi-Pr)₃ in hexanes solution (4.28 mL, 0.35 eq) and stirred 5 min DIPEA (2.87 mL, 1.35 eq) was added and stirred 15 min. A solution of (*S*)-*tert*-butyl 2,2-dimethyl-4-(3-((*R*)-4-methyl-2-oxooxazolidin-3-yl)-3-oxopropyl)oxazolidine-3-carboxylate (5.28 g, 12.22 mmol) in CH₂Cl₂ (50 mL) was added. The reaction mixture was stirred 1 hr at 0 °C. To the solution, t-butylacrylate (2.22 mL, 1.25 eq) was added and the mixture was left stirred over 48 hr with concomitant warming to rt. The mixture was concentrated, partitioned between EtOAc (300 mL) and 1% HCl solution (100 mL). The organic layer was washed with sat. NaHCO₃ solution (60 mL), brine (60 mL), dried over Na₂SO₄. After filtration and concentration, the residue was purified by ISCO (120 g column, 0~35% EtOAc in Hexanes gradient) to afford 4.12 g (60%) (*S*)-*tert*-butyl 4-((*R*)-5-*tert*-butoxy-2-((*R*)-4-methyl-2-oxooxazolidine-3-carbonyl)-5-oxopentyl)-2,2-dimethyloxazolidine-3-carboxylate as a yellowish solid. MS ESI +ve m/z 583 (M+Na).

### Step 7. (S)-tert-butyl 4-((R)-5-tert-butoxy-2-(hydroxymethyl)-5-oxopentyl)-2,2-dimethyloxazolidine-3-carboxylate

(*S*)-*tert*-Butyl 4-((*R*)-5-*tert*-butoxy-2-((*R*)-4-methyl-2-oxooxazolidine-3-carbonyl)-5-oxopentyl)-2,2-dimethyloxazolidine-3-carboxylate (4.12 g, 7.36 mmol) was dissolved in 4:1 THF and methanol (200 mL) and cooled to 0 °C. Sodium borohydride (557 mg, 2 eq) was added slowly. After 10 min., the mixture was warmed up to rt slowly. The mixture was stirred 2 hr at rt. The mixture was concentrated, redissolved in EtOAc (300 mL), washed with 1% HCl solution (100 mL), brine (60 mL), and dried over Na₂SO₄. After filtration and concentration, the residue was purified by ISCO (40 g column, 10-65% EtOAc in Hexanes gradient, check TLC with Ninhydrin stain) to afford 2.86 g of (*S*)-*tert*-butyl *4-((R)-5-tert-*butoxy-2-(hydroxymethyl)-5-oxopentyl)-2,2-dimethyloxazolidine-3-carboxylate as a white solid. MS ESI +m/v 410 (M+Na).

### Step 8. (S)-tert-butyl 4-((R)-5-tert-butoxy-5-oxo-2-(tosyloxymethyl)pentyl)-2,2-dimethyloxazolidine-3-carboxylate

To a solution of (*S*)-*tert*-butyl 4-((*R*)-5-*tert*-butoxy-2-(hydroxymethyl)-5-oxopentyl)-2,2-dimethyloxazolidine-3-carboxylate (244 mg, 0.63 mmol) in anhydrous DCM (6 mL) was added pyridine (2 mL) and catalytic amount of DMAP, the solution was chilled to 0 °C. Tosic chloride (360 mg, 1.88 mmol) was added and stirred at rt overnight. The reaction mixture was diluted with EtOAc (40 mL) and washed with 1 N HCl (2x, 50 ml + 20 ml), followed by H₂O, aq. NaHCO₃, brine, dried over Na₂SO4, and filtered. After evaporation of solvent, the residue was purified on silica gel column, eluted with 0-20% EtOAc in hexane to afford *(S)-tert-*butyl 4-((*R)*-5-*tert*-butoxy-5-oxo-2-(tosyloxymethyl)pentyl)-2,2-dimethyloxazolidine-3-carboxylate (317 mg, yield 93%).

### Step 9. (S)-tert-butyl 4-((R)-5-hydroxy-2-(tosyloxymethyl)pentyl)-2,2-dimethyloxazolidine-3-carboxylate

To a solution of (*S*)-*tert*-butyl4-((*R*)-5-tert-butoxy-5-oxo-2-(tosyloxymethyl)pentyl)-2,2-dimethyloxazolidine-3-carboxylate (317 mg, 0.58 mmol) in anhydrous DCM (8 mL) at -78 °C under N₂ was added DiBAlH (1 M in hexane, 1.75 mL, 1.75 mmol) dropwise. After the addition, the reaction mixture was stirred for another 30 min. The reaction was quenched with MeOH (2 mL), followed by 50% Rochelle's salt aq solution and stirred 2 hr. The resulting solution was extracted with DCM (3 × 20 mL), the combined organic phases were concentrated and dissolved in THF/MeOH (10 mL, 4/1, v/v), and chilled to 0 °C, NaBH₄ (11 mg, 0.29 mmol) was added and stirred at this temperature for 30 min. The reaction was quenched by aqueous NH₄Cl, then extracted with EtOAc (3 × 20 mL), the combined organic phases were washed with H₂O, brine, and dried over Na₂SO₄, and filtered, and concentrated to give crude product *(S)-tert-butyl* 4-((*R*)-5-hydroxy-2-(tosyloxymethyl)pentyl)-2,2-dimethyloxazolidine-3-carboxylate (255 mg, 92%). It was used without further purification.

### Step 10. (S)-tert-butyl 2,2-dimethyl-4-(((R)-tetrahydro-2H-pyran-3-yl)methyl)oxazolidine-3-carboxylate

To a solution of (*S*)-*tert*-butyl 4-((*R*)-5-hydroxy-2-(tosyloxymethyl)pentyl)-2,2-dimethyloxazolidine-3-carboxylate (254 mg, 0.54 mmol) in anhydrous DMF (8 mL) at 0 °C under N₂ was added NaH (43 mg, 1.08 mmol). After stirring at this temperature for 1 hr, the reaction was quenched with aq. NH₄Cl and then evaporated to dryness. The residue was dissolved in EtOAc and H₂O, the separated aqueous phase was extracted with EtOAc. The combined organic phases were washed with H₂O, brine, and dried over Na₂SO₄, filtered, and evaporated. The residue was purified on silica gel column to afford (*S*)-tert-butyl 2,2-dimethyl-4-(((*R*)-tetrahydro-2*H*-pyran-3-yl)methyl)oxazolidine-3-carboxylate (136 mg, 84%).

The following compounds were prepared using procedures analogous to those described above:
1) (*S*)-tert-butyl 4-((*R*)-5-(cyclohexyloxy)-5-oxo-2-((*R*)-2-oxo-4-phenyloxazolidine-3-carbonyl)pentyl-2,2-dimethyloxazolidine-3-carboxylate using (*R*)-4-phenyl-2-oxalozolidinone in Step 5 and cyclohexyl acrylate in Step 6.
2) (*S*)-*tert*-butyl 4-((*R*)-5-ethoxy-5-oxo-2-(tosyloxymethyl)pentyl)-2,2-dimethyloxazolidine-3-carboxylate using (*R*)-4-phenyl-2-oxalozolidinone in Step 5 and using ethyl acrylate in step 6.
3) (*S*)-benzyl 2,2-dimethyl-4-(((*R*)-tetrahydro-2*H*-pyran-3-yl)methyl)oxazolidine-3-carboxylate using benzyl chloroformate in Step 1.

### EXAMPLE 10

### 2,2-dimethyl-4-(((R)-tetrahydro-2H-pyran-3-yl)methyl)oxazolidine

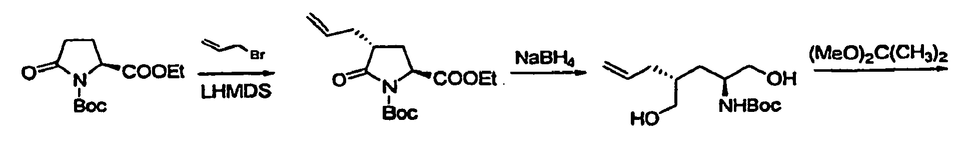

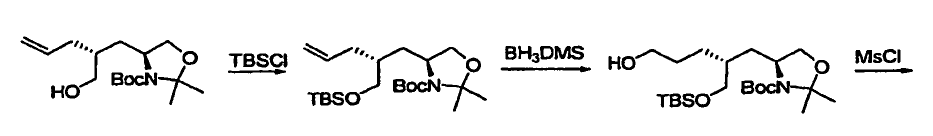

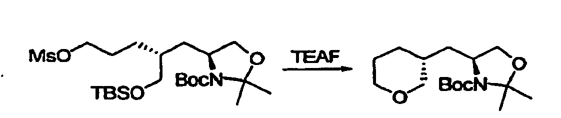

### Step 1. (2S,4R)-1-tert-butyl2-ethyl 4-allyl-5-oxopyrrolidine-1,2-dicarboxylate

To a solution of HMDS in anhydrous THF (200 mL) was added dropwise 2.5 M *n*-BuLi in hexane (130 mL) and the mixture was stirred at -78 °C for 1 hr. To a solution of (S)-1-tert-butyl 2-ethyl 5-oxopyrrolidine-1,2-dicarboxylate (80 g, 0.311 mol) in anhydrous THF (1600 mL) stirred at -78 °C was added lithium hexamethyldisilazide in THF. After the reaction mixture was stirred at -78 °C for 1 hr, 3-brornopropene (38.47 g, 0.318 mol) in THF (200 mL) was added and stirring was continued for 2 hr. The reaction mixture was quenched with saturated ammonium chloride solution (600 mL) at -78 °C and extracted with EtOAc (3 x500 mL). The combined organic layers were dried over Na₂SO₄, filtered and evaporated to dryness. The crude product was separated by column chromatography to afford (2*S*,4*R*)-1-*tert*-butyl 2-ethyl 4-allyl-5-oxopyrrolidine-1,2-dicarboxylate (15 g, 16%).

### Step 2. tert-butyl (2S,4R)-1-hydroxy-4-(hydroxymethyl)hept-6-en-2-ylcarbamate

To a solution of *(2S,4R)-1-tert-butyl* 2-ethyl 4-allyl-5-oxopyrrolidine-1,2-dicarboxylate (30 g, 0.1 mol) in MeOH/H₂O (700/70 mL) was added NaBH₄ (25 g, 0.66 mol), the result mixture was stirred 1 hr at rt and quenched with sat. aq. NH₄Cl (300 mL). The organic solvent was removed under vacuum and extracted with EtOAc (3×250 mL). The combined organic phases were washed with brine (250 mL) and dried over anhydrous Na₂SO₄, filtered and evaporated to afford crude *tert -* butyl (2*S*,4*R*)-1-hydroxy-4-(hydroxymethyl)hept-6-en-2-ylcarbamate (22 g, 85%). It was used in the next step without further purification.

### Step 3. (S)-tert-butyl 4-((R)-2-(hydroxymethyl)pent-4-enyl)-2,2-dimethyloxazolidine-3-carboxylate

To a solution of *tert*-butyl (2*S*,4*R*)-1-hydroxy-4-(hydroxymethyl)hept-6-en-2-ylcarbamate (6.8 g, 26.2 mmol) in acetone (150 mL), PTSA (0.45 g, 2.62 mmol) was added. The reaction mixture was cooled to -20 °C followed by the addition of 2,2-dimethoxypropane (4.1 g, 39.4 mmol). The resulting mixture was stirred and allowed to warm to rt for 1 hr. TEA (0.5 mL) was then added and stirred for another 5 min. The solvent was removed under reduced pressure. The residue was dissolved in Et₂O (300 mL), washed with 1 N HCl (80 mL), sat. aq. NaHCO₃ (80 mL), brine (80 mL) successively, and dried, filtered, and concentrated under vacuum to give crude (*S*)-*tert*-butyl 4-((*R*)-2-(hydroxymethyl)pent-4-enyl)-2,2-dimethyloxazolidine-3-carboxylate (7.5 g, 96%). It was used without further purification.

### Step 4. (S)-tert-butyl 4-((R)-2-((tert-butyldimethylsilyloxy)methyl)pent-4-enyl)-2,2-dimethyloxazolidine-3-carboxylate

To a solution of (*S*)-*tert*-butyl 4-((*R*)-2-(hydroxymethyl)pent-4-enyl)-2,2-dimethyloxazolidine-3-carboxylate (11.5 g, 38.4 mmol), imidazole (7.84 g, 115.2 mmol) and DMAP (234 mg, 1.92 mmol) in CH₂Cl₂ (200 mL) was added a solution of TBSCI (8.68 g, 57.6 mmol) in CH₂Cl₂ (100 mL) dropwise. The reaction mixture was stirred at rt for overnight. The reaction was washed with water (100 mL) and the aqueous layer was extracted with CH₂Cl₂ (3×100mL), the combined organic layers was washed with brine (70 mL), then dried over Na₂SO₄, filtered and concentrated to give the crude product, which was purified by column chromatography to afford (*S*)-*tert*-butyl *4-((R)-2-((tert-*butyldimethylsilyloxy)methyl)pent-4-enyt)-2,2-dimethyloxazolidine-3-carboxylate (9 g, 57%).

### Step 5. (S)-tert-butyl 4-((R)-2-((tert-butyldimethylsilyloxy)methyl)-5-hydroxypentyl)-2,2-dimethyloxazolidine-3-carboxylate

A solution of (*S*)-*tert*-butyl *4-*((*R*)-2-((*tert-*butyldimethylsilyloxy)methyl)pent-4-enyl)-2,2-dimethyloxazolidine-3-carboxylate (26 g, 63 mmol) in THF (200 mL) was cooled in an ice-bath, followed by dropwise addition of 10 M BH_{3.}SMe₂ (6.3 mL). After stirring for 5 hr, 10% NaOH solution (32 mL) followed by 30% H₂O₂ (32 mL) were added carefully. The reaction mixture was stirred at rt for 16 hr. The reaction mixture was diluted with diethyl ether (500 mL) and the aqueous layer was extracted with diethyl ether (3×250 mL). The combined organic layers were washed with brine, dried over Na₂SO₄, filtered and concentrated to give the crude product, which was purified by column chromatography to afford (*S*)-*tert-*butyl 4*-*((*R*)*-*2*-*((*tert-*butyldimethylsilyloxy)methyl)-5-hydroxypentyl)-2,2-dimethyloxazolidine-3-carboxylate (19.6 g, 72%).

### Step 6. (S)-tert-butyl 4-((R)-2-((tert-butyldimethylsilyloxy)methyl)-5-(methylsulfonyloxy)pentyl)-2,2-dimethyloxazolidine-3-carboxylate

To a solution of (*S*)-*tert*-butyl 4-((*R*)-2-((*tert*-butyldimethylsilyloxy)methyl)-5-hydroxypentyl)-2,2-dimethyloxazolidine-3-carboxylate (32 g, 74.2 mmol) and Et₃N (22.5 g, 226 mmol) in CH₂Cl₂ (400 mL) was added a solution of MsCl (10.1 g, 89 mmol) in CH₂Cl₂ (50 mL) at 0-5 °C. After addition, the reaction mixture was allowed to warm to rt and stir for 1 hr. The reaction was washed with water (200 mL) and the aqueous layer was extracted with CH₂Cl₂ (3x150 mL). The combined organic layers was washed with 10% citric acid (60 mL), sat. NaHCO₃ (60 mL) and brine (100 mL), then dried over Na₂SO₄, filtered and concentrated to give *(S)-tert-*butyl 4-((*R*)-2-((*tert*-butyldimethylsilyloxy)methyl)-5-(methylsulfonyloxy)pentyl)-2,2-dimethyloxazolidine-3-carboxylate (37.7 g, 100%), which was used in the next step without purification.

### Step 7. (S)-tert-butyl 2,2-dimethyl-4-(((R)-tetrahydro-2H-pyran-3-yl)methyl)oxazolidine-3-carboxylate

To a solution of (*S*)-*tert*-butyl 4-((*R*)-2-((*tert*-butyldimethylsilyloxy)methyl)-5-(methylsulfonyloxy)pentyl)-2,2-dimethyloxazolidine-3-carboxylate (37.7 g, 74.2 mmol) in THF (1000 mL) was added tetraethylammonium fluoride hydrate (41 g, 185.5 mmol) in portions. The reaction mixture was stirred under reflux overnight. The mixture was diluted with EtOAc (1000 mL), washed with water (300 mL) and brine (500 mL). The organic phase was dried over Na₂SO₄, filtered and concentrated in *vacuo* to give the crude product, which was purified by column chromatography to afford (*S*)-*tert*-butyl 2,2-dimethyl-4-(((*R*)-tetrahydro-2*H*-pyran-3-yl)methyl)oxazolidine-3-carboxylate (12.0 g, 54%).

### EXAMPLE 11

### tert-butyl (S)-1-hydroxy-3-(tetrahydro-2H-pyran-3-yl)propan-2-ylcarbamate

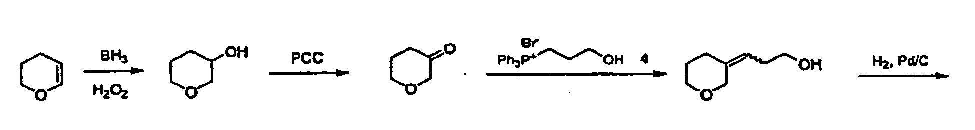

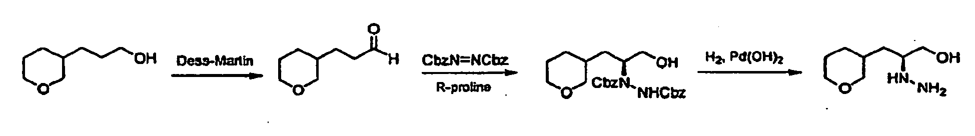

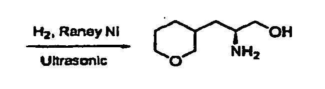

### Step 1. tetrahydro-2H-pyran-3-ol

To the solution of 3,4-dihydro-2*H*-pyran (126 g, 1.5 mol) in dry THF (1350 mL) was added a solution of B₂H₆ in Me₂S (10 M, 75mL, 0.75 mol) under nitrogen atmosphere at 0 °C. The mixture was stirred at this temperature for 3 hr, and then was stirred at 25 °C for another 2 hr. The mixture was warmed to 40-45 °C, and was added aq. NaOH (3 N, 390 mL) and H₂O₂ (30%, 270mL). After stirring for 2 hr, the reaction was quenched by sat. brine. The mixture was filtered, and the filtrate was extracted with EtOAc (3×300 mL). The organic phase was washed with aq. Na₂S₂O₃ (3×100 mL), dried over Na₂SO₄, and concentrated in *vacuo* to give the crude product, which was purified through column chromatography to give tetrahydro-2*H*-pyran-3-ol (72.8 g, 48%). ¹H NMR (CD₃OD) δ 3.7-3.6 (m, 4H), 3.6-3.5 (m, 1H), 3.4-3.3 (m, 1H), 1.9-1.7 (m, 2H), 1.6-1.5 (m, 2H),

### Step 2. dihydro-2H-pyran-3(4H)-one

To the solution of tetrahydro-2*H*-pyran-3-ol (30 g, 0.29 mol) in dry CH₂Cl₂ (900 mL) was added 3Å molecule series (30g) and PCC (94.9g, 0.44mol). The mixture was stirred at rt overnight. When the reaction was over, the mixture was filtered through celite, dried over Na₂SO₄, and concentrated in *vacuo* to give the crude product, which was purified through column chromatography to give dihydro-2*H*-pyran-3(4H)-one (23 g, 76%). ¹H NMR (CD₃OD) δ 3.9 (s, 2H), 3.8-3.7 (t, 2H), 3.7-3.6 (m, 4H), 2.5-2.4 (m, 2H), 2.0-1.9 (m, 2H).

### Step 3. 3-(dihydro-2H-pyran-3(4H)-ylidene)propan-1-ol

To a suspension of the phosphonium salt (69 g, 1.5 eg) in dry THF (1100 mL) at 0 °C under nitrogen atmosphere was added *n*-BuLi (2.5 M, 111 mL, 0.413 mol). The solution was stirred for 1 hr, followed by addition of dihydro-2*H*-pyran-3(4H)-one (11.5 g, 0.115 mol). Stirring was continued at rt overnight. The mixture was quenched by sat. aq. NH₄Cl, and then filtered. The filtrate was dried over Na₂SO₄, and concentrated in *vacuo* to give the crude product, which was purified through column chromatography to give 3-(dihydro-2*H*-pyran-3(4H)-ylidene)propan-1-ol (11.2 g, 69%). ¹H NMR (CD₃OD): δ 4.2-3.9 (d, 2H), ), 3.8-3.5 (m, 4H), 2.4-2.2 (m, 4H), 5.3-5.2 (d, 1H), 2.1-1.8 (s, 1H), 1.8-1.6 (m, 2H).

### Step 4. 3-(tetrahydro-2H-pyran-3-yl)propan-1-ol

To the solution of compound 3-(dihydro-2H-pyran-3(4H)-ylidene)propan-1-ol (11.2 g, 0.0789 mol) in methanol (200 mL) was added Pd(OH)₂/C (1.12g). The reaction flask was degassed and filled into H₂. Stirring was continued until the starting material disappeared. When the reaction was over, the mixture was filtered through celite, and the filter cake was washed with MeOH (2×10 mL). The combined organic layers were dried over Na₂SO₄, and concentrated in vacuo to give 3-(tetrahydro-2*H*-pyran-3-yl)propan-1-ol (10.35g, yield 91 %), which was used for the next step without purification. ¹H NMR (CD₃OD) δ 3.9-3.8 (m, 1H), 3.7-3.6 (m, 2H), 3.5-3.4 (m, 1H), 3.3 (m, 1H), 3.1-2.9 (t, 1H), 2.6-2.4 (m, 1H), 2.3-1.8 (m, 3H), 1.6-1.4 (m, 4H), 1.3-1.0 (m, 2H).

### Step 5. 3-(tetrahydro-2H-pyran-3-yl)propanal

To the solution of 3-(tetrahydro-2*H*-pyran-3-yl)propan-1-ol (10.35 g, 0.0719 mol) in CH₂Cl₂ (200 mL) was added Dess-Martin periodinane (61.24 g, 0.1438 mol). The mixture was stirred at rt. When the reaction was over, the solution was poured into Et₂O (300 mL) and anhydrous K₂CO₃ (19.84 g, 0.1438 mol) was added. The mixture was filtered. The filtrate was dried over Na₂SO₄, and concentrated in *vacuo* to give the crude product, which was purified through column chromatography to give 3-(tetrahydro-2*H*-pyran-3-yl)propanal (8.25 g, 80%).

### Step 6. dibenzyl 1-((2S)-1-hydroxy-3-(tetrahydro-2H-pyran-3-yl)propan-2-yl)hydrazine-1,2-dicarboxylate

To a stirred solution of 3-(tetrahydro-2*H*-pyran-3-yl)propanal (8.25 g, 0.058 mol) and dibenzyl azodicarboxylate (94%, 12.3 g, 0.041 mol) in MeCN (250 mL) at 0 °C was added (*R*-proline) (0.47 g, 0.0041 mol). After stirring the mixture at 0 °C for 15 hr, ethanol (100 mL) and NaBH₄ (1.56 g, 0.041 mol) was added, and the mixture was stirred at 0 °C for 40 min. The reaction was quenched by slow addition of 10% aqueous citric acid (15 ml), and the whole solution was concentrated in *vacuo.* This residue was diluted with EtOAc (200 ml), washed with saturated brine(1×50 mL), dried over Na₂SO₄ and concentrated in *vacuo* to give the crude product, which was purified through column chromatography to give dibenzyl 1-((2*S*)-1-Hydroxy-3-(tetrahydro-2*H*-pyran-3-yl)propan-2-yl)hydrazine-1,2-dicarboxylate (14.68 g, 81%).

### Step 7. (2S)-2-hydrazinyl-3-(tetrahydro-2H-pyran-3-yl)propan-1-ol

To the solution of 1-((2*S*)-1-hydroxy-3-(tetrahydro-2*H*-pyran-3-yl)propan-2-yl)hydrazine-1,2-dicarboxylate (14.68 g, 0.0332 mol) in methanol (250 mL) was added Pd(OH)₂/C (1.47 g). The reaction flask was degassed and filled into H₂. Stirring was continued until the starting material disappeared. When the reaction was over, the mixture was filtered through celite, and the filter cake was washed with MeOH (2×20 mL). The combined organic solvent was dried over Na₂SO₄, and concentrated in *vacuo* to give (2*S*)-2-hydrazinyl-3-(tetrahydro-2*H*-pyran-3-yl)propan-1-ol (5.79 g, 94 %), which was used for the next step without purification.

### Step 8. (2S)-2-amino-3-(tetrahydro-2H-pyran-3-yl)propan-1-ol

To the solution of (2*S*)-2-hydrazinyl-3-(tetrahydro-2*H*-pyran-3-yl)propan-1-ol (5.79 g, 0.033 mol) in MeOH (100 mL) was added Raney Ni. The flask was degassed and equipped with a hydrogen-inflated balloon. The flask was dipped into an ultrasound bath filled with water and sonicated for 4 hr at rt until the starting material was completely consumed. The mixture was then filtered through celite, and the filter cake was washed with MeOH (2×30 mL). Removal under reduced pressure gave (2*S*)-2-amino-3-(tetrahydro-2*H*-pyran-3-yl)propan-1-ol (5.4 g, 90%).

### EXAMPLE 12

### 2-(trimethylsilyl)ethyl (S)-2-amino-3-((R)-tetrahydro-2H-pyran-3-yl)propyl(methyl)carbamate

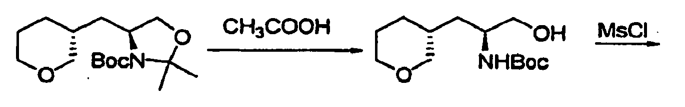

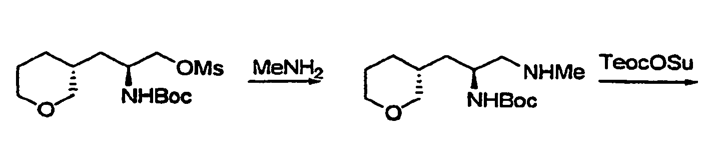

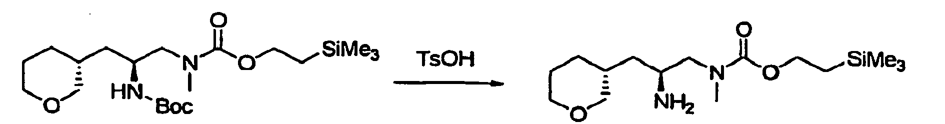

### Step 1. tert-butyl (S)-1-hydroxy-3-((R)-tetrahydro-2H-pyran-3-yl)propan-2-ylcarbamate

(*S*)-*tert*-Butyl-2,2-dimethyl-4-(((*R*)-tetrahydro-2*H*-pyran-3-yl)methyl)oxazolidine- 3-carboxylate (9 g, 30.1 mmol) was dissolved in 80% aq CH₃CO₂H (90ml). The solution was stirred at 50 °C during 1.5 hr and evaporated to dryness at reduced pressure. The residue was dissolved in Et₂O (150ml) and washed with saturated NaHCO₃ (4×100 mL). The organic layer was dried over Na₂SO₄, filtered, and the solvent removed under reduced pressure to give *tert*-butyl (*S*)-1-hydroxy-3-((*R*)-tetrahydro-2*H*-pyran-3-yl)propan-2-ylcarbamate (6.2 g, 79.5%) as an oil ,which was used in the next step without further purification.

### Step 2. (S)-2-(tert-butoxycarbonylamino)-3-((R)-tetrahydro-2H-pyran-3-yl)propyl methanesulfonate

To a solution of *tert*-butyl (*S*)-1-hydroxy-3-((*R*)-tetrahydro-2*H*-pyran-3-yl)propan-2-ylcarbamate (6.2 g, 23.9 mmol) and triethylamine (7.25 g, 71.8 mmol) in CH₂Cl₂ at 0 °C was added mesyl chloride (5.5 g, 47.8 mmol) dropwise. The reaction mixture was stirred at rt until the starting material disappeared. The reaction was quenched with ice-cold water and extracted with CH₂Cl₂ (3×100 ml). The combined organic layers were washed with water (3×50 ml), dried over Na₂SO₄, and concentrated under *vacuo* to give the (*S*)-2-(*tert-*butoxycarbonylamino)-3-((*R*)-tetrahydro-2*H*-pyran-3-yl)propyl methanesulfonate (9 g), which was used for the next step without purification.

### Step 3. tert-butyl (S)-1-(methylamino)-3-((R)-tetrahydro-2H-pyran-3-yl)propan-2-ylcarbamate

To an ethanol solution of MeNH₂ (100 mL) was added *tert*-butyl (*S*)-1-(methylamino)-3-((*R*)-tetrahydro-2*H*-pyran-3-yl)propan-2-ylcarbamate (9 g, 26.7 mmol). The mixture was stirred at 30-40 °C overnight. When the reaction was complete, the solution was concentrated to afford *tert*-butyl (*S*)-1-(methylamino)-3-((*R*)-tetrahydro-2*H*-pyran-3-yl)propan-2-ylcarbamate (10 g), which was used for the further reaction without purification.

### Step 4. (S)-tert-butyl 1-(N-Methyl-2-(trimethylsilyl)ethoxycarbonylamino)-3-((R)-tetrahydro-2H-pyran-3-yl)propylcarbamate

Solid 1-[2-Trimethylsilyl)ethoxycarbonyloxy]pyrrolidin-2,5-dione (9.5 g, 36.7 mmol) was added to a vigorously stirred biphasic solution of the *tert*-butyl (S)-1-(methylamino)-3-((*R*)-tetrahydro-2*H*-pyran-3-yl)propan-2-ylcarbamate (10 g, 36.7 mmol), K₂CO₃ (15.1 g, 110.1mmol), H₂O (50 mL) and CH₂Cl₂ (100 mL). After the reaction was stirred for 2 hr at rt, the reaction was taken up into 65mL of CH₂Cl₂. The solution was washed with aq. NaHCO₃ (3×50 mL) and brine (3×50 mL), then dried over Na₂SO₄. The organic layer was concentrated under vacuum to give the crude product, which was purified through column chromatography to give (*S*)-*tert*-butyl 1-(N-Methyl-2-(trimethylsilyl)ethoxycarbonylamino)-3-((*R*)-tetrahydro-2*H*-pyran-3-yl)propylcarbamate (6 g, 46.2%).

### Step 5. 2-(trimethylsilyl)ethyl (S)-2-amino-3-((R)-tetrahydro-2H-pyran-3-yl)propyl (methyl)carbamate

To a solution of (*S*)-*tert*-butyl 1-(N-Methyl-2-(trimethylsilyl)ethoxycarbonylamino)-3-((*R*)-tetrahydro-2*H*-pyran-3-yl)propylcarbamate (6 g, 14.4 mmol) in Et₂O (100 mL) was added a solution of tosic acid (2.8 g, 14.4 mmol) in 13.0 mL of absolute EtOH. This solution was placed on a rotary evaporator and the Et₂O was removed at ambient temp. The flask was then lowered into a 60 °C water bath and the remainder of the solvent was evaporated over 2 hr to afford a white solid. The solid was cooled to rt and dissolved into 80 mL of a mixture of 1:1 EtOH:H₂O. This was washed with 5:1 Hexanes:EA (3×10mL), basified with 1N NaOH (pH>10), and extracted with Et₂O (3×50 mL). The combined Et₂O extracts were washed with brine (3×5mL), dried over Na₂SO₄, concentrated under vacuum to give 2-(trimethylsilyl)ethyl (*S*)-2-amino-3-((*R*)-tetrahydro-2*H*-pyran-3-yl)propyl(methyl)carbamate 3.3 g (72%).

The following compound was prepared following procedures analogous to those described above:
1) 2-(trimethylsilyl)ethyl (*S*)-2-amino-3-(tetrahydro-2*H*-pyran-3-yl)propyl(methyl)carbamate using *tert*-butyl (*S*)-1-hydroxy-3-(tetrahydro-2*H*-pyran-3-yl)propan-2-ylcarbamate in Step 2.

### EXAMPLE 13

### methyl 2-((R)-(3-chlorophenyl)((R)-1-((S)-1-(methylamino)-3-((R)-tetrahydro-2H-pyran-3-yl)propan-2-ylcarbamoyl)piperidin-3-yl)methoxy)ethylcarbamate (compound 6a)

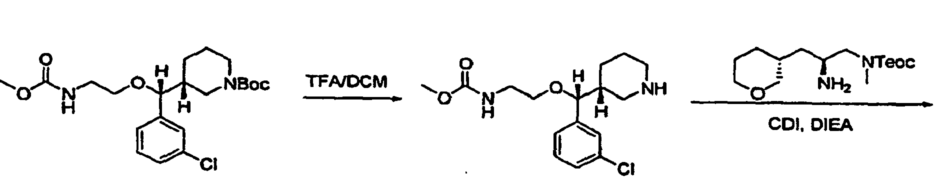

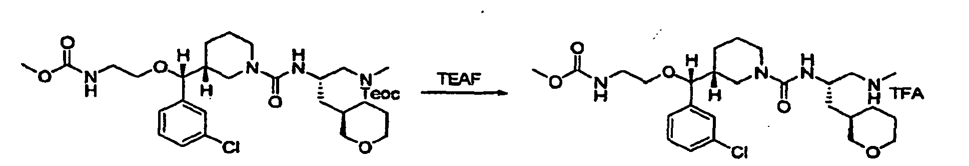

### Step 1. methyl 2-((R)-(3-chlorophenyl)((R)-piperidin-3-yl)methoxy)ethylcarbamate

(*R*)-*tert*-Butyl 3-((*R*)-(3-chlorophenyl)(2-(methoxycarbonylamino)ethoxy)methyl)piperidine-1-carboxylate (4.86 g, 11.4 mmol) was dissolved in a solution of 20% (V/V) TFA/ CH₂Cl₂ (10 mL). The reaction mixture was stirred at rt for 1 hr. The solvent was removed in *vacuo* to afford methyl 2-((*R*)-(3-chlorophenyl)((R)-piperidin-3-yl)methoxy)ethylcarbamate as TFA salt (4.8 g, 100%), which was used for the next step directly without purification.

### Step 2. methyl 2-((R)-(3-chlorophenyl)((R)-1-((S)-1-(N-Methyl-2-(trimethylsilyl)ethoxycarbonylamino)-3-((R)-tetrahydro-2H-pyran-3-yl)propan-2-ylcarbamoyl)piperidin-3-yl)methoxy)ethylcarbamate

At 0 °C, to a solution of 2-(trimethylsilyl)ethyl (*S*)-2-amino-3-((R)-tetrahydro-2*H*-pyran-3-yl)propyl(methyl)carbamate (1.9 g, 6 mmol) and DIPEA (3.87 g, 30 mmol) in anhydrous CH₂Cl₂ (20 mL) was added CDI (1.26 g, 7.8 mmol). After addition, the mixture was stirred for 1 hr at 0 °C, followed by addition of methyl 2-((*R*)-(3-chlorophenyl)((*R*)-piperidin-3-yl)methoxy)ethylcarbamate as TFA salt (2.8 g, 6.6 mmol) in anhydrous CH₂Cl₂ (20 mL). The reaction mixture was allowed to warm to rt and stirred overnight. After the reaction was completed, the solvent was removed in *vacuo.* The product was purified by column chromatography on silica gel eluting with petroleum ether/EtOAc (5:1→2:1) to afford methyl 2-((*R*)-(3-chlorophenyl)((*R*)-1-((*S*)-1-(N-Methyl-2-(trimethylsilyl)ethoxycarbonylamino)-3-((*R*)-tetrahydro-2*H*-pyran-3-yl)propan-2-ylcarbamoyl)piperidin-3-yl)methoxy)ethylcarbamate (3.0 g, 75% yield).

### Step 3. methyl 2-((R)-(3-chlorophenyl)((R)-1-((S)-1-(methylamino)-3-((R)-tetrahydro-2H-pyran-3-yl)propan-2-ylcarbamoyl)piperidin-3-yl)methoxy)ethylcarbamate.trifluoroacetic acid salt

Methyl 2-((*R*)-(3-chlorophenyl)((*R*)-1-((*S*)-1-(N-Methyl-2-(trimethylsilyl)ethoxycarbonylamino)-3-((*R*)-tetrahydro-2*H*-pyran-3-yl)propan-2-ylcarbamoyl)piperidin-3-yl)methoxy)ethylcarbamate (2.9 g, 4.34 mmol) and TEAF (1.42 g, 9.6 mmol) was dissolved in CH₃CN (40 mL). The reaction mixture was heated under reflux for 20 min. Then the mixture was concentrated in *vacuo.* The residue was purified by preparative HPLC to afford methyl 2-((*R*)-(3-chlorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate as TFA salt (2.23 g, 83%).

The following compounds were prepared using procedures analogous to those described above and isloated as their TFA salts:
1) methyl 2-((*R*)-((*R*)-1-((*S*)-1-(methylamino)-3-(tetrahydro-2*H*-pyran-3-yl)propan-2-ylcarbamoyl)piperidin-3-yl)(m-tolyl)methoxy)ethylcarbamate (compound **1**)
2) methyl 2-((*R*)-(3-chloro-4-fluorophenyl)((*R*)-1-((*S*)-1-(methylamino)-3-((*R*)-tetrahydro-2*H*-pyran-3-yl)propan-2-ylcarbamoyl)piperidin-3-yl)methoxy)-ethylcarbamate (compound **8**)
3) ethyl 2-((*R*)-(3-chloro-5-fluorophenyl)((*R*)-1-((*S*)-1-(methylamino)-3-((*R*)-tetrahydro-2*H*-pyran-3-yl)propan-2-ylcarbamoyl)piperidin-3-yl)methoxy)-ethylcarbamate (compound **9**)
4) methyl 2-((*R*)-(5-chloro-2-methylphenyl)((*R*)-1-((*S*)-1-(methylamino)-3-((*R*)-tetrahydro-2*H*-pyran-3-yl)propan-2-ylcarbamoyl)piperidin-3-yl)methoxy)-ethylcarbamate (compound **10**)

### EXAMPLE 14

### methyl 2-((R)-(3-chloro-5-fluorophenyl)((R)-1-((S)-1-(methylamino)-3-((R)-tetrahydro-2H-pyran-3-yl)propan-2-ylcarbamoyl)piperidin-3-yl)methoxy)ethylcarbamate (compound 3a)

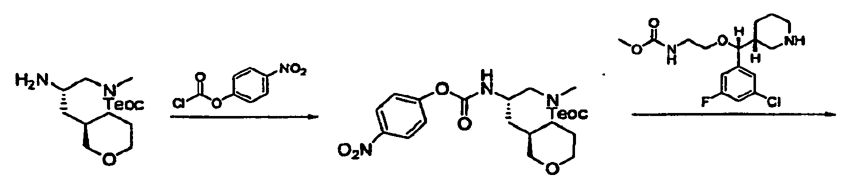

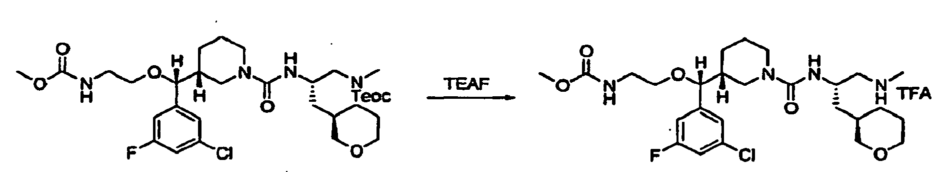

### Step 1. (4-nitrophenyl) (S)-1-(N-methyl-N-(trimethylsilylethoxycarbonyl)amino)-3-((R)-tetrahydro-2H-pyran-3-yl)propan-2-ylcarbamate

A solution of 2-(trimethylsilyl)ethyl (*S*)-2-ainino-3-((*R*)-tetrahydro-2*H*-pyran-3-yl)propyl(methyl)carbamate (0.7350 g, 2.32 mmol, 1.0 equiv, ~7% diastereomeric impurities) in CH₃CN (50 mL) was treated with 4-nitrophenyl chloroformate (0.4950 g, 2.45 mmol, 1.05 equiv) and 0.600 g (7.14 mmol, 3 equiv) of NaHCO₃. The reaction was stirred at rt for 3 hr. The mixture was filtered using Celite® 545. The filtrate was evaporated under reduced pressure to afford 1.1647 g (100%) of (4-nitrophenyl) (*S*)-1-(N-methyl-N-(trimethylsilylethoxycarbonyl)amino)-3-((*R*)-tetrahydro-2*H*-pyran-3-yl)propan-2-ylcarbamate, which was used in the next step without further purification. MS ESI +ve m/z 504 (M+Na).

### Step 2. methyl 2-((R)-(3-chloro-5-fluorophenyl)((R)-1-((S)-1-(N-Methyl-2-(trimethylsilyl)ethoxycarbonylamino)-3-((R)-tetrahydro-2H-pyran-3-yl)propan-2-ylcarbamoyl)piperidin-3-yl)methoxy)ethylcarbamate

A mixture of (*R*)-*tert*-butyl 3-((*R*)-(3-chloro-5-fluorophenyl)(2-(methoxycarbonylamino)ethoxy)methyl)piperidine-1-carboxylate (0.1915 g, 0.43 mmol) in TFA (4 mL) and CH₂Cl₂ (6 mL) was stirred at rt for 2 hr. After the solvents were removed in *vacuo,* the TFA salt of methyl 2-((*R*)-(3-chloro-5-fluorophenyl)((*R*)-piperidin-3-yl)methoxy)ethylcarbamate was directly used in the next step without further purification. MS ESI +ve m/z 345, 347 (M+1).

A mixture of TFA salt of methyl 2-((*R*)-(3-chloro-5-fluorophenyl)((*R*)-piperidin-3-yl)methoxy)ethylcarbamate (0.43 mmol, 1.0 equiv), (4-nitrophenyl) (*S*)-1-(N-methyl-N-(trimethylsilylethoxycarbonyl)amino)-3-((*R*)-tetrahydro-2*H*-pyran-3-yl)propan-2-ylcarbamate (0.2710 g, 0.56 mmol, 1.3 equiv), and DIEA (4 mL) in CH₂Cl₂ was stirred at rt for 19 hr. After the solvents were removed in *vacuo,* the crude product was purified by reversed-phase HPLC (phenomenex® Luna 5µ C18(2) 100A, 250 × 21.20 mm, 5 micron, 70% →90% CH₃CN/H₂O, 0.1% CF₃CO₂H over 8 min and then 90% CH₃CN/H₂O, 0.1 % CF₃CO₂H over 2 min, flow rate 25 mL/min) to afford 0.2840 g (96%) the product as a mixture of diastereoisomers. MS ESI +ve m/z 687, 689 (M+1). The mixture was further separated by chiral HPLC (CHIRALPAK AD-H, 1 cm ø x 25 cm, 10% IPA in hexane with 0.025% diethylamine, flow rate 4 mL/min) to give four fractions in the ratio of 49.8 (t_{R} = 11.00 min): 4.8 (t_{R} = 12.77 min): 43.3 (t_{R} = 13.97 min): 2.1 (t_{R} = 16.23 min). Among them, the two major fractions [methyl 2-((*R*)-(3-chloro-5-fluorophenyl)((*R*)-1-((*S*)-1-(N-Methyl-2-(trimethylsilyl)ethoxycarbonylamino)-3-((*S*)-tetrahydro-2*H*-pyran-3-yl)propan-2-ylcarbamoyl)piperidin-3-yl)methoxy)ethylcarbamate (11 min) and methyl 2-((*R*)-(3-chloro-5-fluorophenyl)((*R*)-1-((*S*)-1-(N-Methyl-2-(trimethylsilyl)ethoxycarbonylamino)-3-((*R*)-tetrahydro-2*H*-pyran-3-yl)propan-2-ylcarbamoyl)piperidin-3-yl)methoxy)ethylcarbamate (13.97 min), were assigned *S* configurations at the amine chiral center and other two minor fractions [methyl 2-((*R*)-(3-chloro-5-fluorophenyl)((3*R*)-1-((*R*)-1-(N-Methyl-2-(trimethylsilyl)ethoxycarbonylamino)-3-(tetrahydro-2*H*-pyran-3-yl)propan-2-ylcarbamoyl)piperidin-3-yl)methoxy)ethylcarbamate Isomer 1(12.77 min) and methyl 2-((*R*)-(3-chloro-5-fluorophenyl)((3*R*)-1-((*R*)-1-(N-Methyl-2-(trimethylsilyl)ethoxycarbonylamino)-3-(tetrahydro-2*H*-pyran-3-yl)propan-2-ylcarbamoyl)piperidin-3-yl)methoxy)ethylcarbamate Isomer 2 (16.23 min), were assigned *R* configurations at the amine chiral center based on stereoselective synthesis of this diamine. The chiral center at 3-pyran portion was finally determined by asymmetric synthesis of the third fraction. For the two minor fractions, however, the chiral centers at 3-pyran portion were not confirmed by asymmetric synthesis.

### Step 3. methyl 2-((R)-(3-chloro-5-fluorophenyl)((R)-1-((S)-1-(methylamino)-3-((R)-tetrahydro-2H-pyran-3-yl)propan-2-ylcarbamoyl)piperidin-3-yl)methoxy)ethylcarbamate

A solution of methyl 2-((*R*)-(3-chloro-5-fluorophenyl)((*R*)-1-((*S*)-1-(N-Methyl-2-(trimethylsilyl)ethoxycarbonylamino)-3-((R)-tetrahydro-2H-pyran-3-yl)propan-2-ylcarbamoyl)piperidin-3-yl)methoxy)ethylcarbamate (0.0948 g) in TFA (5 mL) and CH₂Cl₂ (10 mL) was stirred at rt for 2.5 hr. After the solvents were removed in *vacuo,* the crude product was purified by reversed-phase HPLC (phenomenex® Luna 5µ C18(2) 100A, 250 × 21.20 mm, 5 micron, 10% →90% CH₃CN/H₂O, 0.1% CF₃CO₂H over 13 min, flow rate 25 mL/min) to give 0.0928 g of TFA salt of methyl 2-((*R*)-(3-chloro-5-fluorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate.

Methyl 2-((*R*)-(3-chloro-5-fluorophenyl)((*R*)-1-((*S*)-1-(methylamino)-3-((*S*)-tetrahydro-2*H*-pyran-3-yl)propan-2-ylcarbamoyl)piperidin-3-yl)methoxy)ethylcarbamate (compound **3b**) was prepared following procedures analogous to those described above using 2-(trimethylsilyl)ethyl (*S*)-2-amino-3-((*S*)-tetrahydro-2*H-*pyran-3-yl)propyl(methyl)carbamate in Step 1 and isolated as its TFA salt.

Methyl 2-((*R*)-(3-chloro-5-fluorophenyl)((3*R*)-1-((*R*)-1-(methylamino)-3-(tetrahydro-2*H*-pyran-3-yl)propan-2-ylcarbamoyl)piperidin-3-yl)methoxy)ethylcarbamate Isomers I and 2 (compounds **3c** and **3d**), were prepared following procedures analogous to those described above, using 2-(trimethylsilyl)ethyl (*R*)-2-amino-3-(tetrahydro-2*H*-pyran-3-yl)propyl(methyl)carbamate in Step 1, and isolated as their TFA salts.

The following compounds were prepared using procedures analogous to those described above and isolated as their TFA salts:
1) methyl 2-((*R*)-(3,5-difluorophenyl)((*R*)-1-((*S*)-1-(methylamino)-3-((*S*)-tetrahydro-2*H*-pyran-3-yl)propan-2-ylcarbamoyl)piperidin-3-yl)methoxy)ethylcarbamate (compound **4b**)
2) methyl 2-((*R*)-(3,5-difluorophenyl)((*R*)-1-((*S*)-1-(methylarnino)-3-((*R*)-tetrahydro-2*H*-pyran-3-yl)propan-2-ylcarbamoyl)piperidin-3-yl)methoxy)ethylcarbamate (compound **4a**)
3) methyl 2-((*R*)-(5-fluoro-2-methylphenyl)((*R*)-1-((*S*)-1-(methylamino)-3-((*S*)-tetrahydro-2*H*-pyran-3-yl)propan-2-ylcarbamoyl)piperidin-3-yl)methoxy)-ethylcarbamate (compound **5b**)
4) methyl 2-((*R*)-(5-fluoro-2-methylphenyl)((*R*)-1-((*S*)-1-(methylamino)-3-((*R*)-tetrahydro-2*H*-pyran-3-yl)propan-2-ylcarbamoyl}piperidin-3-yl)methoxy}-ethylcarbamate (compound **5a**)
5) methyl 2-((*R*)-(3-chlorophenyl)((*R*)-1-((*S*)-1-(methylamino)-3-((*S*)-tetrahydro-2*H*-pyran-3-yl)propan-2-ylcarbamoyl)piperidin-3-yl)methoxy)ethylcarbamate (compound **6b**)

### EXAMPLE 15

### methyl 2-((R)-(3-fluorophenyl)((R)-1-((S)-1-(methylamino)-3-((R)-tetrabydro-2H-pyran-3-yl)propan-2-ylcarbamoyl)piperidin-3-yl)methoxy)ethylcarbamate (compound 2a) and methyl 2-((R)-(3-fluorophenyl)((R)-1-((S)-1-(methylamino)-3-((S)-tetrahydro-2H-pyran-3-yl)propan-2-ylcarbamoyl)piperidin-3-yl)methoxy)ethylcarbamate (compound 2b)

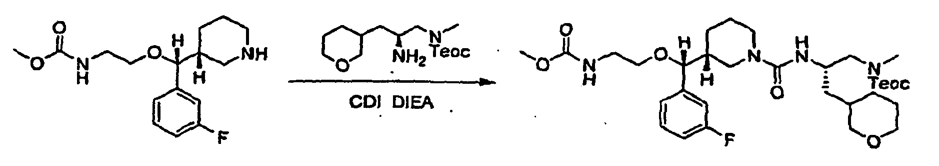

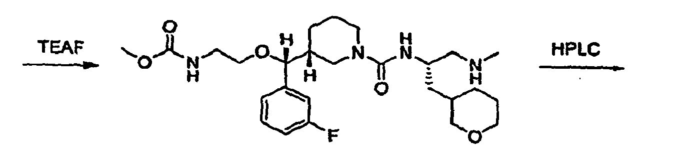

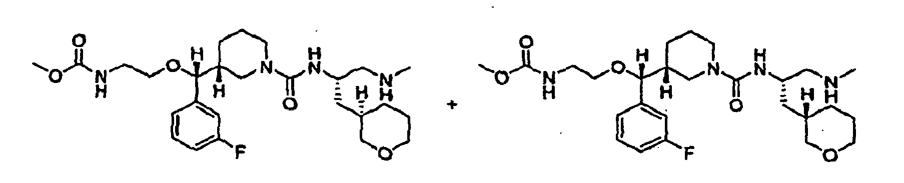

### Step 1. methyl 2-((R)-(3-fluorophenyl)((3R)1-((S)-1-(N-Methyl-2-(trimethylsilyl)ethoxycarbonylamino)-3-(tetrahydro-2H-pyran-3-yl)propan-2-ylcarbamoyl)piperidin-3-yl)methoxy)ethylcarbamate

To a solution of 2-(trimethylsilyl)ethyl (*S*)-2-amino-3-(tetrahydro-2*H*-pyran-3-yl)propyl(methyl)carbamate (300 mg, 0.95 mmol) and CDI (154 mg, 0.95 mmol) in anhydrous CH₂Cl₂ (20 mL), DIEA (612 mg, 4.7 mmol) was added with ice bath. After addition, the mixture was stirred for 1h at 0 °C, then was added to a solution of {2-[(3-fluoro-phenyl)-piperidin-3-yl-methoxy]-ethyl}-carbamic acid methyl ester (245 mg, 0.79 mmol) in anhydrous CH₂Cl₂ (25 mL). The reaction mixture was allowed to warm to rt and stirred overnight. After the reaction was completed, the solvent was removed in *vacuo.* The product was purified by preparative TLC to afford methyl 2-((*R*)-(3-fluorophenyl)((3*R*)-1-((*S*)-1-(N-Methyl-2-(trimethylsilyl)ethoxycarbonylamino)-3-(tetrahydro-2*H*-pyran-3-yl)propan-2-ylcarbamoyl)piperidin-3-yl)methoxy)ethylcarbamate (258 mg, 50 % yield).

### Step 2. methyl 2-((R)-(3-fluorophenyl)((3R)-1-((S)-1-(methylamino)-3-(tetrahydro-2H-pyran-3-yl)propan-2-ylcarbamoyl)piperidin-3-yl)methoxy)ethylcarbamate

A solution of methyl 2-((*R*)-(3-fluorophenyl)((3*R*)-1-((*S*)-1-(*N*-Methyl-2-(trimethylsilyl)ethoxycarbonylamino)-3-(tetrahydro-2*H*-pyran-3-yl)propan-2-ylcarbamoyl)piperidin-3-yl)methoxy)ethylcarbamate (258 mg, 0.40 mmol) in MeCN (25 mL) was treated with TEAF (192 mg, 0.87 mmol) and allowed to stir under reflux for 1 h. The mixture was concentrated in *vacuo* and purified by preparative HPLC to give methyl 2-((*R*)-(3-fluorophenyl)((3*R*)-1-((*S*)-1-(methylamino)-3-(tetrahydro-2*H*-pyran-3-yl)propan-2-ylcarbamoyl)piperidin-3-yl)methoxy)ethylcarbamate as trifluoroacetic acid salt. (162 mg, 81 % yield).

### Step 3. methyl 2-((R)-(3-fluorophenyl)((R)-1-((S)-1-(methylamino)-3-((R)-tetrahydro-2H-pyran-3-yl)propan-2-ylcarbamoyl)piperidin-3-yl)methoxy)ethylcarbamate and methyl 2-((R)-(3-fluorophenyl)((R)-1-((S)-1-(methylamino)-3-((S)-tetrahydro-2H-pyran-3-yl)propan-2-ylcarbamoyl)piperidin-3-yl)methoxy)ethylcarbamate

A solution of methyl 2-((*R*)-(3-fluorophenyl)((3*R*)-1-((*S*)-1-(methylamino)-3-(tetrahydro-2*H*-pyran-3-yl)propan-2-ylcarbamoyl)piperidin-3-yl)methoxy)ethylcarbamate as trifluoroacetic acid salt in CH₂Cl₂ (5 mL) was washed with 1 M NaOH (2 mL, 2x). The aqueous layer was extracted with CH₂Cl₂ (1 mL, 3x) and the combined organic fractions were washed with water, brine, and dried over sodium sulfate. The filtrate was evaporated to afford the free base. The crude product was separated via chiral HPLC (CHIRALPAK AD-H, 1 cm ø x 25 cm, 10% IPA in hexane with 0.025% diethylamine, flow rate 4 mL/min) to afford two isomers, methyl 2-((*R*)-(3-fluorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate (78.57 mg, t_{R}=20.70 min) and methyl 2-((*R*)-(3-fluorophenyl)((*R*)-1-((*S*)-1-(methylamino)-3-((*S*)-tetrahydro-2*H*-pyran-3-yl)propan-2-ylcarbamoyl)piperidin-3-yl)methoxy)ethylcarbamate (90.9 mg, t_{R}= 29.63 min). Step 4. methyl 2-((*R*)-(3-fluorophenyl)((*R*)-1-((*S*)-1-(methylamino*)-3-((R)-*tetrahydro-2*H*-pyran-3-yl)propan-2-ylcarbamoyl)piperidin-3-yl)methoxy)ethylcarbamate fumaric acid salt

An ethanol solution of methyl 2-((*R*)-(3-fluorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate (71.2 mg, 0.14 mmol) was treated with fumaric acid (16.3 mg, 0.14 mmol). The solvent was removed in *vacuo* and the residue redissolved in water. The solution was frozen using a dry ice-acetone bath and placed on a lyopholizer to afford methyl 2-((*R*)-(3-fluorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate fumaric acid salt (87.46 mg) as a white solid.

### Step 5. methyl 2-((R)-(3-fluorophenyl)((R)-1-((S)-1-(methylamino)-3-((S)-tetrahydro-2H-pyran-3-yl)propan-2-ylcarbamoyl)piperidin-3-yl)methoxy)ethylcarbamate fumaric acid salt

An ethanol solution of methyl 2-((*R*)-(3-fluorophenyl)((*R*)-1-((*S*)-1-(methylamino)-3-((*S*)-tetrahydro-2*H*-pyran-3-yl)propan-2-ylcarbamoyl)piperidin-3-yl)methoxy)ethylcarbamate (87.2 mg, 0.17 mmol) was treated with fumaric acid (19.8 mg, 0.17 mmol). The solvent was removed in *vacuo* and the residue redissolved in water. The solution was frozen using a dry ice-acetone bath and placed on a lyopholizer to afford methyl 2-((*R*)-(3-fluorophenyl)((*R*)-1-((*S*)-1-(methylamino)-3-((*S*)-tetrahydro-2*H* pyran-3-yl)propan-2-ylcarbamoyl)piperidin-3-yl)methoxy)ethylcarbamate fumaric acid salt (106.8 mg) as a white solid.

### EXAMPLE 16

### methyl 2-((R)-((R)-1-((S)-1-(methylamino)-3-((R)-tetrahydro-2H-pyran-3-yl)propan-2-ylcarbamoyl)piperidin-3-yl)(phenyl)methoxy)ethylcarbamate (compound 7)

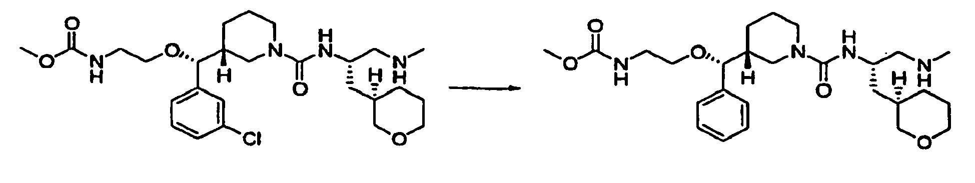

### Step 1. methyl 2-((R)-((R)-1-((S)-1-(methylamino)-3-((R)-tetrahydro-2H-pyran-3-yl)propan-2-ylcarbamoyl)piperidin-3-yl)(phenyl)methoxy)ethylcarbamate

A mixture of methyl 2-((*R*)(3-chlorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate (0.0027 g), HCO₂NH₄ (0.7350 g), and 10% Pd/C (0.0545 g) in MeOH was stirred at rt for 3 hr. The mixture was filtered off precipitates through filter agent, Celite® 545 and washed with MeOH. After the solvent was evaporated under reduced pressure, the crude product was purified by reversed-phase HPLC (phenomenex® Luna 5µ C18(2) 100A, 250 × 21.20 mm, 5 micron, 10% →90% CH₃CN/H₂O, 0.1% CF₃COOH over 13 min, flow rate 25 mL/min) to give TFA salt of methyl 2-((*R*)-((*R*)-1-((*S*)-1-(methylamino)-3-((*R*)-tetrahydro-2*H*-pyran-3-yl)propan-2-ylcarbamoyl)piperidin-3-yl)(phenyl)methoxy)ethylcarbamate. MS ESI +ve m/z 491 (M+1).

### EXAMPLE 17

### methyl 2-((R)-(3-chlorophenyl)((R)-1-((S)-1-(methylamino)-3-((R)-tetrahydro-2H-pyran-3-yl)propan-2-ylcarbamoyl)piperidin-3-yl)methoxy)ethylcarbamate fumaric acid salt

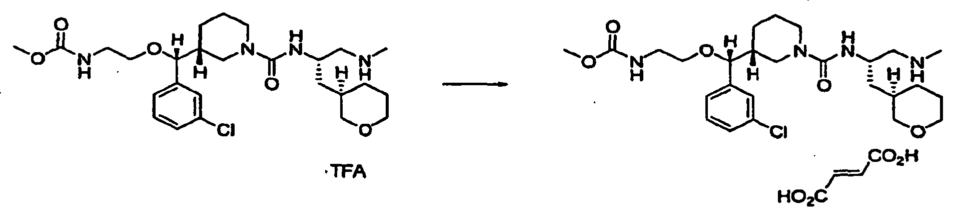

### Step 1. methyl 2-((R)-(3-chlorophenyl)((R)-1-((S)-1-(methylamino)-3-((R)-tetrahydro-2H-pyran-3-yl)propan-2-ylcarbamoyl)piperidin-3-yl)methoxy)ethylcarbamate

The TFA salt of methyl 2-((*R*)-(3-chlorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate (2.2300 g, 3.49 mmol) was treated with 10 mL of 1 *N* NaOH. The mixture was extracted with CH₂Cl₂ (4 ×) and dried over K₂CO₃. After the solvent was removed in *vacuo,* the residue was dissolved into Et₂O and filtered through HPLC filter. The filtrate was evaporated under reduced pressure and the residue was dried in *vacuo* to give 1.6806 g (3.20 mmol, 92%) methyl 2-((R)-(3-chlorophenyl)((R)-1-((S)-1-(methylamino)-3-((R)-tetrahydro-2H-pyran-3-yl)propan-2-ylcarbamoyl)piperidin-3-yl)methoxy)ethylcarbamate as free base. ¹H NMR (CD₃OD, 400 MHz) δ 7.27-7.13 (m, 4H), 4.05 (br d, *J*= 13.5 Hz, 1H), 3.92 (d, *J*= 9.1 Hz, 1H), 3.89-3.83 (m, 2H), 3.79-3.70 (m, 2H), 3.53 (s, 3H), 3.32-3.26 (m, 1H), 3.18-3.13 (m, 4H), 3.01 (dd, *J*= 10.8, 10.0 Hz, 1H), 2.88-2.75 (m, 2H), 2.53-2.44 (m, 2H), 2.29 (s, 3H), 1.78-1.47 (m, 6H), 1.30-1.03 (m, 6H).

### Step 2. methyl 2-((R)-(3-chlorophenyl)((R)-1-((S)-1-(methylamino)-3-((R)-tetrahydro-2H-pyran-3-yl)propan-2-ylcarbamoyl)piperidin-3-yl)methoxy)ethylcarbamate fumaric acid salt

The free base of methyl 2-((*R*)-(3-chlorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate (1.6806 g, 3.20 mmol) and fumaric acid (0.3713 g, 3.20 mmol) were dissolved into EtOH and the solution was evaporated under reduced pressure. The residue was dissolved into H₂O, frozen in a dry ice-acetone bath, and dried by lyophilization to provide fumarate salt of methyl 2-((*R*)-(3-chlorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate as a white powder. ¹H NMR (CD₃OD, 400 MHz) δ 7.27-7.13 (m, 4H), 6.59 (s, 1.76H), 4.04 (br d, *J* = 12.0 Hz, 1H), 3.99-3.96 (m, 1H), 3.92 (d, *J* = 9.1 Hz, 1H), 3.82-3.73 (m, 3H), 3.53 (s, 3H), 3.35-3.28 (m, 1H), 3.18-3.12 (m, 4H), 3.03 (dd, *J =* 10.8, 10.0 Hz, 1H), 2.97 (dd, *J =* 12.6, 3.5 Hz, 1H), 2.93-2.78 (m, 3H), 2.62 (s, 3H), 1.79-1.48 (m, 6H), 1.45-1:02 (m, 6H).

### EXAMPLE 18

### methyl 2-((R)-(3-chlorophenyl)((R)-1-((S)-1-(methylamino)-3-((R)-tetrahydro-2H-pyran-3-yl)propan-2-ylcarbamoyl)piperidin-3-yl)methoxy)ethylcarbamate L-tartaric acid salt

### Step 1. methyl 2-((R)-(3-chlorophenyl)((R)-1-((S)-1-(methylamino)-3-((R)-tetrahydro-2H-pyran-3-yl)propan-2-ylcarbamoyl)piperidin-3-yl)methoxy)ethylcarbamate L-tartaric acid salt

The free base of methyl 2-((*R*)-(3-chlorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate (0.28 g, 0.53 mmol) and *L*-tartaric acid (84.4 mg, 0.56 mmol, 99.5%) were dissolved in ethanol (5 mL) to give a clear solution. The solvent was removed in vacuo to dryness, and the residue was redissolved in 95% ethanol: MeCN (3:97 v/v) (10.5 mL) at 35 °C. A seed crystal was added and the resulting solution was stirred at 35 °C for 2 hr, then cooled to rt slowly, and stirred for 48 hr. The resulting white crystal was filtered and washed with MeCN (2 x 5 mL) to give 1:1 *L*-tartrate of methyl 2-((*R*)-(3-chlorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate (0.31 g, 84%). Selected ¹H NMR (CD₃OD, 400 MHz,) δ: 7.36 (m, 3 H), 7.22 (d, 1 H), 4.40 (s, 2 H), 4.18-4.00 (m, 3 H), 3.86 (m, 3 H), 3.62 (s, 3 H), 3.40 (m, 1 H), 3.24 (m, 3 H), 3.18-2.84 (m, 5 H), 2.72 (s, 3 H), 1.90-1.08 (m, 12 H); mp = 122-127 °C. MS ESI +ve m/z 525 (M+1).

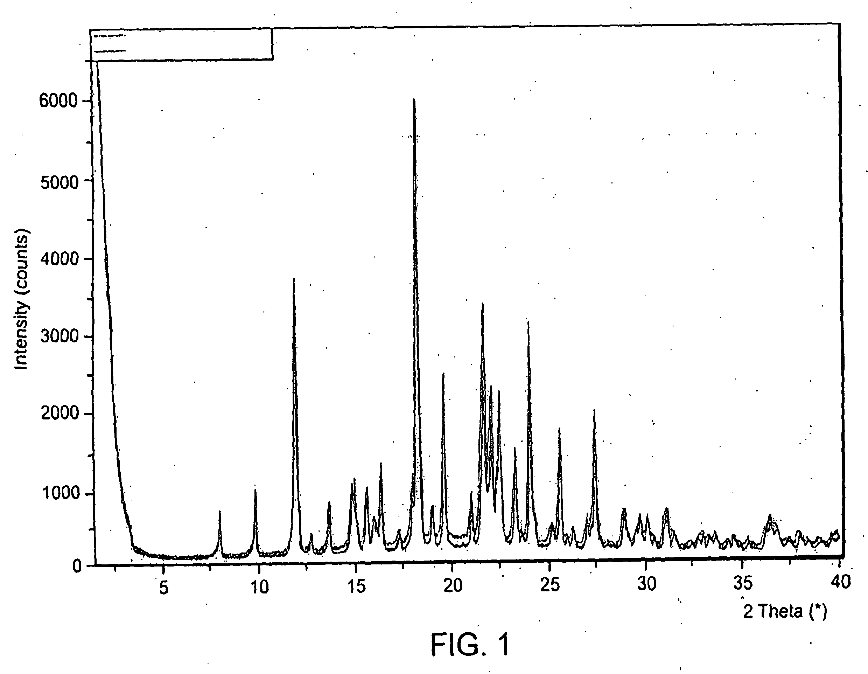

X-ray powder diffraction of two batches of 1:1 methyl 2-((*R*)-(3-chlorophenyl)((*R*)-1-((*S*)-1-(methylamino)-3-((*R*)-tetrahydro-2*H*-pyran-3-yl)propan-2-ylcarbamoyl)piperidin-3-yl)methoxy)ethylcarbamate L-tartaric acid salt is shown in Figure 1.

The following are examples of aspartic protease inhibitors of the invention. When the stereochemistry at a chiral center is not defined in the compound name, this indicates that the sample prepared contained a mixture of isomers at this center.

**Table of Compounds**

| Cpd. No. | Cpd Name | LC-MS^{a} (3min) t_{R} (min) | Mass Observed | Selected ¹H NMR^{b} |
|---|---|---|---|---|
| 1 | methyl 2-((*R*)-((*R*)-1-((*S*)-1-(methylamino)-3-(tetrahydro-2*H*-pyran-3-yl)propan-2-ylcarbamoyl)-piperidin-3-yl)(m-tolyl)methoxy)ethyl carbamate | 1.942 | 505 (M+1) | 7.21 (m, 1H), 7.11 (m, 3H), 4.12 (m, 2H), 3.87 (m, 4H), 3.61 (s, 3H), 3.41 (m, 1H), 2.95 (m, 3H), 2.72 (s, 3H), 2.34 (s, 3H) |
| 2b | methyl 2-((*R*)-(3-fluorophenyl)((*R*)-1-((*S*)-1-(methylamino)-3-((*S*)-tetrahydro-2*H* pyran-3-yl)propan-2-ylcarbamoyl)-piperidin-3-yl)methoxy)-ethylcarbamate | | | 7.26 (q, 1), 7.01-6.83 (m, 3), 3.51 (s, 3), 2.61 (s, 3) |
| 2a | methyl 2-((*R*)-(3-fluorophenyl)((*R*)-1-((*S*)-1-(methylamino)-3-((*R*)-tetrahydro-2*H*-pyran-3-yl)propan-2-ylcarbamoyl)-piperidin-3-yl)methoxy)-ethylcarbamate | | | 7.25 (q, 1), 7.01-6.89 (m, 3), 3.50 (s, 3), 2.59 (s, 3) |
| 3a | methyl 2-((*R*)-(3chloro-5-fluorophenyl)((*R*)-1-((*S*)-1-(methylamino)-3-((*R*)-tetrahydro-2*H* pyran-3-yl)propan-2-ylcarbamoyl)-piperidin-3-yl)methoxy)-ethylcarbamate | 1.34 | 543, 545 (M+1) | 7.12 (m, 1H), 7.08-7.06 (m, 1H), 7.00-6.98 (m, 1H), 4.03-3.95 (m, 2H), 3.97 (d, J = 9.1 Hz, 1H), 3.83-3.74 (m, 3H), 3.55 (s, 3H), 3.36-2.82 (m, 10H), 2.63 (s, 3H), 1.80-1.11 (m, 12H). |
| 3b | methyl 2-((*R*)-(3-chloro-5-fluorophenyl)((R)-1-((*S*)-1-(methylamino)-3-((*S*)-tetrahydro-2*H*-pyran-3-yl)propan-2-ylcarbamoyl)-piperidin-3-yl)methoxy)-ethylcarbamate | 1.34 | 543, 545 (M+1) | 7.08 (m, 1H), 7.03-7.01 (m, 1H), 6.95-6.93 (m, 1H), 4.08-3.96 (m, 2H), 3.91 (d, J = 8.8 Hz, 1H), 3.82-3.73 (m, 3H), 3.49 (s, 3H), 3.32-2.67 (m, 10H), 2.59 (s, 3H), 1.81-1.02 (m, 12H). |
| **4b** | methyl 2-((*R*)-(3,5-difluorophenyl)((R) -1-((*S*)-1-(methylamino)-3-((S)-tetrahydro-2*H*-pyran-3-yl)propan-2-ylcarbamoyl)-piperidin-3-yl)methoxy)-ethylcarbamate | 1.26 | 527 (M+1) | 6.87-6.76 (m, 3H), 4.10-3.99 (m, 2H), 3.95 (d, J = 8.8 Hz, 1H), 3.84-3.75 (m, 3H), 3.52 (s, 3H), 3.35-2.17 (m, 10H), 2.62 (s, 3H), 1.84-1.05 (m, 12H). |
| **4a** | methyl 2-((*R*)-(3,5-difluorophenyl)((*R*) -1-((*S*)-1-(methylamino)-3-((*R*)-tetrahydro-2*H*-pyran-3-yl)propan-2-ylcarbamoyl)-piperidin-3-yl)methoxy)-ethylcarbamate | 1.29 | 527 (M+1) | 6.87-6.76 (m, 3H), 4.02-3.98 (m, 2H), 3.96 (d, J = 9.1 Hz, 1H), 3.80-3.73 (m, 3H), 3.53 (s, 3H), 3.34-2.78 (m, 10H), 2.61 (s, 3H), 1.78-1.09 (m, 12H). |
| **5b** | methyl 2-((*R*)-(5-fluoro-2-methylphenyl)((*R*)-1-((*S*)-1-(methylamino)-3-((*S*)-tetrahydro-2*H*-pyran-3-yl)propan-2-ylcarbamoyl)-piperidin-3-yl)methoxy)-ethylcarbamate | 1.25 | 523 (M+1) | 7.10-7.08 (m, 1H), 7.00-6.97 (m, 1H), 6.84-6.79 (m, 1H), 4.25 (d, J = 9.4 Hz, 1H), 4.04-3.97 (m, 2H), 3.79-3.72 (m, 3H), 3.48 (s, 3H), 3.31-2.72 (m, 10H), 2.59 (s, 3H), 2.19 (s, 3H), 1.81-1.05 (m, 12H). |
| **5a** | methyl 2-((*R*)-(5-fluoro-2-methylphenyl)((*R*)-1-((*S*)-1-(methylamino)-3-((*R*)-tetrahydro-2*H*-pyran-3-yl)propan-2-ylcarbamoyl)piperi din-3-yl)methoxy)ethylca rbamate | 1.25 | 523 (M+1) | 7.09-7.07 (m, 1H), 7.00-6.97 (m, 1H), 6.84-6.79 (m, 1H), 4.26 (d, J = 9.2 Hz, 1H), 3.98-3.95 (m, 2H), 3.77-3.66 (m, 3H), 3.49 (s, 3H), 3.31-2.77 (m, 10H), 2.58 (s, 3H), 2.19 (s, 3H), 1.75-1.07 (m, 12H). |
| **6b** | methyl 2-((*R*)-(3-chlorophenyl)((*R*)-1-((*S*)-1-(methylamino)-3-((*S*)-tetrahydro-2*H*-pyran-3-yl)propan-2-ylcarbamoyl)-piperidin-3-yl)methoxy)-ethylcarbarnate | 1.26 | 525, 527 (M+1) | 7.18-7.04 (m, 4H), 4.04-3.93 (m, 2H), 3.83 (d, J = 9.1 Hz, 1H), 3.77-3.68 (m, 3H), 3.44 (s, 3H), 3.27-2.64 (m, 10H), 2.54 (s, 3H), 1.77-0.97 (m, 12H). |
| **6a** | methyl 2-((*R*)-(3-chlorophenyl)((*R*) 1-((*S*)-1-(methylamino)-3-((*R*)-tetrahydro-2*H*-pyran-3-yl)propan-2-ylcarbamoyl)-piperidin-3-yl)methoxy)-ethylcarbamate | 1.26 | 525, 527 (M+1) | 7.23-7.10 (m, 4H), 4.02-3.93 (m, 2H), 3.89 (d, J = 8.8 Hz, 1H), 3.78-3.70 (m, 3H), 3.50 (s, 3H), 3.31-2.76 (m, 10H), 2.59 (s, 3H), 1.76-1.02 (m, 12H). |
| **3c** | methyl 2-((*R*)-(3-chloro-5-fluorophenyl)((*R*)-1-((*R*)-1-(methylamino)-3-(tetrahydro-2*H*-pyran-3-yl)propan-2-ylcarbamoyl)-piperidin-3-yl)methoxy)ethylca rbamate Isomer 1 | 1.33 | 543, 545 (M+1) | 7.10-6.92 (m, 3H), 4.22 (dm, J = 11.1 Hz, 1H), 4.06-3.99 (m, 1H), 3.95 (d, J = 8.2 Hz, 1H), 3.76-3.67 (m, 3H), 3.52 (s, 3H), 3.32-2.76 (m, 10H), 2.61 (s, 3H), 1.86-1.03 (m, 12H). |
| **3d** | methyl 2-((*R*)-(3-chloro-5-fluorophenyl)((*R*)-1-((*R*)-1-(methylamino)-3-(tetrahydro-2*H*-pyran-3-yl)propan-2-ylcarbamoyl)-piperidin-3-yl)methoxy)ethylca rbamate Isomer 2 | 1.36 | 543, 545 (M+1) | 7.10-6.93 (m, 3H), 4.23 (dm, J = 11.7 Hz, 1H), 3.99-3.93 (m, 1H), 3.95 (d, J = 8.8 Hz, 1H), 3.80-3.69 (m, 3H), 3.51 (s, 3H), 3.33-2.75 (m, 10H), 2.60 (s, 3H), 1.75-1.07 (m, 12H). |
| **7** | methyl 2-((*R*)-((*R*)-1-((*S*)-1-(methylamino)-3-((*R*)-tetrahydro-2*H*-pyran-3-yl)propan- 2-ylcarbamoyl)-piperidin-3-yl)-(phenyl)methoxy)-ethylcarbamate | 1.25 | 491 (M+1) | |
| **8** | methyl 2-((*R*)-(3-chloro-4-fluorophenyl)((*R*)-1-((*S*)-1-(methylamino)-3-((*R*)-tetrahydro-2*H*-pyran-3-yl)propan-2-ylcarbamoyl)-piperidin-3-yl)methoxy)-ethylcarbamate | 1.903 | 543 (M+) | 7.44 (m, 1H), 7.23 (m, 2H), 4.05 (m, 3H), 3.86 (m, 3H), 3.61 (s, 3H), 3.40 (m, 1H), 3.10 (m, 3 H), 2.94 (m, 2H), 2.71 (s, 3H) |
| **9** | ethyl 2-((*R*)-(3-chloro-5-fluorophenyl)((*R*)-1-((*S*)-1-(methylamino)-3-((*R*)-tetrahydro-2*H* pyran-3-yl)propan-2-ylcarbamoyl)-piperidin-3-yl)methoxy)ethylca rbamate | 2.045 | 557 (M+) | 7.20 (s, 1H), 7.14 (m, 1H), 7.07 (m, 1H), 4.08 (m, 5H), 3.87 (m, 3H), 3.10 (m, 3H), 2.90 (m, 2H), 2.71 (s, 3H), 1.23 (t, 3H) |
| **10** | methyl 2-((*R*)-(5-chloro-2-methylphenyl)((*R*)-1-((*S*)-1-(methylamino)-3-((*R*)-tetrahydro-2*H*-pyran-3-yl)propan-2-ylcarbamoyl)-piperidin-3-yl)methoxy)-ethylcarbamate | 1.993 | 539 (M+) | 7.31 (s, 1H), 7.13 (m, 2H), 4.31 (m, 1H), 4.23 (m, 1H), 3.61 (s, 3H), 3.12 (m, 1H), 2.86 (m, 2H), 2.67 (m, 2H), 2.45 (s, 3H), 2.31 (s, 3H). |

| | | | | |
|---|---|---|---|---|
| a. LC-MS (3 min) method Column: Chromolith SpeedRod, RP-18e, 50 x 4.6 mm; Mobil phase: A: 0.01 %TFA/water, B: 0.01%TFA/CH₃CN; Flow rate: 1 mL/min; Gradient: | | | | |

| Time (min) | A% | B% |
|---|---|---|
| 0.0 | 90 | 10 |
| 2.0 | 10 | 90 |
| 2.4 | 10 | 90 |
| 2.5 | 90 | 10 |
| 3.0 | 90 | 10 |

| | | |
|---|---|---|
| b. CD₃OD or MeOD was used as ¹H NMR solvent. | | |

### EXAMPLE 19

### IN VITRO ACTIVITY STUDIES

The disclosed aspartic protease inhibitors have enzyme-inhibiting properties. In particular, they inhibit the action of the natural enzyme renin. The latter passes from the kidneys into the blood where it effects the cleavage of angiotensinogen, releasing the decapeptide angiotensin I which is then cleaved in the blood, lungs, the kidneys and other organs by angiotensin converting enzyme to form the octapeptide angiotensin II. The octapeptide increases blood pressure both directly by binding to its receptor, causing arterial vasoconstriction, and indirectly by liberating from the adrenal glands the sodium-ion-retaining hormone aldosterone, accompanied by an increase in extracellular fluid volume. That increase can be attributed to the action of angiotensin II. Inhibitors of the enzymatic activity of renin bring about a reduction in the formation of angiotensin I. As a result a smaller amount of angiotensin II is produced. The reduced concentration of that active peptide hormone is the direct cause of the hypotensive effect of renin inhibitors.

The action of renin inhibitors *in vitro* was demonstrated experimentally by means of a test which measures the increase in fluorescence of an internally quenched peptide substrate. The sequence of this peptide corresponds to the sequence of human angiotensinogen. The following test protocol was used. All reactions were carried out in a flat bottom white opaque microtiter plate. A 4 µL aliquot of 400 µM renin substrate (DABCYL-γ-Abu-Ile-His-Pro-Phe-His-Leu-Val-Ile-His-Thr-EDANS) in 192 µL assay buffer (50 mM BES, 150 mM NaCl, 0.25 mg/mL bovine serum albumin, pH7.0) was added to 4 µL of test compound in DMSO at various concentrations ranging from 10 µM to 1 nM final concentrations. Next, 100 µL of trypsin-activated recombinant human renin (final enzyme concentration of 0.2-2 nM) in assay buffer was added, and the solution was mixed by pipetting. The increase in fluorescence at 495 nm (excitation at 340 nm) is measured for 60-360 minutes at rt using a Perkin-Elmer Fusion microplate reader. The slope of a linear portion of the plot of fluorescence-increase as a function of time was then determined, and the rate was used for calculating percent inhibition in relation to uninhibited control. The percent inhibition values were plotted as a function of inhibitor concentration, and the IC₅₀ is determined from a fit of this data to a four parameter equation. The IC₅₀ was defined as the concentration of a particular inhibitor that reduces the formation of product by 50% relative to a control sample containing no inhibitor. In the *in vitro* systems the disclosed aspartic protease inhibitors exhibit inhibiting activities at minimum concentrations of from approximately 5 x 10⁻⁵ M to approximately 10⁻¹² M. Specific aspartic protease inhibitors exhibit inhibiting activities at minimum concentrations of from approximately 10⁻⁷ M to approximately 10⁻¹² M. (Wang G. T. et al. Anal. Biochem. 1993, 210, 351; Nakamura, N. et al. J. Biochem. (Tokyo) 1991, 109, 741; Murakami, K. et al. Anal Biochem. 1981, 110, 232).

The action of renin inhibitors *in vitro* in human plasma was demonstrated experimentally by the decrease in plasma renin activity (PRA) levels observed in the presence of the compounds. Incubations mixtures contained in the final volume of 250 µL 95.5 mM N,N-bis(2-hydroxyethyl)-2-aminoethanesulfonic acid, pH 7.0, 8 mM EDTA, 0.1 mM neomycin sulfate, 1 mg/mL sodium azide, 1 mM phenylmethanesulfonyl fluoride, 2% DMSO and 87.3% of pooled mixed-gender human plasma stabilized with EDTA. For plasma batches with low PRA (less than 1 ng/ml/hr) ~2 pM of recombinant human renin was added to achieve PRA of 3-4 ng/ml/hr. The cleavage of endogenous angiotensinogen in plasma was carried out at 37°C for 90 min and the product angiotensin I was measured by competitive radioimmunoassay using DiaSorin PRA kit. Uninhibited incubations containing 2% DMSO and fully inhibited controls with 2 µM of isovaleryl-Phe-Nle-Sta-Ala-Sta-OH were used for deriving percent of inhibition for each concentration of inhibitors and fitting dose-response data into a four parametric model from which IC₅₀ values, defined as concentrations of inhibitors at which 50% inhibition occurs, were determined.

The *in vitro* enzyme activity studies were carried out for compounds **1, 2a, 2b, 3a, 3b, 3c, 3d, 4a, 4b, 5a, 5b, 6a, 6b, 7, 8, 9 and 10** and the data is shown in Table 1.

**Table 1. In vitro IC₅₀ and PRA data for aspartic protease inhibitors.**

| **Cpd No.** | **IC₅₀** | **PRA** |
|---|---|---|
| 1 | *** | *** |
| 2a | *** | *** |
| 2b | *** | ** |
| 3a | **** | **** |
| 3b | *** | *** |
| 3c | *** | *** |
| 3d | ** | ** |
| 4a | *** | *** |
| 4b | *** | * |
| 5a | *** | *** |
| 5b | ** | * |
| 6a | **** | **** |
| 6b | *** | *** |
| 7 | ** | * |
| 8 | *** | *** |
| 9 | *** | * |
| 10 | *** | **** |

| | | |
|---|---|---|
| * represents less than 50 nM; ** represents less than 20 nM; *** represents less than 10 nM; **** represents less than 1 nM. | | |

### EXAMPLE 20

### IN VIVO ACTIVITY STUDIES

The cardiac and systemic hemodynamic efficacy of selective renin inhibitors can be evaluated *in vivo* in sodium-depleted, normotensive cynomolgus monkeys. Arterial blood pressure is monitored by telemetry in freely moving, conscious animals.

Cynomolgus Monkey (prophetic example): Six male naïve cynomolgus monkeys weighing between 2.5 and 3.5 kg are to be used in the studies. At least 4 weeks before the experiment, the monkeys are anesthetized with ketamine hydrochloride (15 mg/kg, i.m.) and xylazine hydrochloride (0.7 mg/kg, i.m.), and are implanted into the abdominal cavity with a transmitter (Model #TL11M2-D70-PCT, Data Sciences, St. Paul, MN). The pressure catheter is inserted into the lower abdominal aorta *via* the femoral artery. The bipotential leads are placed in Lead II configuration. The animals are housed under constant temperature (19-25°C), humidity (>40%) and lighting conditions (12 h light and dark cycle), are fed once daily, and are allowed free access to water. The animals are sodium depleted by placing them on a low sodium diet (0.026%, Expanded Primate Diet 829552 MP-VENaCl (P), Special Diet Services, Ltd., UK) 7 days before the experiment and furosemide (3 mg/kg, intramuscularly i.m., Aventis Pharmaceuticals) is administered at -40 h and -16 h prior to administration of test compound.

For oral dosing, the renin inhibitors are formulated in 0.5% methylcellulose at dose levels of 10 and 30 mg/kg (5 mL/kg) by infant feeding tubes. For intravenous delivery, a silastic catheter is implanted into posterior vena cava *via* a femoral vein. The catheter is attached to the delivery pump *via* a tether system and a swivel joint. Test compound (dose levels of 0.1 to 10 mg/kg, formulated at 5% dextrose) is administered by continuous infusion (1.67 mL/kg/h) or by bolus injection (3.33 mL/kg in 2 min).

Arterial blood pressures (systolic, diastolic and mean) and body temperature are recorded continuously at 500 Hz and 50 Hz, respectively, using the Dataquest™ A.R.T. (Advanced Research Technology) software. Heart rate is derived from the phasic blood pressure tracing. During the recording period, the monkeys are kept in a separate room without human presence to avoid pressure changes secondary to stress. All data are expressed as mean ± SEM. Effects of the renin inhibitors on blood pressure are assessed by ANOVA, taking into account the factors dose and time compared with the vehicle group.

Double Transgenic Rat: The efficacy of the renin inhibitor **6a** was evaluated *in vivo* in double transgenic rats engineered to express human renin and human angiotensinogen (Bohlender J, Fukamizu A, Lippoldt A, Nomura T, Dietz R, Menard J, Murakami K, Luft FC, Ganten D. High human renin hypertension in transgenic rats. Hypertension 1997, 29, 428-434).

Experiments were conducted in 6-week-old double transgenic rats (dTGRs). The model has been described in detail earlier. Briefly, the human renin construct used to generate transgenic animals made up the entire genomic human renin gene (10 exons and 9 introns), with 3.0 kB of the 5'-promoter region and 1.2 kB of 3' additional sequences. The human angiotensinogen construct made up the entire human angiotensinogen gene (5 exons and 4 introns), with 1.3 kB of 5'-flanking and 2.4 kB of 3'-flanking sequences. The rats were purchased from RCC Ltd (Füllinsdorf, Switzerland). Radio telemetry transmitters were surgically implanted at 4 weeks of age. The telemetry system provided 24-h recordings of systolic, mean, diastolic arterial pressure (SAP, MAP, DAP, respectively) and heart rate (HR). Beginning on day 42, animals were transferred to telemetry cages. A 24 h telemetry reading was obtained. Rats were then dosed orally on the following 4 consecutive days (days 43-46). The rats were monitored continuously and allowed free access to standard 0.3%-sodium rat chow and drinking water.

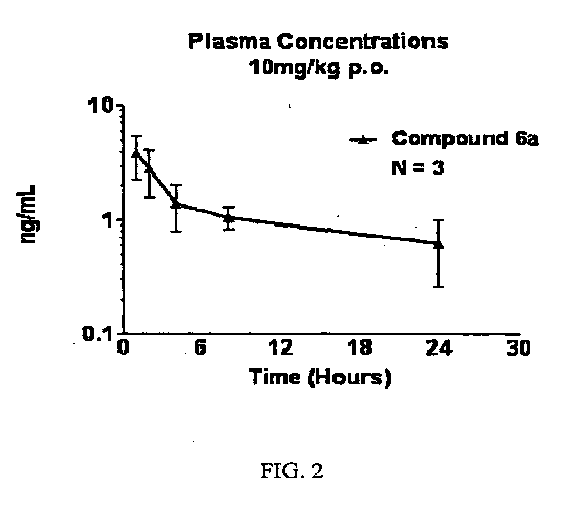

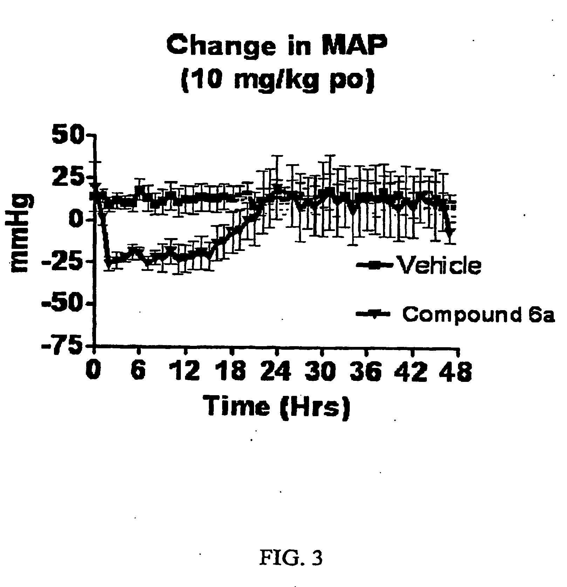

The *in vivo* transgenic rat activities for compound **6a** are shown in Figures 2 and 3. As shown in Figure 2, compound **6a** is readily available in rat's plasma following oral adminstration and the plasma concentration of compound **6a** remains relatively high over 24 h period, demonstrating its excellent oral bioavailability and metabolic stability. In addition, compound 6a exhibited significant effect in lowering blood pressures of transgenic rats at a dosage of 10 mg/kg, as shown in Figure 3.

## Claims

1. A compound represented by the following structural formula: 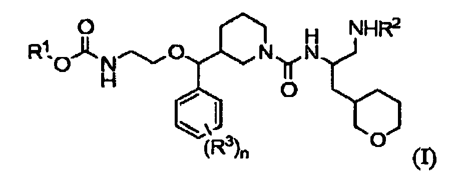 or a pharmaceutically acceptable salt thereof, wherein:
R¹ is alkyl, cycloalkyl or cycloalkylalkyl;
R² is H or alkyl;
R³ is F, Cl, Br, cyano, nitro, alkyl, haloalkyl, alkoxy, haloalkoxy or alkanesulfonyl; and
n is 0, 1, 2, or 3.

2. The compound of Claim 1 wherein:
R¹ is (C₁-C₃)alkyl;
R² is H or (C₁-C₃)alkyl;
R³ is F, Cl, Br, cyano, nitro, (C₁-C₃)alkyl, halo(C₁-C₃)alkyl, (C₁-C₃)alkoxy, halo(C₁-C₃)alkoxy or (C₁-C₃)alkanesulfonyl; and
n is 0, 1, 2, or 3.

3. The compound of Claim 1, wherein the compound is represented by the following structural formula: 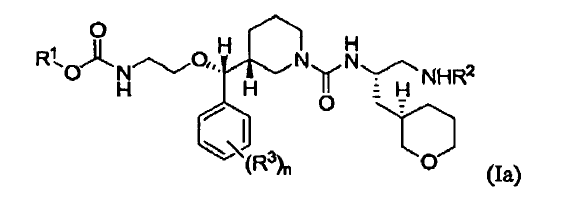 or a pharmaceutically acceptable salt thereof, wherein:
R¹ is (C₁-C₃)alkyl;
R² is H or (C₁-C₃)alkyl;
R³ is F, Cl, Br, cyano, nitro, (C₁-C₃)alkyl, halo(C₁-C₃)alkyl, (C₁-C₃)alkoxy, halo(C₁-C₃)alkoxy or (C₁-C₃)alkanesulfonyl; and n is 0, 1, 2, 3, or 4.

4. The compound according to any one of Claims 1-3, wherein R¹ is methyl or ethyl, and wherein R² is methyl.

5. The compound according to any one of Claims 1-4, wherein R³ is F, Cl, or methyl, and wherein n is 1 or 2.

6. The compound of Claim 1, wherein the compound is represented by a structural formula selected from: 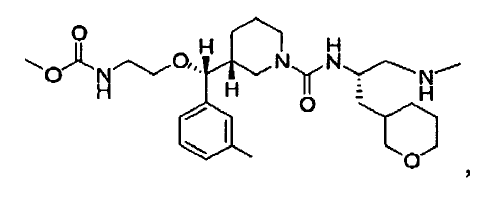 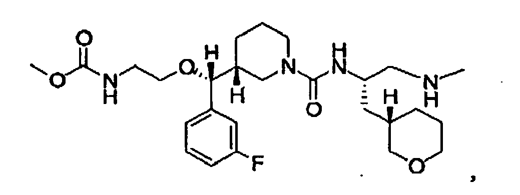 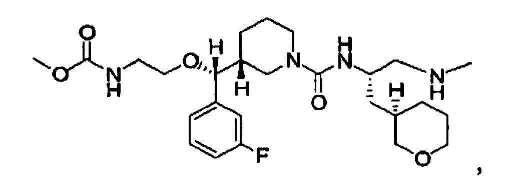 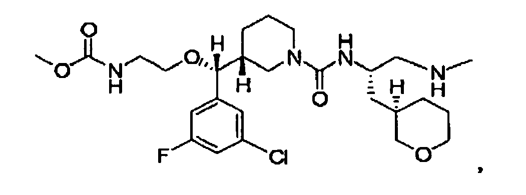 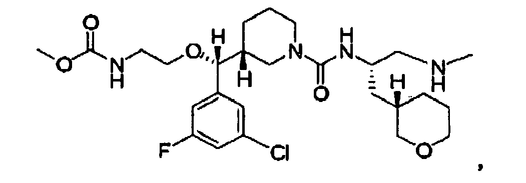 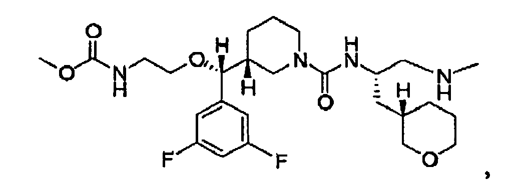 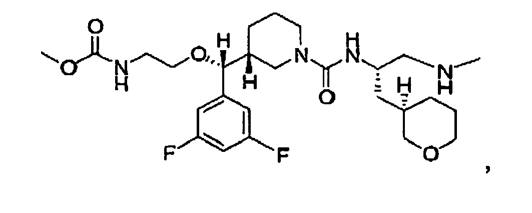 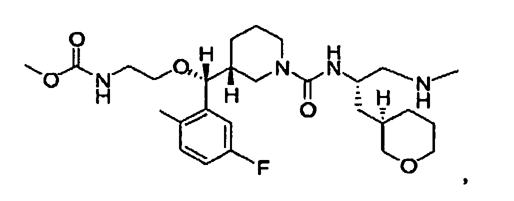 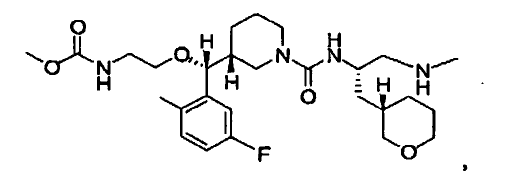 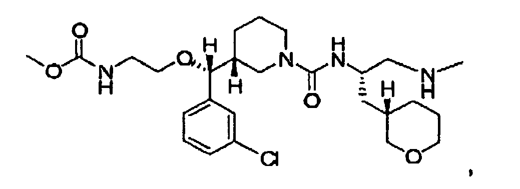 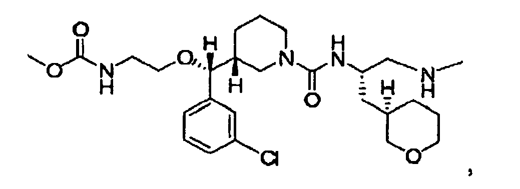 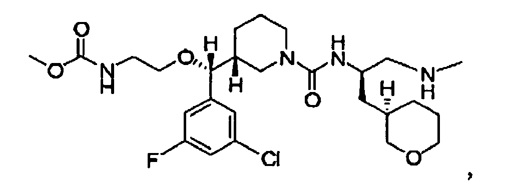 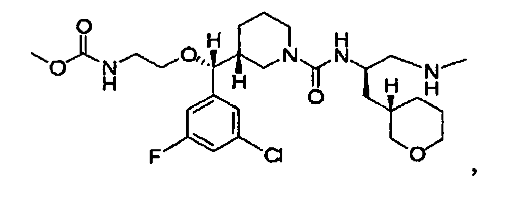 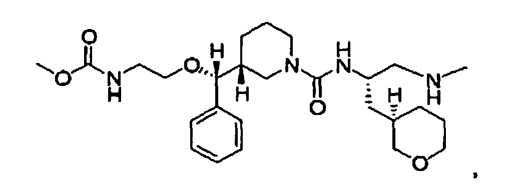 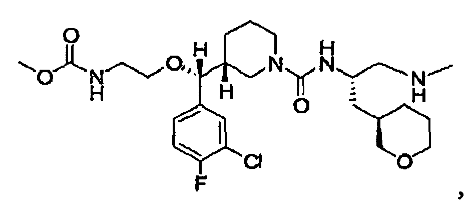 and 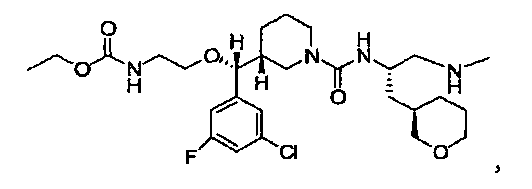 or a pharmaceutically acceptable salt thereof.

7. A compound according to Claim 1, represented by the following structural formula: 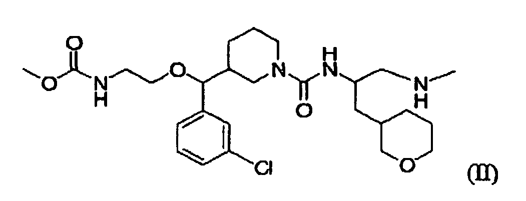 or a pharmaceutically acceptable salt thereof.

8. A compound according to Claim 1, represented by the following structural formula: 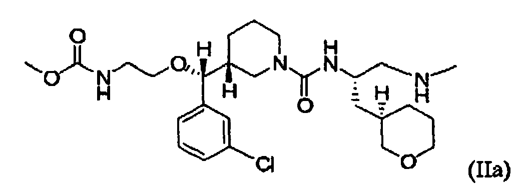 or a pharmaceutically acceptable salt thereof, wherein the compound is at least 90% optically pure.

9. The compound of Claim 8, wherein the compound is in the form of the tartrate salt.

10. The compound of Claim 9, wherein the tartrate salt is **characterized by** a x-ray powder diffraction pattern of Figure 1.

11. A compound according to Claim 1, represented by the following structural formula: 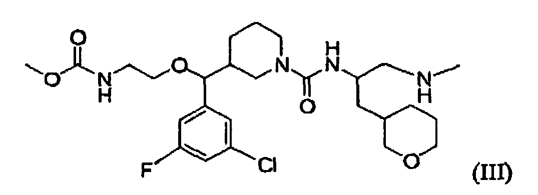 or a pharmaceutically acceptable salt thereof.

12. A compound according to Claim 1, represented by the following structural formula: 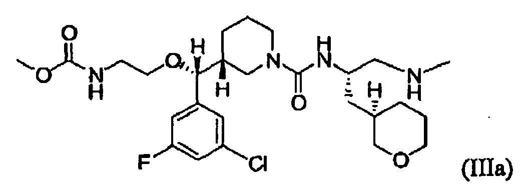 or a pharmaceutically acceptable salt thereof, wherein the compound is at least 90% optically pure.

13. A pharmaceutical composition comprising a pharmaceutically acceptable carrier or diluent and the compound according to any one of Claims 1-12.

14. The pharmaceutical composition of Claim 13, further comprising a α-blocker, β-blocker, calcium channel blocker, diuretic, natriuretic, saluretic, centrally acting antiphypertensive, angiotensin converting enzyme inhibitor, dual angiotensin converting enzyme and neutral endopeptidase inhibitor, angiotensin-receptor blocker, dual angiotensin-receptor blocker and endothelin receptor antagonist, aldosterone synthase inhibitor, aldosterone-receptor antagonist, or endothelin receptor antagonist.

15. A compound according to any one of Claims 1-12 fon use in antagonizing one or more aspartic proteases in a subject in need thereof, comprising administering to the subject an effective amount of said compound.

16. The compound for use in antagonizing one or more aspartic proteases in a subject in need thereof according to Claim 15, wherein the aspartic protease is renin.

17. Use of a compound according to any one of Claims 1-12 for the manufacture of a medicament for antagonizing one or more aspartic proteases in a subject in need thereof.

18. A compound according to any one of Claims 1-12 for use in treating an aspartic protease mediated disorder in a subject comprising administering to the subject an effective amount of said compound.

19. The compound for use in treating an aspartic protease mediated disorder in a subject according to Claim 18, wherein said disorder is hypertension, congestive heart failure, cardiac hypertrophy, cardiac fibrosis, cardiomyopathy post-infarction, nephropathy, vasculopathy and neuropathy, a disease of the coronary vessels, post-surgical hypertension, restenosis following angioplasty, raised intra-ocular pressure, glaucoma, abnormal vascular growth, hyperaldosteronism, an anxiety state, or a cognitive disorder.

20. The compound for use in treating an aspartic protease mediated disorder in a subject according to Claim 18, further comprising administering to the subject one or more additional agents selected from the group consisting of an α-blockers, a β-blocker, a calcium channel blocker, a diuretic, an angiotensin converting enzyme inhibitor, a dual angiotensin converting enzyme and neutral endopeptidase inhibitor, an angiotensin-receptor blocker, dual angiotensin-receptor blocker and endothelin receptor antagonist, an aldosterone synthase inhibitor, an aldosterone-receptor antagonist, and an endothelin receptor antagonist.

21. The compound for use in treating an aspartic protease mediated disorder in a subject according to Claim 18, wherein the aspartic protease is β-secretase, plasmepsin or HIV protease.

22. Use of a compound according to any one of Claims 1-12 for the manufacture of a medicament for treating an aspartic protease mediated disorder in a subject.

23. A compound represented by a structural formula selected from the group consisting of: 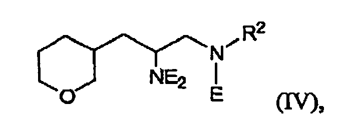 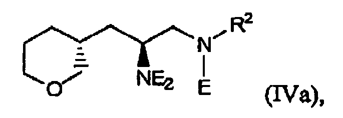 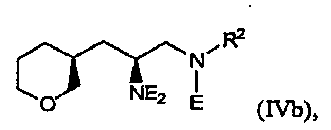 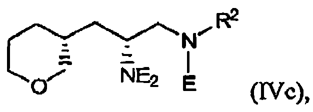 and 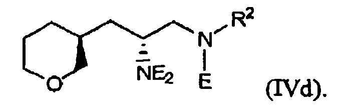 or salt thereof, wherein:
E, for each occurrence, is independently H, an amine protecting group; and
R² is H or (C₁-C₃)alkyl.

24. The compound of Claim 23, wherein the compound is selected from the group consisting of:
tert-butyl (S)-1-(methylamino)-3-((R)-tetrahydro-2H-pyran-3-yl)propan-2-ylcarbamate;
(S)-tert-butyl-1-(N-methyl-2-(trimethylsilyl)ethoxycarbonyl-amino)-3-((R)-tetrahydro-2H-pyran-3-yl)propylcarbamate;
2-(trimethylsilyl)ethyl (S)-2-amino-3-((R)-tetrahydro-2H-pyran-3-yl)propyl(methyl)carbamate;
tert-butyl (S)-1-(methylamino)-3-(tetrahydro-2H-pyran-3-yl)proparan-2-ylcarbamate;
(S)-tert-butyl-1-(N-methyl-2-(trimethylsilyl)ethoxycarbonyl-amino)-3-(tetrahydro-2H-pyran-3-yl)propylcarbamate;
2-(trimethylsilyl)ethyl (S)-2-amino-3-((R)-tetrahydro-2H-pyran-3-yl)propyl(methyl)carbamate;
tert-butyl (S)-1-(methylamino)-3-((S)-tetrahydro-2H-pyran-3-yl)propan-2-ylcarbamate;
(S)-tert-butyl-1-(N-methyl-2-(trimethylsilyl)ethoxycarbonyl-amino)-3-((S)-tetrahydro-2H-pyran-3-yl)propylcarbamate; and
2-(trimethylsilyl)ethyl (S)-2-amino-3-((S)-tetrahydro-2H-pyran-3-yl)propyl(methyl)carbamate.

25. The compound of Claim 23, wherein: E is an amine protecting group, selected from the group consisting of: carbamate, amide or sulfonamide.

26. The compound of Claim 25, wherein: E is an amine protecting group, selected from the group consisting of: tert-butoxycarbonyl, benzyloxycarbonyl or 1-[2-(trimethylsilyl)ethoxycarbonyl].

## Patentansprüche

1. Verbindung der folgenden Strukturformel: 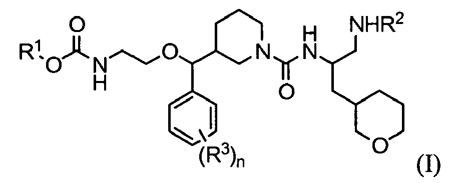 oder ein pharmazeutisch verträgliches Salz davon, wobei:
R¹ Alkyl, Cycloalkyl oder Cycloalkylalkyl ist;
R² H oder Alkyl ist;
R³ F, Cl, Br, Cyano, Nitro, Alkyl, Halogenalkyl, Alkoxy,
Halogenalkoxy oder Alkansulfonyl ist; und
n 0, 1, 2 oder 3 ist.

2. Verbindung nach Anspruch 1, wobei:
R¹ (C₁-C₃)-Alkyl ist;
R² H oder (C₁-C₃)-Alkyl ist;
R³ F, Cl, Br, Cyano, Nitro, (C₁-C₃)-Alkyl, Halogen(C₁-C₃)alkyl, (C₁-C₃)-Alkoxy, Halogen(C₁-C₃)alkoxy oder
(C₁-C₃)-Alkansulfonyl ist; und
n 0, 1, 2 oder 3 ist.

3. Verbindung nach Anspruch 1, wobei die Verbindung durch die folgende Strukturformel dargestellt ist: 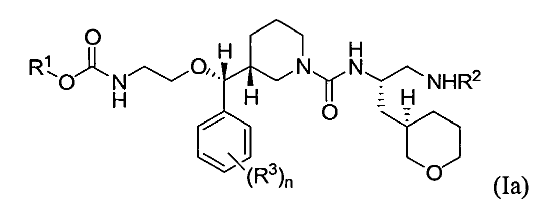 oder ein pharmazeutisch verträgliches Salz davon, wobei:
R¹ (C₁-C₃)-Alkyl ist;
R² H oder (C₁-C₃)-Alkyl ist;
R³ F, Cl, Br, Cyano, Nitro, (C₁-C₃)-Alkyl, Halogen(C₁-C₃)alkyl, (C₁-C₃)-Alkoxy,
Halogen(C₁-C₃)alkoxy oder (C₁-C₃)-Alkansulfonyl ist; und
n 0, 1, 2, 3 oder 4 ist.

4. Verbindung nach einem der Ansprüche 1-3, wobei R¹ Methyl oder Ethyl ist und wobei R² Methyl ist.

5. Verbindung nach einem der Ansprüche 1-4, wobei R³ F, Cl oder Methyl ist und wobei n 1 oder 2 ist.

6. Verbindung nach Anspruch 1, wobei die Verbindung durch eine Strukturformel dargestellt ist, ausgewählt aus: 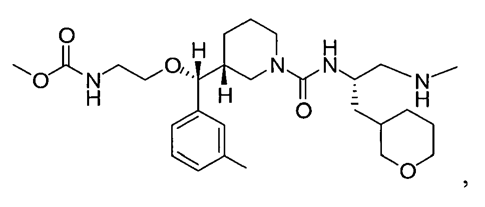 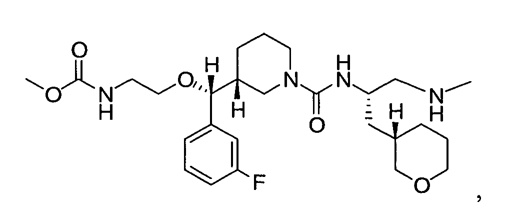 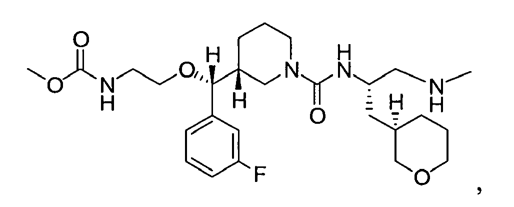 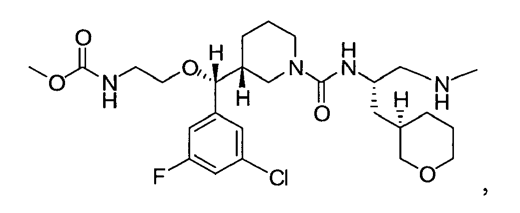 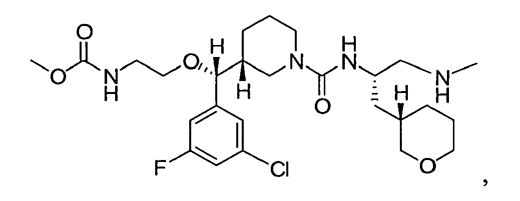 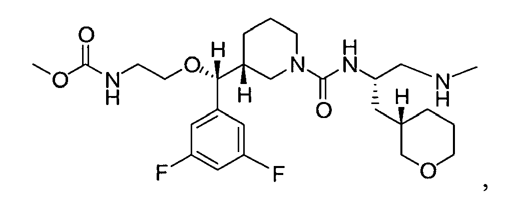 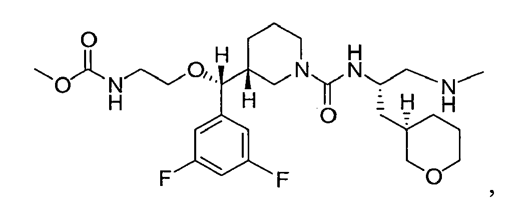 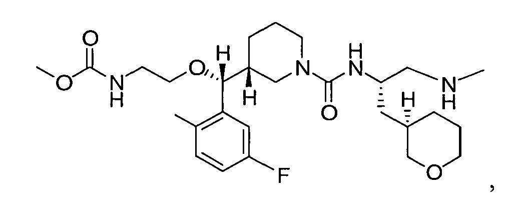 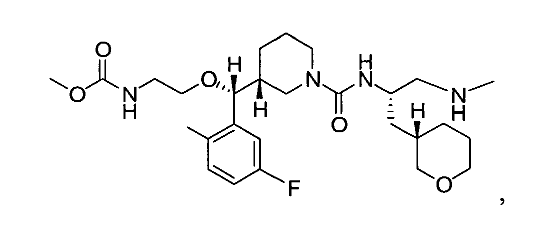 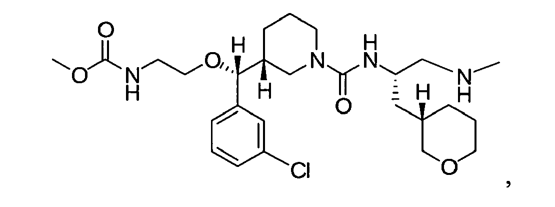 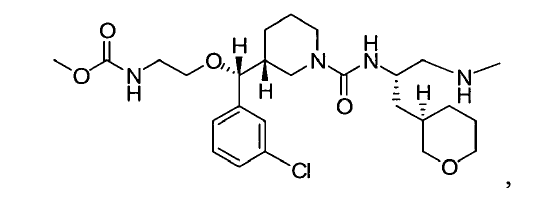 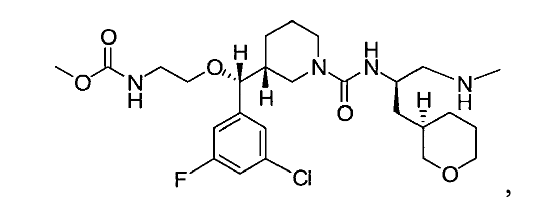 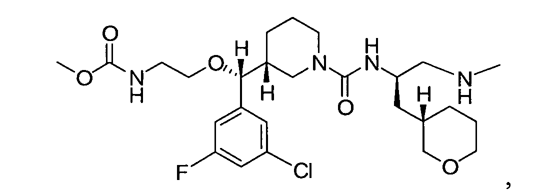 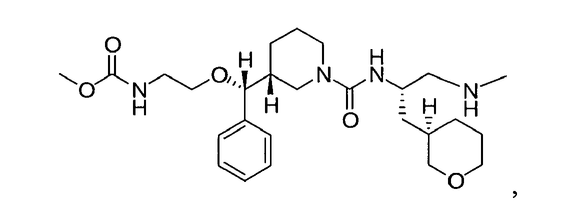 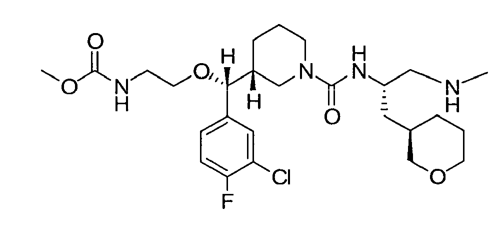 und 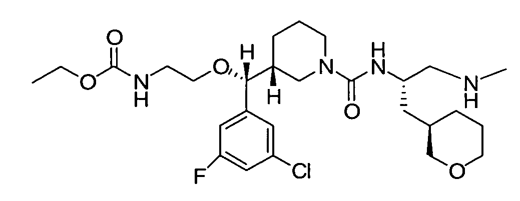 oder ein pharmazeutisch verträgliches Salz davon.

7. Verbindung nach Anspruch 1 der folgenden Strukturformel: 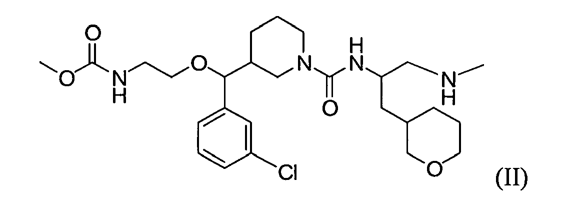 oder ein pharmazeutisch verträgliches Salz davon.

8. Verbindung nach Anspruch 1 der folgenden Strukturformel: 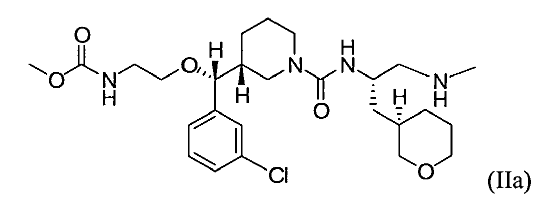 oder ein pharmazeutisch verträgliches Salz davon, wobei die Verbindung zu mindestens 90% optisch rein ist.

9. Verbindung nach Anspruch 8, wobei die Verbindung in der Form des Tartratsalzes vorliegt.

10. Verbindung nach Anspruch 9, wobei das Tartratsalz durch ein Röntgenpulverbeugungsmuster von Figur 1 **gekennzeichnet** ist.

11. Verbindung nach Anspruch 1 der folgenden Strukturformel: 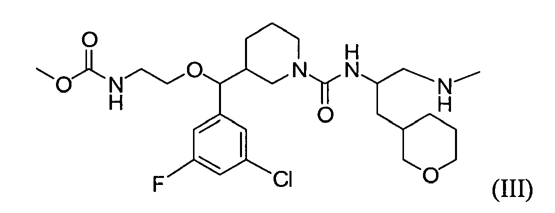 oder ein pharmazeutisch verträgliches Salz davon.

12. Verbindung nach Anspruch 1 der folgenden Strukturformel: 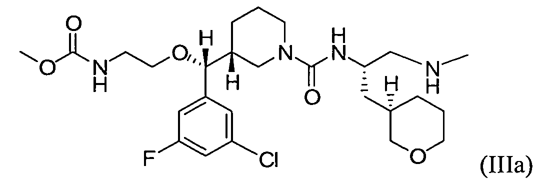 oder ein pharmazeutisch verträgliches Salz davon, wobei die Verbindung zu mindestens 90% optisch rein ist.

13. Arzneimittel, umfassend einen pharmazeutisch verträglichen Träger oder ein ebensolches Verdünnungsmittel und die Verbindung nach einem der Ansprüche 1-12.

14. Arzneimittel nach Anspruch 13, des Weiteren umfassend einen α-Blocker, β-Blocker, Calciumkanalblocker, Diuretikum, Natriuretikum, Saluretikum, zentral wirkendes Mittel gegen Bluthochdruck, Hemmer für Angiotensin umwandelnde Enzyme, Hemmer für duale Angiotensin umwandelnde Enzyme und neutrale Endopeptidase, Angiotensinrezeptorblocker, dualen Angiotensinrezeptorblocker und Endothelinrezeptorantagonisten, Aldosteronsynthasehemmer, Aldosteronrezeptorantagonisten oder Endothelinrezeptorantagonisten.

15. Verbindung nach einem der Ansprüche 1-12 zur Verwendung beim Antagonisieren von einer oder mehreren Aspartamproteasen bei einem dies benötigenden Patienten, umfassend das Verabreichen einer wirksamen Menge der Verbindung dem Patienten.

16. Verbindung zur Verwendung beim Antagonisieren von einer oder mehreren Aspartamproteasen bei einem dies benötigenden Patienten nach Anspruch 15, wobei die Aspartamprotease Renin ist.

17. Verwendung einer Verbindung nach einem der Ansprüche 1-12 zur Herstellung eines Medikaments zum Antagonisieren von einer oder mehreren Aspartamproteasen bei einem dies benötigenden Patienten.

18. Verbindung nach einem der Ansprüche 1-12 zur Verwendung bei der Behandlung einer Aspartamprotease-vermittelten Störung bei einem Patienten, umfassend das Verabreichen einer wirksamen Menge der Verbindung dem Patienten.

19. Verbindung zur Verwendung bei der Behandlung einer Aspartamprotease-vermittelten Störung bei einem Patienten nach Anspruch 18, wobei die Störung Bluthochdruck, kongestives Herzversagen, Herzhypertrophie, Herzfibrose, Kardiomyopathie nach einem Infarkt, Nephropathie, Vaskulopathie und Neuropathie, eine Erkrankung der Herzkranzgefäße, postoperativer Bluthochdruck, Restenose infolge von Angioplastie, erhöhter Augeninnendruck, Glaukom, abnormales Gefäßwachstum, Hyperaldosteronismus, ein Angstzustand oder eine kognitive Störung ist.

20. Verbindung zur Verwendung bei der Behandlung einer Aspartamprotease-vermittelten Störung bei einem Patienten nach Anspruch 18, des Weiteren umfassend das Verabreichen von einem oder mehreren zusätzlichen Mitteln, ausgewählt aus der Gruppe, bestehend aus einem α-Blocker, einem β-Blocker, einem Calciumkanalblocker, einem Diuretikum, einem Hemmer für Angiotensin umwandelnde Enzyme, einem Hemmer für duale Angiotensin umwandelnde Enzyme und neutrale Endopeptidase, einem Angiotensinrezeptorblocker, einem dualen Angiotensinrezeptorblocker und Endothelinrezeptorantagonisten, einem Aldosteronsynthasehemmer, einem Aldosteronrezeptorantagonisten und einem Endothelinrezeptorantagonisten, dem Patienten.

21. Verbindung zur Verwendung bei der Behandlung einer Aspartamprotease-vermittelten Störung bei einem Patienten nach Anspruch 18, wobei die Aspartamprotease β-Secretase, Plasmepsin oder HIV-Protease ist.

22. Verwendung einer Verbindung nach einem der Ansprüche 1-12 zur Herstellung eines Medikaments zur Behandlung einer Aspartamprotease-vermittelten Störung bei einem Patienten.

23. Verbindung einer Strukturformel, ausgewählt aus der Gruppe, bestehend aus: 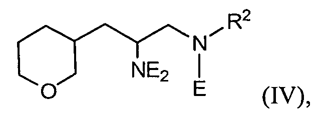 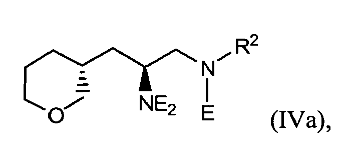 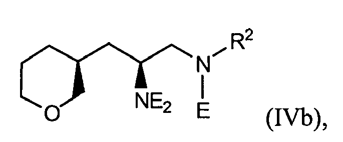 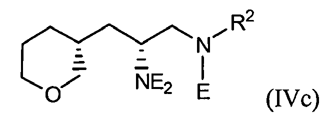 und 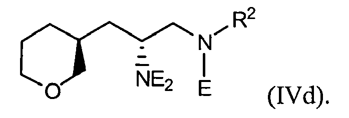 oder ein Salz davon, wobei:
E für jedes Vorkommen unabhängig H, eine Aminschutzgruppe ist; und
R² H oder (C₁-C₃)-Alkyl ist.

24. Verbindung nach Anspruch 23, wobei die Verbindung ausgewählt ist aus der Gruppe, bestehend aus:
tert-Butyl-(S)-1-(methylamino)-3-((R)-tetrahydro-2H-pyran-3-yl)propan-2-ylcarbamat;
(S)-tert-Butyl-1-(N-methyl-2-(trimethylsilyl)ethoxycarbonylamino)-3-((R)-tetrahydro-2H-pyran-3 -yl)propylcarbamat;
2-(Trimethylsilyl)ethyl-(S)-2-amino-3-((R)-tetrahydro-2H-pyran-3-yl)propyl(methyl)carbamat;
tert-Butyl (S)-1-(methylamino)-3-(tetrahydro-2H-pyran-3-yl)propan-2-ylcarbamat;
(S)-tert-Butyl-1-(N-methyl-2-(trimethylsilyl)ethoxycarbonylamino)-3-(tetrahydro-2H-pyran-3-yl)propylcarbamat;
2-(Trimethylsilyl)ethyl-(S)-2-amino-3-((R)-tetrahydro-2H-pyran-3-yl)propyl(methyl)carbamat;
tert-Butyl-(S)-1-(methylamino)-3-((S)-tetrahydro-2H-pyran-3-yl)propan-2-ylcarbamat;
(S)-tert-Butyl-1-(N-methyl-2-(trimethylsilyl)ethoxycarbonylamino)-3-((S)-tetrahydro-2H-pyran-3-yl)propylcarbamat; und
2-(Trimethylsilyl)ethyl-(S)-2-amino-3-((S)-tetrahydro-2H-pyran-3-yl)propyl(methyl)carbamat.

25. Verbindung nach Anspruch 23, wobei E eine Aminschutzgruppe ist, ausgewählt aus der Gruppe, bestehend aus: Carbamat, Amid oder Sulfonamid.

26. Verbindung nach Anspruch 25, wobei E eine Aminschutzgruppe ist, ausgewählt aus der Gruppe, bestehend aus: tert-Butoxycarbonyl, Benzyloxycarbonyl oder 1-[2-(Trimethylsilyl)ethoxycarbonyl].

## Revendications

1. Composé représenté par la formule développée suivante : 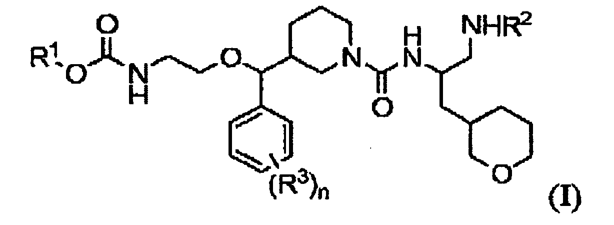 ou l'un de ses sels pharmaceutiquement acceptables , dans lequel :
R¹ est un groupe alkyle, cycloalkyle ou cycloalkylalkyle ;
R² est H ou un groupe alkyle ;
R³ est F, Cl, Br, un groupe cyano, nitro, alkyle, halogènalkyle, alcoxy, halogènalcoxy ou alcanesulfonyle ; et
n vaut 0, 1, 2 ou 3.

2. Composé selon la revendication 1, dans lequel :
R¹ est un groupe alkyle en C₁-C₃ ;
R² est H ou un groupe alkyle en C₁-C₃ ;
R³ est F, Cl, Br, un groupe cyano, nitro, alkyle, halogènalkyle en C₁-C₃, alcoxy en C₁-C₃, halogènalcoxy en C₁-C₃ ou alcanesulfonyle en C₁-C₃ ; et
n vaut 0, 1, 2 ou 3.

3. Composé selon la revendication 1, dans lequel le composé est représenté par la formule développée suivante : 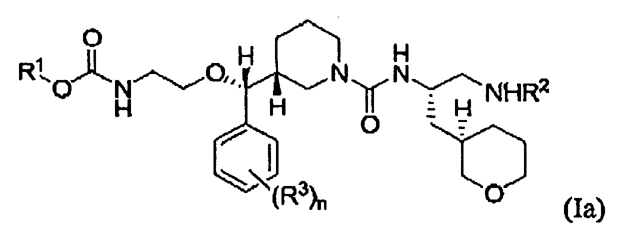 ou l'un de ses sels pharmaceutiquement acceptables, dans lequel :
R¹ est un groupe alkyle en C₁-C₃ ;
R² est H ou un groupe alkyle en C₁-C₃ ;
R³ est F, Cl, Br, un groupe cyano, nitro, alkyle en C₁-C₃, halogènalkyle en C₁-C₃, alcoxy en C₁-C₃, halogènalcoxy en C₁-C₃ ou alcanesulfonyle en C₁-C₃ ; et n vaut 0, 1, 2, 3 ou 4.

4. Composé selon l'une quelconque des revendications 1 à 3, dans lequel R¹ est le groupe méthyle ou éthyle, et R² est le groupe méthyle.

5. Composé selon l'une quelconque des revendications 1 à 4, dans lequel R³ est F, Cl ou le groupe méthyle, et n vaut 1 ou 2.

6. Composé selon la revendication 1, dans lequel le composé est représenté par la formule développée choisie parmi : 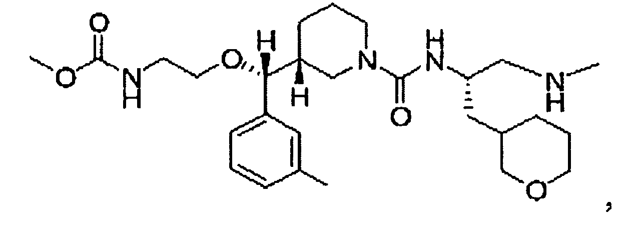 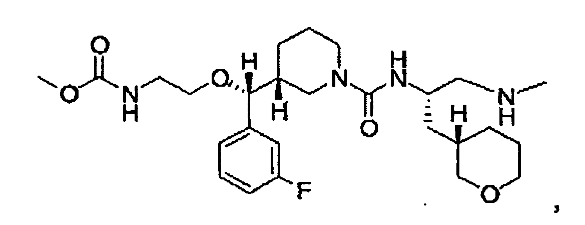 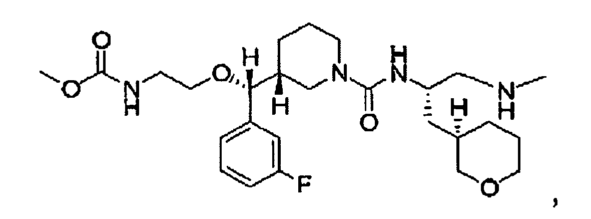 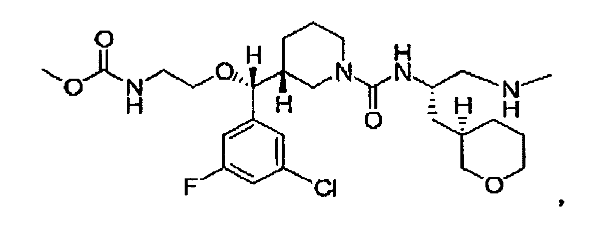 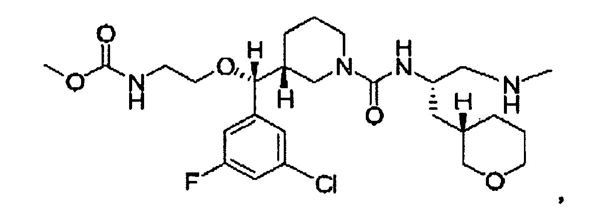 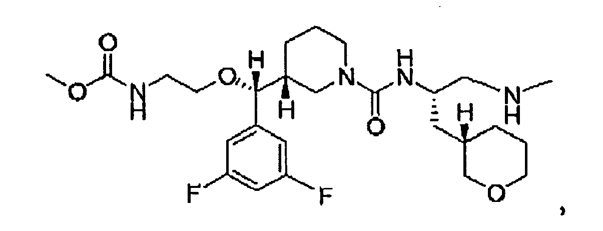 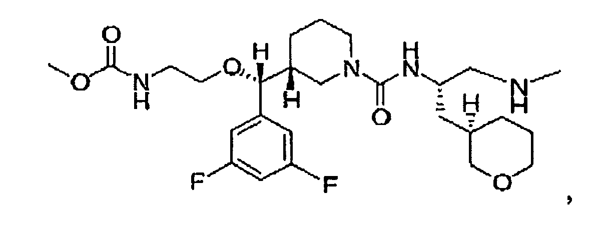 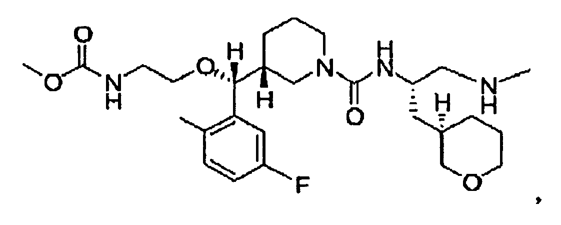 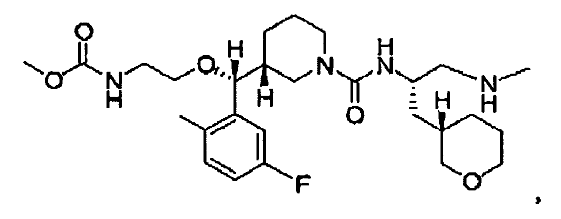 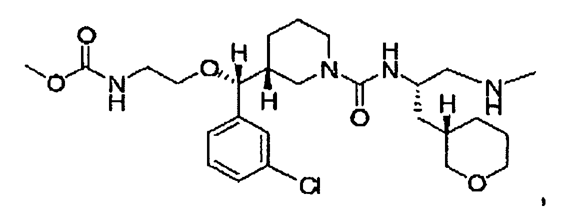 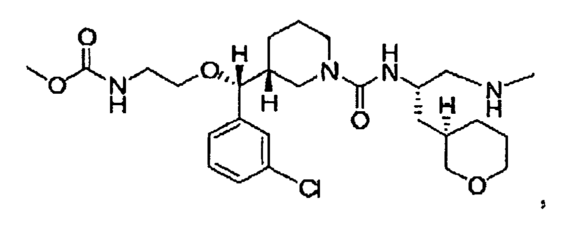 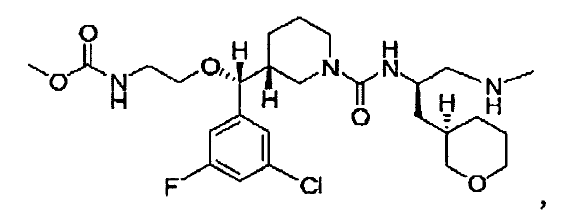 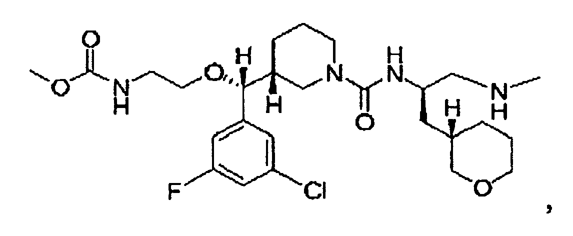 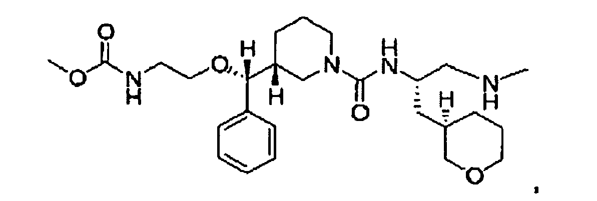 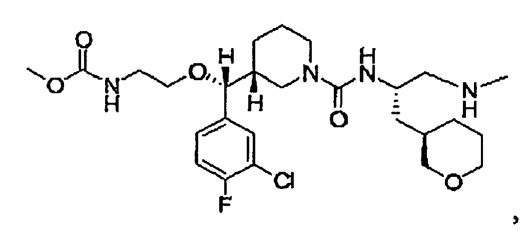 and 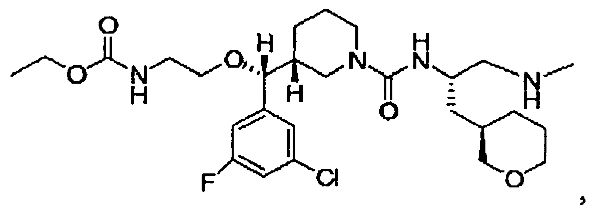 *(and* = *ou)*
ou l'un de ses sels pharmaceutiquement acceptables.

7. Composé selon la revendication 1, représenté par la formule développée suivante : 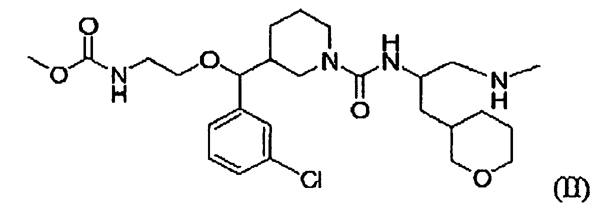 ou l'un de ses sels pharmaceutiquement acceptables.

8. Composé selon la revendication 1, représenté par la formule développée suivante : 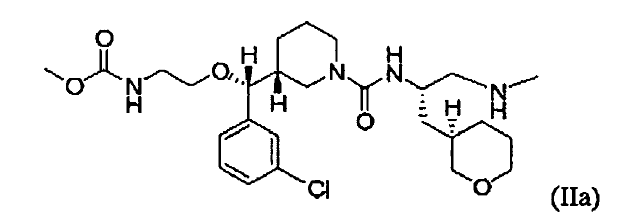 ou l'un de ses sels pharmaceutiquement acceptables, le composé étant optiquement pur à au moins 90 %.

9. Composé selon la revendication 8, dans lequel le composé se présente sous forme du sel tartrate.

10. Composé selon la revendication 9, dans lequel le sel tartrate est **caractérisé par** une image de diffraction aux rayons X par la méthode des poudres de la Figure 1.

11. Composé selon la revendication 1, représenté par la formule développée suivante : 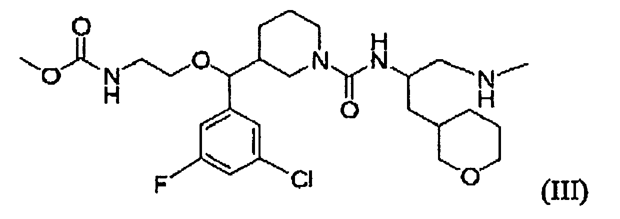 ou l'un de ses sels pharmaceutiquement acceptables.

12. Composé selon la revendication 1, représenté par la formule développée suivante : 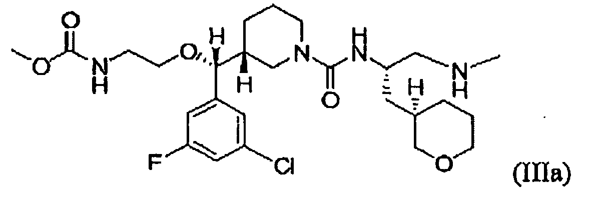 ou l'un de ses sels pharmaceutiquement acceptables, le composé étant optiquement pur à au moins 90 %.

13. Composition pharmaceutique comprenant un support ou diluant pharmaceutiquement acceptable et le composé selon l'une quelconque des revendications 1 à 12.

14. Composition pharmaceutique selon la revendication 13, qui comprend en outre un α-bloquant, un β-bloquant, un inhibiteur calcique, un diurétique, un natriurétique, un salidiurétique, un antihypertenseur à action centrale, un inhibiteur de l'enzyme de conversion de l'angiotensine, un inhibiteur double de l'enzyme de conversion de l'angiotensine et de l'endopeptidase neutre, un agent bloquant le récepteur de l'angiotensine, un antagoniste double de l'agent bloquant le récepteur de l'angiotensine et le récepteur de l'endothéline, un inhibiteur de l'aldostérone synthase, un antagoniste du récepteur de l'aldostérone ou un antagoniste du récepteur de l'endothéline.

15. Composé selon l'une quelconque des revendications 1 à 12, pour utilisation dans le but d'antagoniser une ou plusieurs protéases aspartiques chez un sujet qui en a besoin, comprenant l'administration au sujet d'une quantité efficace dudit composé.

16. Composé pour utilisation dans le but d'antagoniser une ou plusieurs protéases aspartiques chez un sujet qui en a besoin selon la revendication 15, la protéase aspartique étant la rénine.

17. Utilisation d'un composé selon l'une quelconque des revendications 1 à 12 pour la fabrication d'un médicament destiné à antagoniser une ou plusieurs protéases aspartiques chez un sujet qui en a besoin.

18. Composé selon l'une quelconque des revendications 1 à 12 pour utilisation dans le traitement d'un trouble médié par les protéases aspartiques chez un sujet, comprenant l'administration au sujet d'une quantité efficace dudit composé.

19. Composé pour utilisation dans le traitement d'un trouble médié par les protéases aspartiques chez un sujet selon la revendication 18, dans lequel ledit trouble est l'hypertension, l'insuffisance cardiaque congestive, l'hypertrophie cardiaque, la fibrose cardiaque, la cardiomyopathie post-infarctus, la néphropathie, la vasculopathie et la neuropathie, une maladie des vaisseaux coronaires, une hypertension post-chirurgicale, une resténose après angioplastie, une hypertension intra-oculaire, un glaucome, une croissance vasculaire anormale, un hyperaldostéronisme, un état d'anxiété ou un trouble cognitif.

20. Composé pour utilisation dans le traitement d'un trouble médié par la protéase aspartique chez un sujet selon la revendication 18, qui comprend en outre l'administration au sujet d'un ou plusieurs agents additionnels choisis dans le groupe consistant en un α-bloquant, un β-bloquant, un inhibiteur calcique, un diurétique, un natriurétique, un salidiurétique, un antihypertenseur à action centrale, un inhibiteur de l'enzyme de conversion de l'angiotensine, un inhibiteur double de l'enzyme de conversion de l'angiotensine et de l'endopeptidase neutre, un agent bloquant le récepteur de l'angiotensine, un antagoniste double de l'agent bloquant le récepteur de l'angiotensine et le récepteur de l'endothéline, un inhibiteur de l'aldostérone synthase, un antagoniste du récepteur de l'aldostérone ou un antagoniste du récepteur de l'endothéline.

21. Composé pour utilisation dans le traitement d'un trouble médié par les protéases aspartiques, chez un sujet selon la revendication 18, dans lequel la protéase aspartique est la β-sécrétase, la plasmepsine ou la protéase du VIH.

22. Utilisation d'un composé selon l'une quelconque des revendications 1 à 12 pour la fabrication d'un médicament destiné au traitement d'un trouble médié par la protéase aspartique chez un sujet.

23. Composé représenté par la formule développée choisie dans le groupe consistant en : 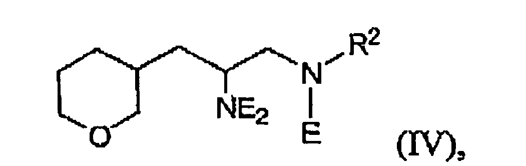 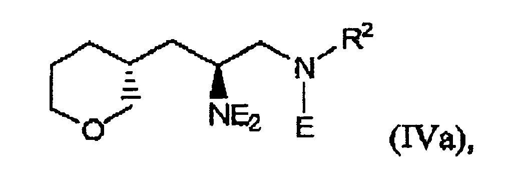 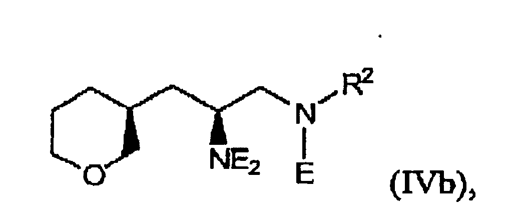 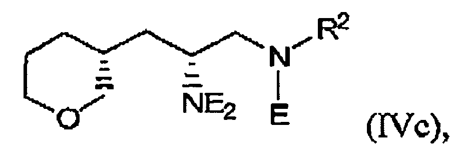 and 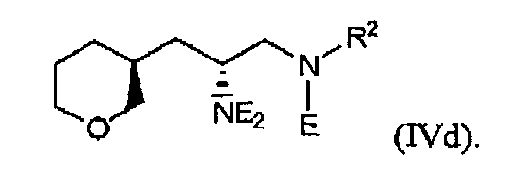 *(and = et)*
ou l'un de ses sels, dans lequel :
E pour chaque occurrence, représente indépendamment H, un groupe protecteur d'amine ; et
R² est H ou un groupe alkyle en C₁-C₃.

24. Composé selon la revendication 23, dans lequel le composé est choisi dans le groupe consistant en :
le (S)-1-(méthylamino)-3-((R)-tétrahydro-2H-pyrann-3-yl)propan-2-ylcarbamate de tert-butyle ;
le 1-(N-méthyl-2-(triméthylsilyl)éthoxycarbonyl-amino)-3-((R)-tétrahydro-2H-pyrann-3-yl)propylcarbamate de (S)-tert-butyle ;
le (S)-2-amino-3-((R)-tétrahydro-2H-pyrann-3-yl)propyl(méthyl)carbamate de 2-(triméthylsilyl)éthyle ;
le (S)-1-méthylamino)-3-(tétrahydro-2H-pyrann-3-yl)propan-2-ylcarbamate de tert-butyle ;
le 1-(N-méthyl-2-(triméthylsilyl)éthoxycarbonyl-amino)-3-(tétrahydro-2H-pyrann-3-yl)propylcarbamate de (S)-tert-butyle ;
le (S)-2-amino-3-((R)-tétrahydro-2H-pyrann-3-yl)propyl(méthyl)carbamate de 2-(triméthylsilyl)éthyle ;
le (S)-1-(méthylamino)-3-((S)-tétrahydro-2H-pyrann-3-yl)propan-2-ylcarbamate de tert-butyle ;
le 1-(N-méthyl-2-(triméthylsilyl)éthoxycarbonyl-amino)-3-((S)-tétrahydro-2H-pyrann-3-yl)propylcarbamate de (S)-tert-butyle ; et
le (S)-2-amino-3-((S)-tétrahydro-2H-pyrann-3-yl)propyl(méthyl)carbamate de 2-(triméthylsilyl)éthyle.

25. Composé selon la revendication 23, dans lequel E est un groupe protecteur d'amine, choisi dans le groupe comprenant les carbamates, les amides ou les sulfonamides.

26. Composé selon la revendication 25, dans lequel E est un groupe protecteur d'amine choisi dans le groupe consistant en les groupes tert-butoxycarbonyle, benzyloxy-carbonyle ou 1-[2-(triméthylsilyl)éthoxy-carbonyle.
